# EUROPEAN PATENT APPLICATION

(11) **EP 1 754 487 A1**
(43) Date of publication of application: **21.02.2007**
(21) Application number: 05016308.8
(22) Date of filing: 27.07.2005
(51) Int. Cl.: A61K 38/17, C07K 14/00, G01N 33/50, A61P 3/04, A61P 3/10

(54) **Modulators of the Melted protein and uses thereof**

(71) Applicant: The European Molecular Biology Laboratory, 69117 Heidelberg (DE)
(72) Inventor: Cohen, Stephen, 69115 Heidelberg (DE); Teleman, Aurelio, 69115 Heidelberg (DE); Chen, Ya-Wen, 69117 Heidelberg (DE)
(74) Representative: Schüssler, Andrea

(57) **Abstract**

Described is a pharmaceutical composition comprising a compound (a) reducing or inhibiting, or a compound (b) increasing the biological activity of the protein or its expression. Said compounds are useful for the prevention/treatment of diseases wherein decrease or increase of the activity of the protein has a therapeutic effect, e.g., anorexia, adiposity or diabetes. Finally, methods for the diagnosis of diseases associated with an aberrant activity of the protein are described.

The present invention discloses novel uses for an energy homeostasis regulating protein and polynucleotides encoding said protein in the diagnosis, study, prevention, and treatment of metabolic diseases and disorders.

### Annex to the application documents - subsequently filed sequences listing

SEQUENCE LISTING

<110>  European Molecular Biology Laboratory

<120>  Modulators of the Activity of a Protein Involved in the
       Regulation of Energy Homeostasis

<130>  E 2214EP

<140>  EP05016308
<141>  2005-07-27

<160>  26

<170>  PatentIn version 3.2

<210>  1
<211>  30
<212>  DNA
<213>  artificial

<220>
<223>  Primer 1 for PH domain

<400>  1
ccccctcgag gctttctcaa tggcagcaat                                    30

<210>  2
<211>  32
<212>  DNA
<213>  artificial

<220>
<223>  Primer 2 for PH domain

<400>  2
cgcggatcct acgtgggatt atctcgggcc tg                                  32

<210>  3
<211>  27
<212>  DNA
<213>  artificial

<220>
<223>  Primer for UAS-melted-RNAi transgene

<400>  3
tctagactcg cacgcggact atgtgaa                                        27

<210>  4
<211>  27
<212>  DNA
<213>  artificial

<220>
<223>  Primer for UAS-melted-RNAi transgene

<400>  4
tctagacttg gcatggggtg gctcttc                                        27

<210>  5
<211>  27
<212>  DNA
<213>  artificial

<220>
<223>  Primer for UAS-melted RNAi transgene

<400>  5
tctagacgcc cacacgttgc agcattg                                          27


<210>  6
<211>  24
<212>  DNA
<213>  artificial

<220>
<223>  Primer for UAS-melted-RNAi transgene

<400>  6
tctagaggac tcccgcgtgc taag                                             24


<210>  7
<211>  992
<212>  PRT
<213>  Drosophila CG8624-PA

<400>  7

Met His Glu Leu Phe Asn Lys Val Leu Ala Lys Arg Asp Leu Ser Arg
1               5                   10                  15

Ala Gly Asp Leu Phe Ser Val Pro Asp Ala Asp Ile Val Asp Asp Ile
            20                  25                  30

Thr Glu Val Leu Ser Glu Ile Ser Pro Ile Ile Ser His Ala Asp Tyr
        35                  40                  45

Val Lys Asn Asn Asn Asp Gln Ser Val Val Glu Ile Cys Val Thr Arg
    50                  55                  60

Val Leu Ser Cys Ile Arg Glu Thr Lys Thr Ala Glu Arg Tyr Cys Ala
65                  70                  75                  80

Ala Leu Val Asp Leu Leu Arg Thr Cys Leu Leu Trp Asn Leu Gln Pro
                85                  90                  95

Ser Gly Thr Thr Lys Glu Glu Pro Pro His Ala Lys Ile Ala Ala Asp
            100                 105                 110

Ile Ile Ser Ser Ile Phe Leu Asn Tyr Asp Lys Lys Glu Leu Met Lys
        115                 120                 125

Ile Ala Leu Pro Val Ala Val Gln Phe Leu Pro Lys Gly Asn Arg Glu
    130                 135                 140

Leu Ser Arg Asn Leu Ala Ser Tyr Leu Ser Leu Ala Ala Ile Asp His
145                 150                 155                 160

Ala Gly Leu Leu Ser Pro His Thr Glu Ser Val Met Glu Ser Ile Leu

                165                 170                 175

Ser Gly Asn Tyr Gly Leu Leu Arg Val Leu Ser Gln Val Tyr Glu Val
            180                 185                 190

Ala Pro Glu Ala Val Thr Pro His Ala Pro Leu Leu Met Pro Leu Leu
        195                 200                 205

Pro Gln Cys Asp Pro Gln Glu Arg Met Ala Val Phe Gln Leu Tyr Leu
    210                 215                 220

Leu Ile Val Gln Lys Ser Pro Glu Val Leu Glu Ser Cys Val Pro Gln
225                 230                 235                 240

Leu Cys Gly Ser Leu Leu Asp Ser Asp Thr Ser Ser Ile Thr Met Gln
                245                 250                 255

Ile Leu Leu Lys Leu Ser Gln His Ser Pro Ser Leu Leu Val Asp His
            260                 265                 270

Phe Glu Gln Leu Arg Leu Ala Ala Lys Thr Asn Pro Gly Thr Ile Pro
        275                 280                 285

Leu Cys Ala Gln Ile Met Thr Thr Ala Gly Ile Gly Ser Lys Glu Thr
    290                 295                 300

Ala Gln Gln Ala Leu Asp Phe Val Leu Glu His Leu Pro Thr Gln Ala
305                 310                 315                 320

Leu Leu Gln Gln Glu Ala Thr Arg Leu Cys Ser Ala Tyr Pro Val Leu
                325                 330                 335

Phe Thr Asp Lys Val Leu Ala Cys Val Arg Gln Lys Asn Ala Ala Leu
            340                 345                 350

Ser Ser Gln Gln Asp Val Gly Asn Gly Leu Ala Asn Lys Thr Ser Gly
        355                 360                 365

Gly Val Thr Ile Val Ser Leu Asn Ser Ser Ser Pro Ser Pro Pro Leu
    370                 375                 380

Lys Pro Val Gly Ala Pro Ala Ile Asn Thr Thr Ile Val Gln Pro Ala
385                 390                 395                 400

Ser Val Val Ser Ala Thr Pro Thr Pro Pro Pro Ser Ser Ala Ser Ala
                405                 410                 415

Pro Ile Ala Ser Ser Thr Pro Val Ser Ala Ala Thr Pro Thr Pro Gln
            420                 425                 430

His Ala Gly Tyr Thr Arg Arg Val Lys Leu Gly Asp Ser Arg Ser Thr

435                 440                 445

Gly Arg Leu His Pro Ala Ser Asn Thr His Arg Ser Val Thr Arg Leu
    450                 455                 460

Asn Val Ala Ser Gly Ser Val Gly Gly Leu His Lys Ser Met Thr Arg
465                 470                 475                 480

Leu Ser Asn Ser Gln Ile Asn Gln Gln Pro Gly Gly Ser Ser Asn Gly
                485                 490                 495

Asn Val Val Val Gln Ser Gly Ala Thr Pro Lys Thr Pro Thr Gly Leu
            500                 505                 510

Ser Asn Pro Pro Val Thr Pro Val Pro Pro Leu Ser Asn Asn Val Val
        515                 520                 525

Ile Thr Gly His Asn Arg His Gly Ile Pro Val Thr Ser Gly Gly Val
    530                 535                 540

Thr Val Thr Thr Ser Pro Ser Lys Val Arg Pro His Ser Gln Gly Pro
545                 550                 555                 560

Ser Thr Leu Leu Asn Ser Ser Thr Val Leu Met Lys Tyr Ser Thr Asp
                565                 570                 575

Ala Leu Asn Gln Ser Val Gly Ser Ile Ser Ile Pro Gln Ser Ala Ala
            580                 585                 590

Val Ala Thr Leu Pro Ser Thr Gln Thr Gln Asn Ala Val Ser Val His
        595                 600                 605

His Ala Ser Pro Ala Leu Pro Gln Ser Ala Ser Asn Gly His Ala Lys
    610                 615                 620

Ser Val Met Lys Leu Pro Val Asn Gly Asn Ser Glu Val Ile Val Ser
625                 630                 635                 640

Gly Pro Thr Thr Asn Val Ala Pro Arg Arg Ser Asp Asn Thr Ser Arg
                645                 650                 655

Thr Leu Leu Asn Ala Asn Ser Val Met Asp Gln Arg Met Ser Thr Phe
            660                 665                 670

Glu Pro Tyr Gln Ile Met Arg Asp Pro Val Gln Gln Phe Cys Glu Lys
        675                 680                 685

Asn Phe Asn Ser Ile Lys Ser Tyr Met Asp Glu Val Ser Gln His Leu
    690                 695                 700

Pro Pro Pro Thr Arg Cys Ser Ile Glu Val Ser Asn Glu Phe Ala Glu

705                 710                 715                 720

Arg Arg Thr Lys Lys Val Ala Lys Leu His Phe Ala Cys Gln Ile Arg
                725                 730                 735

Gly Pro His Cys Leu Tyr Ser Lys Thr Cys Phe Thr Met Arg Thr Arg
            740                 745                 750

Asn Pro Lys Thr Trp Ile His Met Met Phe Leu Asp Phe Gln Val Arg
        755                 760                 765

His Phe Val Lys Glu Lys Cys Val Leu Ser Thr Arg Glu Ser Gly Ile
    770                 775                 780

Ser Asn Leu Lys Asn Ile Trp Gln Ile Leu Lys Cys Glu Asn Arg Ser
785                 790                 795                 800

Phe Thr Glu Leu Val Thr Ser Gln Phe Pro Gln Val Lys Asp Arg Glu
                805                 810                 815

Ile Leu Val Asn Glu Leu Arg His Ser Gly Phe Leu Asp Val Phe Glu
            820                 825                 830

Val Ser Lys Thr Asp Lys Ser Asn Pro Asn Cys Asn Glu Leu Glu Tyr
        835                 840                 845

Gln Trp Gly Cys Phe Leu Cys Asn His Pro Asp Lys Ala Val Gly Phe
    850                 855                 860

Leu Asn Gly Ser Asn Gln Pro Met Ile Glu Gly Gln Leu Lys Glu Lys
865                 870                 875                 880

Lys Gly Lys Trp Arg Leu Phe Arg Arg Trp Arg Thr Arg Tyr Phe Thr
                885                 890                 895

Leu Ser Gly Ala His Leu Ser Cys Lys Gly Ser Ser Gly Gly Glu Ser
            900                 905                 910

Ile Asp Val Asn Gln Ile Arg Ser Val Lys Val Ser Arg Gly Ala Arg
        915                 920                 925

Asn Ile Pro Lys Ala Phe Glu Ile Phe Thr Ala Asp Gln Thr Leu Ile
    930                 935                 940

Leu Lys Pro Lys Asp Gly Lys Asn Ala Glu Glu Trp Val Gln Cys Leu
945                 950                 955                 960

Ser Ile Val Val Ala His Ser Gln Ala Arg Asp Asn Pro Thr Ala Lys
                965                 970                 975

Thr Asn Ser Leu Pro Ala Arg Ser Ile Gly Ser Ser Lys Pro Ser Phe

980                 985                 990

<210>  8
<211>  988
<212>  PRT
<213>  Drosophila CG8624-PB

<400>  8

Met His Glu Leu Phe Asn Lys Val Leu Ala Lys Arg Asp Leu Ser Arg
1               5                   10                  15

Ala Gly Asp Leu Phe Ser Val Pro Asp Ala Asp Ile Val Asp Asp Ile
            20                  25                  30

Thr Glu Val Leu Ser Glu Ile Ser Pro Ile Ile Ser His Ala Asp Tyr
        35                  40                  45

Val Lys Asn Asn Asn Asp Gln Ser Val Val Glu Ile Cys Val Thr Arg
    50                  55                  60

Val Leu Ser Cys Ile Arg Glu Thr Lys Thr Ala Glu Arg Tyr Cys Ala
65                  70                  75                  80

Ala Leu Val Asp Leu Leu Arg Thr Cys Leu Leu Trp Asn Leu Gln Pro
                85                  90                  95

Ser Gly Thr Thr Lys Glu Glu Pro Pro His Ala Lys Ile Ala Ala Asp
            100                 105                 110

Ile Ile Ser Ser Ile Phe Leu Asn Tyr Asp Lys Lys Glu Leu Met Lys
        115                 120                 125

Ile Ala Leu Pro Val Ala Val Gln Phe Leu Pro Lys Gly Asn Arg Glu
    130                 135                 140

Leu Ser Arg Asn Leu Ala Ser Tyr Leu Ser Leu Ala Ala Ile Asp His
145                 150                 155                 160

Ala Gly Leu Leu Ser Pro His Thr Glu Ser Val Met Glu Ser Ile Leu
                165                 170                 175

Ser Gly Asn Tyr Gly Leu Leu Arg Val Leu Ser Gln Val Tyr Glu Val
            180                 185                 190

Ala Pro Glu Ala Val Thr Pro His Ala Pro Leu Leu Met Pro Leu Leu
        195                 200                 205

Pro Gln Cys Asp Pro Gln Glu Arg Met Ala Val Phe Gln Leu Tyr Leu
    210                 215                 220

Leu Ile Val Gln Lys Ser Pro Glu Val Leu Glu Ser Cys Val Pro Gln
225                 230                 235                 240

Leu Cys Gly Ser Leu Leu Asp Ser Asp Thr Ser Ser Ile Thr Met Gln
                245                 250                 255

Ile Leu Leu Lys Leu Ser Gln His Ser Pro Ser Leu Leu Val Asp His
            260                 265                 270

Phe Glu Gln Leu Arg Leu Ala Ala Lys Thr Asn Pro Gly Thr Ile Pro
        275                 280                 285

Leu Cys Ala Gln Ile Met Thr Thr Ala Gly Ile Gly Ser Lys Glu Thr
    290                 295                 300

Ala Gln Gln Ala Leu Asp Phe Val Leu Glu His Leu Pro Thr Gln Ala
305                 310                 315                 320

Leu Leu Gln Gln Glu Ala Thr Arg Leu Cys Ser Ala Tyr Pro Val Leu
                325                 330                 335

Phe Thr Asp Lys Val Leu Ala Cys Val Arg Gln Lys Asn Ala Ala Leu
            340                 345                 350

Ser Ser Gln Gln Asp Val Gly Asn Gly Leu Ala Asn Lys Thr Ser Gly
        355                 360                 365

Gly Val Thr Ile Val Ser Leu Asn Ser Ser Ser Pro Ser Pro Pro Leu
    370                 375                 380

Lys Pro Val Pro Val Gly Ala Pro Ala Ile Asn Thr Thr Ile Val Gln
385                 390                 395                 400

Pro Ala Ser Val Val Ser Ala Thr Pro Thr Pro Pro Pro Ser Ser Ala
                405                 410                 415

Ser Ala Pro Ile Ala Ser Ser Thr Pro Val Ser Ala Ala Thr Pro Thr
            420                 425                 430

Pro Gln His Ala Gly Tyr Thr Arg Arg Val Lys Leu Gly Asp Ser Arg
        435                 440                 445

Ser Thr Gly Arg Leu His Pro Ala Ser Asn Thr His Arg Ser Val Thr
    450                 455                 460

Arg Leu Asn Val Ala Ser Gly Ser Val Gly Gly Leu His Lys Ser Met
465                 470                 475                 480

Thr Arg Leu Ser Asn Ser Gln Ile Asn Gln Gln Pro Gly Gly Ser Ser
                485                 490                 495

Asn Gly Asn Val Val Val Gln Ser Gly Ala Thr Pro Lys Thr Pro Thr
            500                 505                 510

Gly Leu Ser Asn Pro Pro Val Thr Pro Val Pro Pro Leu Ser Asn Asn
        515                 520                 525

Val Val Ile Thr Gly His Asn Arg His Gly Ile Pro Val Thr Ser Gly
    530                 535                 540

Gly Val Thr Val Thr Thr Ser Pro Ser Lys Val Arg Pro His Ser Gln
545                 550                 555                 560

Gly Pro Ser Thr Leu Leu Asn Ser Ser Thr Val Leu Met Lys Tyr Ser
                565                 570                 575

Thr Asp Ala Leu Asn Gln Ser Val Gly Ser Ile Ser Ile Pro Gln Ser
            580                 585                 590

Ala Ala Val Ala Thr Leu Pro Ser Thr Gln Thr Gln Asn Ala Val Ser
        595                 600                 605

Val His His Ala Ser Pro Ala Leu Pro Gln Ser Ala Ser Asn Gly His
    610                 615                 620

Ala Lys Ser Val Met Lys Leu Pro Val Asn Gly Asn Ser Glu Val Ile
625                 630                 635                 640

Val Ser Gly Pro Thr Thr Asn Val Ala Pro Arg Arg Ser Asp Asn Thr
                645                 650                 655

Ser Arg Thr Leu Leu Asn Ala Asn Ser Val Met Asp Gln Arg Met Ser
            660                 665                 670

Thr Phe Glu Pro Tyr Gln Ile Met Arg Asp Pro Val Gln Gln Phe Cys
        675                 680                 685

Glu Lys Asn Phe Asn Ser Ile Lys Ser Tyr Met Asp Glu Val Ser Gln
    690                 695                 700

His Leu Pro Pro Pro Thr Arg Cys Ser Ile Glu Glu Arg Arg Thr Lys
705                 710                 715                 720

Lys Val Ala Lys Leu His Phe Ala Cys Gln Ile Arg Gly Pro His Cys
                725                 730                 735

Leu Tyr Ser Lys Thr Cys Phe Thr Met Arg Thr Arg Asn Pro Lys Thr
            740                 745                 750

Trp Ile His Met Met Phe Leu Asp Phe Gln Val Arg His Phe Val Lys
        755                 760                 765

Glu Lys Cys Val Leu Ser Thr Arg Glu Ser Gly Ile Ser Asn Leu Lys
    770                 775                 780

Asn Ile Trp Gln Ile Leu Lys Cys Glu Asn Arg Ser Phe Thr Glu Leu
785                 790                 795                 800

Val Thr Ser Gln Phe Pro Gln Val Lys Asp Arg Glu Ile Leu Val Asn
                805                 810                 815

Glu Leu Arg His Ser Gly Phe Leu Asp Val Phe Glu Val Ser Lys Thr
            820                 825                 830

Asp Lys Ser Asn Pro Asn Cys Asn Glu Leu Glu Tyr Gln Trp Gly Cys
        835                 840                 845

Phe Leu Cys Asn His Pro Asp Lys Ala Val Gly Phe Leu Asn Gly Ser
    850                 855                 860

Asn Gln Pro Met Ile Glu Gly Gln Leu Lys Glu Lys Lys Gly Lys Trp
865                 870                 875                 880

Arg Leu Phe Arg Arg Trp Arg Thr Arg Tyr Phe Thr Leu Ser Gly Ala
                885                 890                 895

His Leu Ser Cys Lys Gly Ser Ser Gly Gly Glu Ser Ile Asp Val Asn
            900                 905                 910

Gln Ile Arg Ser Val Lys Val Ser Arg Gly Ala Arg Asn Ile Pro Lys
        915                 920                 925

Ala Phe Glu Ile Phe Thr Ala Asp Gln Thr Leu Ile Leu Lys Pro Lys
    930                 935                 940

Asp Gly Lys Asn Ala Glu Glu Trp Val Gln Cys Leu Ser Ile Val Val
945                 950                 955                 960

Ala His Ser Gln Ala Arg Asp Asn Pro Thr Ala Lys Thr Asn Ser Leu
                965                 970                 975

Pro Ala Arg Ser Ile Gly Ser Ser Lys Pro Ser Phe
            980                 985

<210>   9
<211>   989
<212>   PRT
<213>   Drosophila

<400>   9

Met His Glu Leu Phe Asn Lys Val Leu Ala Lys Arg Asp Leu Ser Arg
1               5                   10                  15

Ala Gly Asp Leu Phe Ser Val Pro Asp Ala Asp Ile Val Asp Asp Ile
            20                  25                  30

Thr Glu Val Leu Ser Glu Ile Ser Pro Ile Ile Ser His Ala Asp Tyr
        35                  40                  45

Val Lys Asn Asn Asn Asp Gln Ser Val Val Glu Ile Cys Val Thr Arg
    50                  55                  60

Val Leu Ser Cys Ile Arg Glu Thr Lys Thr Ala Glu Arg Tyr Cys Ala
65                  70                  75                  80

Ala Leu Val Asp Leu Leu Arg Thr Cys Leu Leu Trp Asn Leu Gln Pro
                85                  90                  95

Ser Gly Thr Thr Lys Glu Glu Pro Pro His Ala Lys Ile Ala Ala Asp
            100                 105                 110

Ile Ile Ser Ser Ile Phe Leu Asn Tyr Asp Lys Lys Glu Leu Met Lys
        115                 120                 125

Ile Ala Leu Pro Val Ala Val Gln Phe Leu Pro Lys Gly Asn Arg Glu
    130                 135                 140

Leu Ser Arg Asn Leu Ala Ser Tyr Leu Ser Leu Ala Ala Ile Asp His
145                 150                 155                 160

Ala Gly Leu Leu Ser Pro His Thr Glu Ser Val Met Glu Ser Ile Leu
                165                 170                 175

Ser Gly Asn Tyr Gly Leu Leu Arg Val Leu Ser Gln Val Tyr Glu Val
            180                 185                 190

Ala Pro Glu Ala Val Thr Pro His Ala Pro Leu Leu Met Pro Leu Leu
        195                 200                 205

Pro Gln Cys Asp Pro Gln Glu Arg Met Ala Val Phe Gln Leu Tyr Leu
    210                 215                 220

Leu Ile Val Gln Lys Ser Pro Glu Val Leu Glu Ser Cys Val Pro Gln
225                 230                 235                 240

Leu Cys Gly Ser Leu Leu Asp Ser Asp Thr Ser Ser Ile Thr Met Gln
                245                 250                 255

Ile Leu Leu Lys Leu Ser Gln His Ser Pro Ser Leu Leu Val Asp His
            260                 265                 270

Phe Glu Gln Leu Arg Leu Ala Ala Lys Thr Asn Pro Gly Thr Ile Pro
        275                 280                 285

Leu Cys Ala Gln Ile Met Thr Thr Ala Gly Ile Gly Ser Lys Glu Thr
    290                 295                 300

Ala Gln Gln Ala Leu Asp Phe Val Leu Glu His Leu Pro Thr Gln Ala
305                 310                 315                 320

Leu Leu Gln Gln Glu Ala Thr Arg Leu Cys Ser Ala Tyr Pro Val Leu
                325                 330                 335

Phe Thr Asp Lys Val Leu Ala Cys Val Arg Gln Lys Asn Ala Ala Leu
            340                 345                 350

Ser Ser Gln Gln Asp Val Gly Asn Gly Leu Ala Asn Lys Thr Ser Gly
        355                 360                 365

Gly Val Thr Ile Val Ser Leu Asn Ser Ser Ser Pro Ser Pro Pro Leu
    370                 375                 380

Lys Pro Val Pro Val Gly Ala Pro Ala Ile Asn Thr Thr Ile Val Gln
385                 390                 395                 400

Pro Ala Ser Val Val Ser Ala Thr Pro Thr Pro Pro Pro Ser Ser Ala
                405                 410                 415

Ser Ala Pro Ile Ala Ser Ser Thr Pro Val Ser Ala Ala Thr Pro Thr
            420                 425                 430

Pro Gln His Ala Gly Tyr Thr Arg Arg Val Lys Leu Gly Asp Ser Arg
        435                 440                 445

Ser Thr Gly Arg Leu His Pro Ala Ser Asn Thr His Arg Ser Val Thr
    450                 455                 460

Arg Leu Asn Val Ala Ser Gly Ser Val Gly Gly Leu His Lys Ser Met
465                 470                 475                 480

Thr Arg Leu Ser Asn Ser Gln Ile Asn Gln Gln Pro Gly Gly Ser Ser
                485                 490                 495

Asn Gly Asn Val Val Val Gln Ser Gly Ala Thr Pro Lys Thr Pro Thr
            500                 505                 510

Gly Leu Ser Asn Pro Pro Val Thr Pro Val Pro Pro Leu Ser Asn Asn
        515                 520                 525

Val Val Ile Thr Gly His Asn Arg His Gly Ile Pro Val Thr Ser Gly
    530                 535                 540

Gly Val Thr Val Thr Thr Ser Pro Ser Lys Val Arg Pro His Ser Gln
545                 550                 555                 560

Gly Pro Ser Thr Leu Leu Asn Ser Ser Thr Val Leu Met Lys Tyr Ser
                565                 570                 575

Thr Asp Ala Leu Asn Gln Ser Val Gly Ser Ile Ser Ile Pro Gln Ser
            580                 585                 590

Ala Ala Val Ala Thr Leu Pro Ser Thr Gln Thr Gln Asn Ala Val Ser
        595                 600                 605

Val His His Ala Ser Pro Ala Leu Pro Gln Ser Ala Ser Asn Gly His
    610                 615                 620

Ala Lys Ser Val Met Lys Leu Pro Val Asn Gly Asn Ser Glu Val Ile
625                 630                 635                 640

Val Ser Gly Pro Thr Thr Asn Val Ala Pro Arg Arg Ser Asp Asn Thr
                645                 650                 655

Ser Arg Thr Leu Leu Asn Ala Asn Ser Val Met Asp Gln Arg Met Ser
            660                 665                 670

Thr Phe Glu Pro Tyr Gln Ile Met Arg Asp Pro Val Gln Gln Phe Cys
        675                 680                 685

Glu Lys Asn Phe Asn Ser Ile Lys Ser Tyr Met Asp Glu Val Ser Gln
    690                 695                 700

His Leu Pro Pro Pro Thr Arg Cys Ser Ile Glu Glu Arg Arg Thr Lys
705                 710                 715                 720

Lys Val Ala Lys Leu His Phe Ala Cys Gln Ile Arg Gly Pro His Cys
                725                 730                 735

Leu Tyr Ser Lys Thr Cys Phe Thr Met Arg Thr Arg Asn Pro Lys Thr
            740                 745                 750

Trp Ile His Met Met Phe Leu Asp Phe Gln Val Arg His Phe Val Lys
        755                 760                 765

Glu Lys Cys Val Leu Ser Thr Arg Glu Ser Gly Ile Ser Asn Leu Lys
    770                 775                 780

Asn Ile Trp Gln Ile Leu Lys Cys Glu Asn Arg Ser Phe Thr Glu Leu
785                 790                 795                 800

Val Thr Ser Gln Phe Pro Gln Val Lys Asp Arg Glu Ile Leu Val Asn
                805                 810                 815

Glu Leu Arg His Ser Gly Phe Leu Asp Val Phe Glu Val Ser Lys Thr
            820                 825                 830

Asp Lys Ser Asn Pro Asn Cys Asn Glu Leu Glu Tyr Gln Trp Gly Cys
        835                 840                 845

Phe Leu Cys Asn His Pro Asp Lys Ala Val Gly Phe Leu Asn Gly Ser
    850                 855                 860

Asn Gln Pro Met Ile Glu Gly Gln Leu Lys Glu Lys Lys Gly Lys Trp
865                 870                 875                 880

Arg Leu Phe Arg Arg Trp Arg Thr Arg Tyr Phe Thr Leu Ser Gly Ala
                885                 890                 895

His Leu Ser Cys Lys Gly Ser Ser Gly Gly Glu Ser Ile Asp Val Asn
            900                 905                 910

Gln Ile Arg Ser Val Lys Val Ser Arg Gly Ala Arg Asn Ile Pro Lys
        915                 920                 925

Ala Phe Glu Ile Phe Thr Ala Asp Gln Thr Leu Ile Leu Lys Pro Lys
    930                 935                 940

Asp Gly Lys Asn Ala Glu Glu Trp Val Gln Cys Leu Ser Ile Val Val
945                 950                 955                 960

Ala His Ser Gln Ala Arg Asp Asn Pro Thr Ala Lys Thr Asn Ser Leu
                965                 970                 975

Pro Ala Arg Ser Ile Gly Ser Ser Lys Pro Ser Phe Glx
            980                 985

<210>   10
<211>   834
<212>   PRT
<213>   Homo sapiens

<400>   10

Met His Gln Leu Phe Arg Leu Val Leu Gly Gln Lys Asp Leu Ser Arg
1               5                   10                  15

Ala Gly Asp Leu Phe Ser Leu Asp Asp Ser Glu Ile Glu Asp Ser Leu
            20                  25                  30

Thr Glu Ala Leu Glu Gln Ile Lys Ile Ile Ser Ser Ser Ser Asp Tyr
        35                  40                  45

Gln Thr Asn Asn Asn Asp Gln Ala Val Val Glu Ile Cys Ile Thr Arg
    50                  55                  60

Ile Thr Thr Ala Ile Arg Glu Thr Glu Ser Ile Glu Lys His Ala Lys
65                  70                  75                  80

Ala Leu Val Gly Leu Trp Asp Ser Cys Leu Glu His Asn Leu Arg Pro
                85                  90                  95

Phe Gly Lys Asp Glu Asp Thr Pro His Ala Lys Ile Ala Ser Asp Ile
            100                 105                 110

Met Ser Cys Ile Leu Gln Asn Tyr Asn Arg Pro Pro Val Met Ala Leu
        115                 120                 125

Ala Ile Pro Ile Ala Val Lys Phe Leu His Arg Gly Asn Lys Glu Leu
    130                 135                 140

Cys Arg Asn Met Ser Asn Tyr Leu Ser Leu Ala Ala Ile Thr Lys Ala
145                 150                 155                 160

Asp Leu Leu Ala Asp His Thr Glu Val Ile Val Lys Ser Ile Leu Gln
                165                 170                 175

Gly Asn Thr Met Leu Leu Arg Val Leu Pro Ala Val Tyr Glu Lys Gln
            180                 185                 190

Pro Gln Pro Ile Asn Arg His Leu Thr Glu Leu Leu Ala Leu Met Ser
        195                 200                 205

Gln Leu Glu Gln Pro Glu Gln Tyr His Leu Leu Arg Leu Leu His Val
    210                 215                 220

Ala Ala Lys Lys Lys Gln Leu Glu Val Val Gln Lys Cys Ile Pro Phe
225                 230                 235                 240

Leu Ile Gly His Leu Lys Asp Ser Thr His Asn Asp Ile Ile Leu Asn
                245                 250                 255

Ile Leu Ile Glu Ile Ala Val Tyr Glu Pro Val Ala Leu Asn Ser Phe
            260                 265                 270

Leu Pro Met Leu Lys Glu Ile Gly Glu Arg Phe Pro Tyr Leu Thr Gly
        275                 280                 285

Gln Met Ala Arg Ile Tyr Gly Ala Val Gly His Val Asp Glu Glu Arg
    290                 295                 300

Ala Arg Ser Cys Leu Thr Tyr Leu Val Ser Gln Leu Ala Asn Met Glu
305                 310                 315                 320

His Ser Phe His His Ile Leu Leu Leu Glu Ile Lys Ser Ile Thr Asp
                325                 330                 335

Thr Phe Ser Ser Ile Leu Gly Pro Gln Ser Arg Asp Ile Phe Arg Met
            340                 345                 350

Ser Asn Ser Phe Thr Ala Ile Ala Lys Leu Leu Thr Arg Gln Leu Glu
        355                 360                 365

Asn Thr Lys Ala Gly Ser Gly Arg Arg Lys Ile Ser Thr Glu Ile Glu
    370                 375                 380

Phe Pro Glu Lys Leu Glu Glu Thr Lys Leu Ile Val Thr Glu Asn Glu
385                 390                 395                 400

Asp His Glu Lys Leu Gln Val Lys Ile Gln Ala Phe Glu Asp Lys Ile
                405                 410                 415

Asn Ala Gly Ser Asn Thr Pro Gly Ser Ile Arg Arg Tyr Ser Leu Gly
            420                 425                 430

Gln Val Ser Lys Glu Glu Arg Lys Asn Ile Arg Phe Asn Arg Ser Lys
        435                 440                 445

Ser Leu Ala Phe His Thr Met Leu Thr Lys Gly Val Gly Ser Asp Asp
    450                 455                 460

Gly Glu Asp Glu Asn Arg Gly Asp Ile Pro Ala Ser Ile Ser Leu Ser
465                 470                 475                 480

Glu Ile Asp Pro Leu Gly Gln Gly Asn Asp Lys Leu Pro Phe Lys Thr
                485                 490                 495

Asp Thr Glu Arg Ser Gln Leu Gly Glu Ser Ser Val Ser Tyr Pro Asn
            500                 505                 510

Ile Ile His Ile Asp Ser Glu Asn Leu Ser Glu Thr Val Lys Glu Asn
        515                 520                 525

Ser Gln Glu Glu Thr Pro Glu Thr Thr Ala Ser Pro Ile Glu Tyr Gln
    530                 535                 540

Asp Lys Leu Tyr Leu His Leu Lys Lys Asn Leu Ser Lys Val Lys Ala
545                 550                 555                 560

Tyr Ala Met Glu Ile Gly Lys Lys Ile Pro Val Pro Asp Gln Cys Thr
                565                 570                 575

Ile Glu Asp Thr Val Arg Ser Cys Val Ala Lys Leu Phe Phe Thr Cys
            580                 585                 590

Ser Leu Lys Gly His Tyr Cys Leu Tyr Ser Lys Ser Ser Phe Ile Leu
        595                 600                 605

Ile Ser Gln Glu Pro Gln Pro Trp Ile Gln Ile Met Phe Leu Phe Gln
    610                 615                 620

Gln Ser Leu Phe Pro Glu Pro Leu Ser Ile Gln Ser His Ser Val Gln
625                 630                 635                 640

Phe Leu Arg Ala Leu Trp Glu Lys Thr Gln Ala Gly Gly Ala His Ser
                645                 650                 655

Phe Glu Thr Ala Met Met Glu Ser Thr Phe Pro Gln Gln Lys Asp Leu
            660                 665                 670

Asp Gln Val Gln Leu His Leu Glu Glu Val Arg Phe Phe Asp Val Phe
        675                 680                 685

Gly Phe Ser Glu Thr Ala Gly Ala Trp Gln Cys Phe Met Cys Asn Asn
    690                 695                 700

Pro Glu Lys Ala Thr Val Val Asn Gln Asp Gly Gln Pro Leu Ile Glu
705                 710                 715                 720

Gly Lys Leu Lys Glu Lys Gln Val Arg Trp Lys Phe Ile Lys Arg Trp
                725                 730                 735

Lys Thr Arg Tyr Phe Thr Leu Ala Gly Asn Gln Leu Leu Phe Gln Lys
            740                 745                 750

Gly Lys Ser Lys Asp Asp Pro Asp Asp Cys Pro Ile Glu Leu Ser Lys
        755                 760                 765

Val Gln Ser Val Lys Ala Val Ala Lys Lys Arg Arg Asp Arg Ser Leu
    770                 775                 780

Pro Arg Ala Phe Glu Ile Phe Thr Asp Asn Lys Thr Tyr Val Phe Lys
785                 790                 795                 800

Ala Lys Asp Glu Lys Asn Ala Glu Glu Trp Leu Gln Cys Ile Asn Val
                805                 810                 815

Ala Val Ala Gln Ala Lys Glu Arg Glu Ser Arg Glu Val Thr Thr Tyr
            820                 825                 830

Leu Glx


<210>  11
<211>  833
<212>  PRT
<213>  Homo sapiens

<400>  11

Met His Gln Leu Phe Arg Leu Val Leu Gly Gln Lys Asp Leu Ser Arg
1               5                   10                  15

Ala Gly Asp Leu Phe Ser Leu Asp Asp Ser Glu Ile Glu Asp Ser Leu
            20                  25                  30

Thr Glu Ala Leu Glu Gln Ile Lys Ile Ile Ser Ser Ser Ser Asp Tyr

35                  40                  45

Gln Thr Asn Asn Asn Asp Gln Ala Val Val Glu Ile Cys Ile Thr Arg
    50                  55                  60

Ile Thr Thr Ala Ile Arg Glu Thr Glu Ser Ile Glu Lys His Ala Lys
65                  70                  75                  80

Ala Leu Val Gly Leu Trp Asp Ser Cys Leu Glu His Asn Leu Arg Pro
                85                  90                  95

Phe Gly Lys Asp Glu Asp Thr Pro His Ala Lys Ile Ala Ser Asp Ile
            100                 105                 110

Met Ser Cys Ile Leu Gln Asn Tyr Asn Arg Pro Pro Val Met Ala Leu
        115                 120                 125

Ala Ile Pro Ile Ala Val Lys Phe Leu His Arg Gly Asn Lys Glu Leu
    130                 135                 140

Cys Arg Asn Met Ser Asn Tyr Leu Ser Leu Ala Ala Ile Thr Lys Ala
145                 150                 155                 160

Asp Leu Leu Ala Asp His Thr Glu Val Ile Val Lys Ser Ile Leu Gln
                165                 170                 175

Gly Asn Thr Met Leu Leu Arg Val Leu Pro Ala Val Tyr Glu Lys Gln
            180                 185                 190

Pro Gln Pro Ile Asn Arg His Leu Thr Glu Leu Leu Ala Leu Met Ser
        195                 200                 205

Gln Leu Glu Gln Pro Glu Gln Tyr His Leu Leu Arg Leu Leu His Val
    210                 215                 220

Ala Ala Lys Lys Lys Gln Leu Glu Val Val Gln Lys Cys Ile Pro Phe
225                 230                 235                 240

Leu Ile Gly His Leu Lys Asp Ser Thr His Asn Asp Ile Ile Leu Asn
                245                 250                 255

Ile Leu Met Glu Ile Ala Val Tyr Glu Pro Val Ala Leu Asn Ser Phe
            260                 265                 270

Leu Pro Met Leu Lys Glu Ile Gly Glu Arg Phe Pro Tyr Leu Thr Gly
        275                 280                 285

Gln Met Ala Arg Ile Tyr Gly Ala Val Gly His Val Asp Glu Glu Arg
    290                 295                 300

Ala Arg Ser Cys Leu Thr Tyr Leu Val Ser Gln Leu Ala Asn Met Glu

305                 310                 315                 320

His Ser Phe His His Ile Leu Leu Leu Glu Ile Lys Ser Ile Thr Asp
                325                 330                 335

Thr Phe Ser Ser Ile Leu Gly Pro Gln Ser Arg Asp Ile Phe Arg Met
            340                 345                 350

Ser Asn Ser Phe Thr Ala Ile Ala Lys Leu Leu Thr Arg Gln Leu Glu
        355                 360                 365

Asn Thr Lys Ala Gly Ser Gly Arg Arg Lys Ile Ser Thr Glu Ile Glu
    370                 375                 380

Phe Pro Glu Lys Leu Glu Glu Thr Lys Leu Ile Val Thr Glu Asn Glu
385                 390                 395                 400

Asp His Glu Lys Leu Gln Val Lys Ile Gln Ala Phe Glu Asp Lys Ile
                405                 410                 415

Asn Ala Gly Ser Asn Thr Pro Gly Ser Ile Arg Arg Tyr Ser Leu Gly
            420                 425                 430

Gln Val Ser Lys Glu Glu Arg Lys Asn Ile Arg Phe Asn Arg Ser Lys
        435                 440                 445

Ser Leu Ala Phe His Thr Met Leu Thr Lys Gly Val Gly Ser Asp Asp
    450                 455                 460

Gly Glu Asp Glu Asn Arg Gly Asp Ile Pro Ala Ser Ile Ser Leu Ser
465                 470                 475                 480

Glu Ile Asp Pro Leu Gly Gln Gly Asn Asp Lys Leu Pro Phe Lys Thr
                485                 490                 495

Asp Thr Glu Arg Ser Gln Leu Gly Glu Ser Ser Val Ser Tyr Pro Asn
            500                 505                 510

Ile Ile His Ile Asp Ser Glu Asn Leu Ser Glu Thr Val Lys Glu Asn
        515                 520                 525

Ser Gln Glu Glu Thr Pro Glu Thr Thr Ala Ser Pro Ile Glu Tyr Gln
    530                 535                 540

Asp Lys Leu Tyr Leu His Leu Lys Lys Asn Leu Ser Lys Val Lys Ala
545                 550                 555                 560

Tyr Ala Met Glu Ile Gly Lys Lys Ile Pro Val Pro Asp Gln Cys Thr
                565                 570                 575

Ile Glu Asp Thr Val Arg Ser Cys Val Ala Lys Leu Phe Phe Thr Cys

            580                 585                 590

Ser Leu Lys Gly His Tyr Cys Leu Tyr Ser Lys Ser Ser Phe Ile Leu
        595                 600                 605

Ile Ser Gln Glu Pro Gln Pro Trp Ile Gln Ile Met Phe Leu Phe Gln
    610                 615                 620

Gln Ser Leu Phe Pro Glu Pro Leu Ser Ile Gln Ser His Ser Val Gln
625                 630                 635                 640

Phe Leu Arg Ala Leu Trp Glu Lys Thr Gln Ala Gly Gly Ala His Ser
                645                 650                 655

Phe Glu Thr Ala Met Met Glu Ser Thr Phe Pro Gln Gln Lys Asp Leu
            660                 665                 670

Asp Gln Val Gln Leu His Leu Glu Glu Val Arg Phe Phe Asp Val Phe
        675                 680                 685

Gly Phe Ser Glu Thr Ala Gly Ala Trp Gln Cys Phe Met Cys Asn Asn
    690                 695                 700

Pro Glu Lys Ala Thr Val Val Asn Gln Asp Gly Gln Pro Leu Ile Glu
705                 710                 715                 720

Gly Lys Leu Lys Glu Lys Gln Val Arg Trp Lys Phe Ile Lys Arg Trp
                725                 730                 735

Lys Thr Arg Tyr Phe Thr Leu Ala Gly Asn Gln Leu Leu Phe Gln Lys
            740                 745                 750

Gly Lys Ser Lys Asp Asp Pro Asp Asp Cys Pro Ile Glu Leu Ser Lys
        755                 760                 765

Val Gln Ser Val Lys Ala Val Ala Lys Lys Arg Arg Asp Arg Ser Leu
    770                 775                 780

Pro Arg Ala Phe Glu Ile Phe Thr Asp Asn Lys Thr Tyr Val Phe Lys
785                 790                 795                 800

Ala Lys Asp Glu Lys Asn Ala Ala Glu Trp Leu Gln Cys Ile Asn Val
                805                 810                 815

Ala Val Ala Gln Ala Lys Glu Arg Glu Ser Arg Glu Val Thr Thr Tyr
            820                 825                 830

Leu

<210> 12

<211>  833
<212>  PRT
<213>  Mus musculus

<400>  12

Met His Gln Leu Phe Arg Leu Val Leu Gly Gln Lys Asp Leu Ser Lys
1               5                   10                  15

Ala Gly Asp Leu Phe Ser Leu Asp Asp Ala Glu Ile Glu Asp Ser Leu
            20                  25                  30

Thr Glu Ala Leu Glu Gln Ile Lys Val Ile Ser Ser Ser Leu Asp Tyr
        35                  40                  45

Gln Thr Asn Asn Asn Asp Gln Ala Val Val Glu Ile Cys Ile Thr Arg
    50                  55                  60

Ile Thr Thr Ala Ile Arg Glu Thr Glu Ser Ile Glu Lys His Ala Arg
65                  70                  75                  80

Ala Leu Val Gly Leu Trp Asp Ser Cys Leu Glu His Asn Leu Arg Pro
                85                  90                  95

Ala Gly Lys Asp Glu Asp Thr Pro His Ala Lys Ile Ala Ser Asp Ile
            100                 105                 110

Met Ser Cys Ile Leu Gln Asn Tyr Asn Arg Thr Pro Val Met Val Leu
        115                 120                 125

Ala Val Pro Ile Ala Val Lys Phe Leu His Arg Gly Ser Lys Glu Leu
    130                 135                 140

Cys Arg Asn Met Ser Asn Tyr Leu Ser Leu Ala Ala Ile Thr Lys Ala
145                 150                 155                 160

Asp Leu Leu Ala Asp His Thr Glu Gly Ile Ile Lys Ser Ile Leu Gln
                165                 170                 175

Gly Asn Ala Met Leu Leu Arg Val Leu Pro Ala Val Tyr Glu Lys Gln
            180                 185                 190

Pro Gln Pro Ile Asn Arg His Leu Ala Glu Leu Leu Ala Leu Met Ser
        195                 200                 205

Gln Leu Glu Gln Thr Glu Gln Tyr His Leu Leu Arg Leu Leu His Val
    210                 215                 220

Ala Ala Lys Arg Lys Asp Val Glu Val Val Gln Lys Cys Val Pro Phe
225                 230                 235                 240

Leu Ile Arg Asn Leu Lys Asp Ser Thr Tyr Asn Asp Ile Ile Leu Asn
                245                 250                 255

Ile Leu Ile Glu Ile Ala Gly His Glu Pro Leu Ala Leu Asn Ser Phe
            260                 265                 270

Leu Pro Met Leu Lys Glu Ile Ala Glu Gln Phe Pro Tyr Leu Thr Gly
        275                 280                 285

Gln Met Ala Arg Ile Phe Gly Ala Val Gly His Val Asp Glu Glu Arg
    290                 295                 300

Ala Arg Ser Cys Leu Arg Tyr Leu Val Ser Gln Leu Ala Asn Met Glu
305                 310                 315                 320

His Pro Phe His His Ile Leu Leu Leu Glu Ile Lys Ser Ile Thr Asp
                325                 330                 335

Ala Phe Ser Ser Ile Leu Gly Pro His Ser Arg Asp Ile Phe Arg Met
            340                 345                 350

Ser Asn Ser Phe Thr Asn Ile Ala Lys Leu Leu Ser Arg Gln Leu Glu
        355                 360                 365

Asn Ser Lys Ala Asp Ser Ser Arg Arg Lys Thr Ser Thr Glu Val Ser
    370                 375                 380

Ile Pro Glu Lys Leu Arg Glu Leu Asn Ser Met Glu Pro Glu Ser Glu
385                 390                 395                 400

Asp His Glu Lys Leu Gln Val Lys Ile Gln Ala Phe Glu Asp Lys Ile
                405                 410                 415

Asn Ala Glu Ser Asn Thr Pro Gly Ser Gly Arg Arg Tyr Ser Leu Asp
            420                 425                 430

His Ile Ser Lys Glu Glu Arg Lys Ser Ile Arg Phe Ser Arg Ser Arg
        435                 440                 445

Ser Leu Ala Leu Asn Thr Val Leu Thr Asn Gly Val Ser Val Glu Asp
    450                 455                 460

Asn Glu Val Glu Glu Lys Ala Gly Met His Ala Ser Ile Ser Leu Ser
465                 470                 475                 480

Gln Ile Asp Pro Leu Ser His Gly Ile Gly Lys Leu Pro Phe Lys Thr
                485                 490                 495

Asp Thr His Gly Ser Pro Leu Arg Asn Ser Ser Ala Ser His Pro Ser
            500                 505                 510

Ile Ile His Thr Glu Pro Glu Thr Met Pro Glu Thr Phe Lys Glu Asn
        515                 520                 525

Ile Gln Glu Glu Ile Leu Glu Ala Ala Thr Ser Pro Ile Glu Tyr Gln
    530                 535                 540

Asp Lys Leu Tyr Leu His Leu Arg Glu Asn Leu Ser Lys Val Lys Ala
545                 550                 555                 560

Tyr Ala Leu Glu Ile Ala Lys Lys Val Pro Ile Pro Asp Gln Cys Thr
                565                 570                 575

Ile Glu Asp Thr Met Arg Ser Cys Val Ala Lys Leu Phe Phe Thr Cys
            580                 585                 590

Ser Leu Lys Gly His Tyr Cys Leu Tyr Ser Lys Ser Ser Phe Ile Leu
        595                 600                 605

Val Ser Gln Ala Pro Gln Pro Trp Ile Gln Val Met Phe Leu Ser Gln
    610                 615                 620

Gln Ser Leu Phe Pro Glu Pro Leu Ser Ile Gln Ser Gly Ser Val Gln
625                 630                 635                 640

Phe Leu Lys Ala Leu Trp Glu Lys Thr Gln Asp Thr Gly Ala His Ser
                645                 650                 655

Phe Glu Val Ala Met Thr Glu Ser Thr Phe Pro Gln Gln Lys Asp Leu
            660                 665                 670

Glu Gln Leu Gln Leu His Leu Glu Glu Val Arg Phe Phe Asp Val Phe
        675                 680                 685

Gly Phe Ser Glu Thr Ala Gly Ala Trp Gln Cys Phe Met Cys Asn Asn
    690                 695                 700

Pro Glu Lys Ala Thr Val Val Asn Gln Asp Gly Gln Pro Leu Ile Glu
705                 710                 715                 720

Gly Lys Leu Lys Glu Lys Gln Val Arg Trp Lys Phe Ile Lys Arg Trp
                725                 730                 735

Lys Thr Arg Tyr Phe Thr Leu Ala Gly Asn Gln Leu Leu Phe Gln Lys
            740                 745                 750

Gly Lys Ser Lys Asp Asp Pro Asp Asp Pro Pro Ile Glu Leu Ser Lys
        755                 760                 765

Val Gln Ser Val Lys Ala Val Ala Lys Lys Arg Arg Asp Arg Ser Leu
    770                 775                 780

Pro Arg Ala Phe Glu Ile Phe Thr Asp Ser Lys Thr Tyr Val Phe Lys
785                 790                 795                 800

Ala Lys Asp Glu Lys Asn Ala Glu Glu Trp Leu Gln Cys Ile Asn Val
                805                 810                 815

Ala Leu Ala Gln Ala Lys Glu Arg Glu Ser Arg Glu Val Thr Thr Tyr
            820                 825                 830

Leu

<210>  13
<211>  253
<212>  PRT
<213>  Mus musculus

<400>  13

Met Arg Ser Cys Val Ala Lys Leu Phe Phe Thr Cys Ser Leu Lys Gly
1               5                   10                  15

His Tyr Cys Leu Tyr Ser Lys Ser Ser Phe Ile Leu Val Ser Gln Ala
            20                  25                  30

Pro Gln Pro Trp Ile Gln Val Met Phe Leu Ser Gln Gln Ser Leu Phe
        35                  40                  45

Pro Glu Pro Leu Ser Ile Gln Ser Gly Ser Val Gln Phe Leu Lys Ala
    50                  55                  60

Leu Trp Glu Lys Thr Gln Asp Thr Gly Ala His Ser Phe Glu Val Ala
65                  70                  75                  80

Met Thr Glu Ser Thr Phe Pro Gln Gln Lys Asp Leu Glu Gln Leu Gln
                85                  90                  95

Leu His Leu Glu Glu Val Arg Phe Phe Asp Val Phe Gly Phe Ser Glu
            100                 105                 110

Thr Ala Gly Ala Trp Gln Cys Phe Met Cys Asn Asn Pro Glu Lys Ala
        115                 120                 125

Thr Val Val Asn Gln Asp Gly Gln Pro Leu Ile Glu Gly Lys Leu Lys
    130                 135                 140

Glu Lys Gln Val Arg Trp Lys Phe Ile Lys Arg Trp Lys Thr His Tyr
145                 150                 155                 160

Phe Thr Leu Ala Gly Asn Gln Leu Leu Phe Gln Lys Gly Lys Ser Lys
                165                 170                 175

Asp Asp Pro Asp Asp Ser Pro Ile Glu Leu Ser Lys Val Gln Ser Val
            180                 185                 190

Lys Ala Val Ala Lys Lys Arg Arg Asp Arg Ser Leu Pro Arg Ala Phe
        195                 200                 205

Glu Ile Phe Thr Asp Ser Lys Thr Tyr Val Phe Lys Ala Lys Asp Glu
    210                 215                 220

Lys Asn Ala Glu Glu Trp Leu Gln Cys Ile Asn Val Ala Leu Ala Gln
225                 230                 235                 240

Ala Lys Glu Arg Glu Ser Arg Glu Val Thr Thr Tyr Leu
                245                 250

<210>  14
<211>  3042
<212>  DNA
<213>  Drosophila

<400>  14
atgcacgaac ttttcaacaa agtgctcgcc aaacgcgatc tctcaagagc cggagatctc      60

ttttccgtgc cggatgcaga tatagtcgac gacatcacgg aggtgctttc cgagatcagt     120

ccgatcatct cgcacgcgga ctatgtgaag aacaacaatg accagagtgt ggtggagatc     180

tgtgtgacac gggtgctatc ctgcatccgg gaaacgaaaa ctgcggagcg atactgtgct     240

gctctggtgg atcttctgag gacctgtctg ctatggaacc tccaaccctc gggaacaaca     300

aaggaagagc caccccatgc caagattgct gccgatatca tctctagcat tttcctgaac     360

tatgacaaaa aagaactgat gaaaatagcc cttcccgtcg ccgtgcagtt ccttccgaag     420

ggaaaccgtg agctatcgcg aaacttggcc agttacctct cactggcggc catcgatcac     480

gccggcttgc tgagccctca tacggagtcc gtgatggagt ccattctgtc cggaaactat     540

ggtctactcc gagtgctttc acaggtgtac gaggtggctc cggaggcggt gacgccacat     600

gcaccacttc tcatgcccct gctgccacag tgtgatccgc aggagagaat ggcggtgttc     660

cagctgtatc tgctgatcgt tcagaagtca ccagaggttc tggaatcttg cgttccgcaa     720

ctttgtggct ccctgctcga tagcgacact tctagcataa ccatgcagat tctgctgaag     780

ctttcccagc actcgcccag ccttcttgtc gaccatttcg agcaattgcg cctggcagct     840

aaaaccaatc ctggaacgat tccgctttgt gcccagatta tgactaccgc gggaatcggg     900

agcaaggaga cggcccagca ggctttggac tttgtgctgg agcacctgcc cacgcaggca     960

ctcctgcaac aggaggccac tcgcctctgt tccgcctatc cggttttgtt caccgacaaa    1020

gtactggcct gcgtgcggca gaagaacgca gctctcagct cgcaacagga tgtggggaat    1080

ggcttggcga acaagacctc tggtggtgtc accattgtca gcttgaatag ctccagtccc    1140

agtccgccgc ttaagccagt tccagttggc gcaccagcta ttaacaccac aatcgtccag    1200

ccggccagcg tagtcagtgc cacgcccaca ccacctccat cctccgcctc ggcgcccatt    1260

gccagctcca cgcccgtatc ggctgccacg cccactcccc agcatgcggg atacacgcgg    1320

cgggtgaagc taggcgattc ccggagcaca ggtcgcctgc atcccgccag caatacgcac    1380

cggagtgtga cccggttgaa tgtggccagc ggatccgtgg gaggacttca caagagcatg 1440
actcggctga gtaactcgca gattaaccag caacctggag gcagctccaa tgggaatgtc 1500
gtggtgcaat cgggggctac tccgaagacc ccaactggtc taagcaatcc gcctgtgaca 1560
ccagtgccgc ccttgagcaa caacgtggtc ataactggac acaatcgaca tggtatacct 1620
gttacctctg gaggagtgac tgtgaccacg tcgccctcga aggtgcgacc acactcccaa 1680
ggaccatcaa cgctgctcaa ctcgagcacg gtgctgatga agtacagcac ggatgcactg 1740
aatcaatcgg tgggctcgat ctccatacca cagtcggcag cggtggccac tttgccatcg 1800
acgcagacac agaatgcggt atccgtccat catgcctcgc cggcgctgcc ccaatccgcc 1860
agcaatggtc atgccaagtc agtgatgaaa ctgccagtca acggaaatag cgaggtaatc 1920
gtttcgggac ccaccaccaa tgtggcacct cggcgcagcg ataataccag caggacccta 1980
ctgaatgcca acagtgtcat ggaccagcgg atgagcacct ttgagccata ccagattatg 2040
cgtgatcctg ttcagcaatt ctgcgagaag aacttcaatt cgatcaagtc ctacatggat 2100
gaggtatcgc agcatctgcc gccgcccaca cgttgcagca ttgaggagcg tcgtaccaaa 2160
aaggtggcca aactgcattt cgcctgccag attcgtggtc cccattgcct gtactccaaa 2220
acttgcttca cgatgcgaac tcggaacccc aagacctgga tacatatgat gttcctggac 2280
tttcaagtgc gccattttgt gaaggagaag tgcgtgctta gcacgcggga gtccggcatt 2340
agcaatctca agaatatttg gcagatactc aagtgcgaga accgcagctt caccgagctg 2400
gttaccagtc agtttcctca ggttaaggac cgcgagatcc tggtgaacga gctgcgtcac 2460
tcgggctttc tggatgtgtt cgaggtctcc aagacagaca agtccaaccc aaactgcaat 2520
gagttggaat accagtgggg ctgcttcctc tgcaatcatc cggacaaggc ggtgggcttt 2580
ctcaatggca gcaatcagcc catgatcgag ggccagctga aggagaagaa gggcaagtgg 2640
cgactattca ggcgctggcg aacgcgctac ttcactttgt ccggagcgca cttgtcctgc 2700
aaaggctcta gcggaggtga gagcatcgat gtgaatcaga tacgttcggt aaaggtatct 2760
cgtggtgccc gtaacatacc gaaggccttc gaaatcttca ccgccgacca gacattaata 2820
ctcaagccca aggacggcaa gaatgccgag gagtgggtgc agtgcctcag catcgtggta 2880
gcccactcgc aggcccgaga taatcccacg gccaagacga acagcctgcc cgcccgcagc 2940
ataggcagca gcaagccctc cttctaggcg gccgctttgg gcggatcccc cgggctgcag 3000
gaattcgata tcaagcttat cgataccgtc gacctcgagg gg 3042

<210> 15
<211> 3739
<212> DNA
<213> Drosophila

<400> 15
agtcttcatt cgggcccaaa cgcgttcgct tggccagcgg ctcgtatccg tcgctttctt 60

cgcctatcgg tgtgtgcgtg cgcctgccta cgtgtgtgcc tgtgcatgcg agtgtgtgtg 120

ttaacaaaat gtgattagca aaaatacaaa gaaatcaggc atagtggaac aaggcattgt 180
ggctgaaaca acagtcggcg gcagtaacag tcgacactaa aaaacaacaa aatatacaca 240
tatacatata ttaataatag tacatacgaa acatatcttt tgagatatac acgaaatgcg 300
aaaatttgca taaaaagcaa tgcgctggcg cggcaacaaa gcgcggccgt aaaaaaataa 360
gtaacgccaa cgacaattct gaattttgtg ctttatcggc agcagcagca acaattaaat 420
taatattgaa ctacacccaa agttaacaaa agtagcagcc ataaaaaaaa tacaacacca 480
cagccgcaac aacaatagcg gcagcagcaa caaccacaaa cgttgtctac cttttttacc 540
ttttcaagtc tgttgatttt ttttttttgg attttgtgtt gctgctgcaa gagcgatgta 600
ttgagagagc ggcttagctc cgaatcggaa accgactccg aattcgaggc ccacacttcg 660
aagtgatacg gactggtcat ctgtctgcca gcagccgcag atgcaattcg ttgctgcaga 720
tgaagaattt tgacgaatac taattgggga attctccacg atgcacgaac ttttcaacaa 780
agtgctcgcc aaacgcgatc tctcaagagc cggagatctc ttttccgtgc cggatgcaga 840
tatagtcgac gacatcacgg aggtgctttc cgagatcagt ccgatcatct cgcacgcgga 900
ctatgtgaag aacaacaatg accagagtgt ggtggagatc tgtgtgacac gggtgctatc 960
ctgcatccgg gaaacgaaaa ctgcggagcg atactgtgct gctctggtgg atcttctgag 1020
gacctgtctg ctatggaacc tccaaccctc gggaacaaca aaggaagagc caccccatgc 1080
caagattgct gccgatatca tctctagcat tttcctgaac tatgacaaaa aagaactgat 1140
gaaaatagcc cttcccgtcg ccgtgcagtt ccttccgaag ggaaaccgtg agctatcgcg 1200
aaacttggcc agttacctct cactggcggc catcgatcac gccggcttgc tgagccctca 1260
tacggagtcc gtgatggagt ccattctgtc cggaaactat ggtctactcc gagtgctttc 1320
acaggtgtac gaggtggctc cggaggcggt gacgccacat gcaccacttc tcatgcccct 1380
gctgccacag tgtgatccgc aggagagaat ggcggtgttc cagctgtatc tgctgatcgt 1440
tcagaagtca ccagaggttc tggaatcttg cgttccgcaa ctttgtggct ccctgctcga 1500
tagcgacact tctagcataa ccatgcagat tctgctgaag ctttcccagc actcgcccag 1560
ccttcttgtc gaccatttcg agcaattgcg cctggcagct aaaaccaatc ctggaacgat 1620
tccgctttgt gcccagatta tgactaccgc gggaatcggg agcaaggaga cggcccagca 1680
ggctttggac tttgtgctgg agcacctgcc cacgcaggca ctcctgcaac aggaggccac 1740
tcgcctctgt tccgcctatc cggttttgtt caccgacaaa gtactggcct gcgtgcggca 1800
gaagaacgca gctctcagct cgcaacagga tgtggggaat ggcttggcga acaagacctc 1860
tggtggtgtc accattgtca gcttgaatag ctccagtccc agtccgccgc ttaagccagt 1920
tggcgcacca gctattaaca ccacaatcgt ccagccggcc agcgtagtca gtgccacgcc 1980
cacaccacct ccatcctccg cctcggcgcc cattgccagc tccacgcccg tatcggctgc 2040
cacgcccact ccccagcatg cgggatacac gcggcgggtg aagctaggcg attcccggag 2100
cacaggtcgc ctgcatcccg ccagcaatac gcaccggagt gtgacccggt tgaatgtggc 2160

cagcggatcc gtgggaggac ttcacaagag catgactcgg ctgagtaact cgcagattaa 2220
ccagcaacct ggaggcagct ccaatgggaa tgtcgtggtg caatcggggg ctactccgaa 2280
gaccccaact ggtctaagca atccgcctgt gacaccagtg ccgcccttga gcaacaacgt 2340
ggtcataact ggacacaatc gacatggtat acctgttacc tctggaggag tgactgtgac 2400
cacgtcgccc tcgaaggtgc gaccacactc ccaaggacca tcaacgctgc tcaactcgag 2460
cacggtgctg atgaagtaca gcacggatgc actgaatcaa tcggtgggct cgatctccat 2520
accacagtcg gcagcggtgg ccactttgcc atcgacgcag acacagaatg cggtatccgt 2580
ccatcatgcc tcgccggcgc tgccccaatc cgccagcaat ggtcatgcca agtcagtgat 2640
gaaactgcca gtcaacggaa atagcgaggt aatcgtttcg ggacccacca ccaatgtggc 2700
acctcggcgc agcgataata ccagcaggac cctactgaat gccaacagtg tcatggacca 2760
gcggatgagc acctttgagc cataccagat tatgcgtgat cctgttcagc aattctgcga 2820
gaagaacttc aattcgatca agtcctacat ggatgaggta tcgcagcatc tgccgccgcc 2880
cacacgttgc agcattgagg tttccaatga atttgcagag cgtcgtacca aaaaggtggc 2940
caaactgcat ttcgcctgcc agattcgtgg tccccattgc ctgtactcca aaacttgctt 3000
cacgatgcga actcggaacc ccaagacctg gatacatatg atgttcctgg actttcaagt 3060
gcgccatttt gtgaaggaga agtgcgtgct tagcacgcgg gagtccggca ttagcaatct 3120
caagaatatt tggcagatac tcaagtgcga gaaccgcagc ttcaccgagc tggttaccag 3180
tcagtttcct caggttaagg accgcgagat cctggtgaac gagctgcgtc actcgggctt 3240
tctggatgtg ttcgaggtct ccaagacaga caagtccaac ccaaactgca atgagttgga 3300
ataccagtgg ggctgcttcc tctgcaatca tccggacaag gcggtgggct ttctcaatgg 3360
cagcaatcag cccatgatcg agggccagct gaaggagaag aagggcaagt ggcgactatt 3420
caggcgctgg cgaacgcgct acttcacttt gtccggagcg cacttgtcct gcaaaggctc 3480
tagcggaggt gagagcatcg atgtgaatca gatacgttcg gtaaaggtat ctcgtggtgc 3540
ccgtaacata ccgaaggcct tcgaaatctt caccgccgac cagacattaa tactcaagcc 3600
caaggacggc aagaatgccg aggagtgggt gcagtgcctc agcatcgtgg tagcccactc 3660
gcaggcccga gataatccca cggccaagac gaacagcctg cccgcccgca gcataggcag 3720
cagcaagccc tccttctag 3739

<210> 16
<211> 3727
<212> DNA
<213> Drosophila

<400> 16
agtcttcatt cgggcccaaa cgcgttcgct tggccagcgg ctcgtatccg tcgctttctt 60
cgcctatcgg tgtgtgcgtg cgcctgccta cgtgtgtgcc tgtgcatgcg agtgtgtgtg 120
ttaacaaaat gtgattagca aaaatacaaa gaaatcaggc atagtggaac aaggcattgt 180
ggctgaaaca acagtcggcg gcagtaacag tcgacactaa aaaacaacaa aatatacaca 240

tatacatata ttaataatag tacatacgaa acatatcttt tgagatatac acgaaatgcg 300
aaaatttgca taaaaagcaa tgcgctggcg cggcaacaaa gcgcggccgt aaaaaaataa 360
gtaacgccaa cgacaattct gaattttgtg ctttatcggc agcagcagca acaattaaat 420
taatattgaa ctacacccaa agttaacaaa agtagcagcc ataaaaaaaa tacaacacca 480
cagccgcaac aacaatagcg gcagcagcaa caaccacaaa cgttgtctac ctttttaccc 540
ttttcaagtc tgttgatttt ttttttttgg attttgtgtt gctgctgcaa gagcgatgta 600
ttgagagagc ggcttagctc cgaatcggaa accgactccg aattcgaggc ccacacttcg 660
aagtgatacg gactggtcat ctgtctgcca gcagccgcag atgcaattcg ttgctgcaga 720
tgaagaattt tgacgaatac taattgggga attctccacg atgcacgaac ttttcaacaa 780
agtgctcgcc aaacgcgatc tctcaagagc cggagatctc ttttccgtgc cggatgcaga 840
tatagtcgac gacatcacgg aggtgctttc cgagatcagt ccgatcatct cgcacgcgga 900
ctatgtgaag aacaacaatg accagagtgt ggtggagatc tgtgtgacac gggtgctatc 960
ctgcatccgg gaaacgaaaa ctgcggagcg atactgtgct gctctggtgg atcttctgag 1020
gacctgtctg ctatggaacc tccaaccctc gggaacaaca aaggaagagc caccccatgc 1080
caagattgct gccgatatca tctctagcat tttcctgaac tatgacaaaa aagaactgat 1140
gaaaatagcc cttcccgtcg ccgtgcagtt ccttccgaag ggaaaccgtg agctatcgcg 1200
aaacttggcc agttacctct cactggcggc catcgatcac gccggcttgc tgagccctca 1260
tacggagtcc gtgatggagt ccattctgtc cggaaactat ggtctactcc gagtgctttc 1320
acaggtgtac gaggtggctc cggaggcggt gacgccacat gcaccacttc tcatgcccct 1380
gctgccacag tgtgatccgc aggagagaat ggcggtgttc cagctgtatc tgctgatcgt 1440
tcagaagtca ccagaggttc tggaatcttg cgttccgcaa ctttgtggct ccctgctcga 1500
tagcgacact tctagcataa ccatgcagat tctgctgaag ctttcccagc actcgcccag 1560
ccttcttgtc gaccatttcg agcaattgcg cctggcagct aaaaccaatc ctggaacgat 1620
tccgctttgt gcccagatta tgactaccgc gggaatcggg agcaaggaga cggcccagca 1680
ggctttggac tttgtgctgg agcacctgcc cacgcaggca ctcctgcaac aggaggccac 1740
tcgcctctgt tccgcctatc cggttttgtt caccgacaaa gtactggcct gcgtgcggca 1800
gaagaacgca gctctcagct cgcaacagga tgtggggaat ggcttggcga acaagacctc 1860
tggtggtgtc accattgtca gcttgaatag ctccagtccc agtccgccgc ttaagccagt 1920
tccagttggc gcaccagcta ttaacaccac aatcgtccag ccggccagcg tagtcagtgc 1980
cacgcccaca ccacctccat cctccgcctc ggcgcccatt gccagctcca cgcccgtatc 2040
ggctgccacg cccactcccc agcatgcggg atacacgcgg cgggtgaagc taggcgattc 2100
ccggagcaca ggtcgcctgc atcccgccag caatacgcac cggagtgtga cccggttgaa 2160
tgtggccagc ggatccgtgg gaggacttca caagagcatg actcggctga gtaactcgca 2220
gattaaccag caacctggag gcagctccaa tgggaatgtc gtggtgcaat cgggggctac 2280

tccgaagacc ccaactggtc taagcaatcc gcctgtgaca ccagtgccgc ccttgagcaa 2340
caacgtggtc ataactggac acaatcgaca tggtatacct gttacctctg gaggagtgac 2400
tgtgaccacg tcgccctcga aggtgcgacc acactcccaa ggaccatcaa cgctgctcaa 2460
ctcgagcacg gtgctgatga agtacagcac ggatgcactg aatcaatcgg tgggctcgat 2520
ctccatacca cagtcggcag cggtggccac tttgccatcg acgcagacac agaatgcggt 2580
atccgtccat catgcctcgc cggcgctgcc ccaatccgcc agcaatggtc atgccaagtc 2640
agtgatgaaa ctgccagtca acggaaatag cgaggtaatc gtttcgggac ccaccaccaa 2700
tgtggcacct cggcgcagcg ataataccag caggacccta ctgaatgcca acagtgtcat 2760
ggaccagcgg atgagcacct ttgagccata ccagattatg cgtgatcctg ttcagcaatt 2820
ctgcgagaag aacttcaatt cgatcaagtc ctacatggat gaggtatcgc agcatctgcc 2880
gccgcccaca cgttgcagca ttgaggagcg tcgtaccaaa aaggtggcca aactgcattt 2940
cgcctgccag attcgtggtc cccattgcct gtactccaaa acttgcttca cgatgcgaac 3000
tcggaacccc aagacctgga tacatatgat gttcctggac tttcaagtgc gccattttgt 3060
gaaggagaag tgcgtgctta gcacgcggga gtccggcatt agcaatctca agaatatttg 3120
gcagatactc aagtgcgaga accgcagctt caccgagctg gttaccagtc agtttcctca 3180
ggttaaggac cgcgagatcc tggtgaacga gctgcgtcac tcgggctttc tggatgtgtt 3240
cgaggtctcc aagacagaca agtccaaccc aaactgcaat gagttggaat accagtgggg 3300
ctgcttcctc tgcaatcatc cggacaaggc ggtgggcttt ctcaatggca gcaatcagcc 3360
catgatcgag ggccagctga aggagaagaa gggcaagtgg cgactattca ggcgctggcg 3420
aacgcgctac ttcactttgt ccggagcgca cttgtcctgc aaaggctcta gcggaggtga 3480
gagcatcgat gtgaatcaga tacgttcggt aaaggtatct cgtggtgccc gtaacatacc 3540
gaaggccttc gaaatcttca ccgccgacca gacattaata ctcaagccca aggacggcaa 3600
gaatgccgag gagtgggtgc agtgcctcag catcgtggta gcccactcgc aggcccgaga 3660
taatcccacg gccaagacga acagcctgcc cgcccgcagc ataggcagca gcaagccctc 3720
cttctag 3727

<210> 17
<211> 4119
<212> DNA
<213> Homo sapiens

<400> 17
cagctgcagt gctctaaaga tcaaggattt tgccttctat tttctatgac cagagattcc 60
tgtgcaaaga gagagtgtca atgaagacct caaagtctgg agaaaaatga cctttcatgg 120
aataagaagt atacctcctt ctacatgttt ttgtcttact gacctctgat aactggaaca 180
catgactctg ggtctgtaga aagtcaactg atcaaactca tcctcaccat gcatcaactg 240
ttcagactgg ttttgggaca aaaagatctt tcacgagctg gggacctctt ctccttagat 300

gactctgaga ttgaagacag ccttacagaa gctttggagc aaattaagat aattagctca 360
tcttcagatt accaaaccaa taacaatgac caggcagtag ttgaaatctg tatcacaaga 420
atcacaacag ccatcagaga gaccgagtcc attgaaaagc atgcaaaggc ccttgtgggg 480
ctctgggact cctgcttgga acataacctg agaccctttg ggaaagacga agacactcct 540
catgcaaaaa tcgcatctga tatcatgagt tgcattttac agaattacaa ccgaccccca 600
gtgatggcat tagccatccc cattgcagtg aaattcctcc acagaggcaa caaggaactg 660
tgcaggaata tgtctaacta cctgtctctg gctgcaatta ccaaggcaga tctcctggct 720
gatcacacgg aagttatagt aaagagcata ctccaaggta acaccatgtt gttgagagtg 780
ttacctgctg tgtatgaaaa gcagcctcag ccaattaata gacacctgac agaactcctg 840
gccttgatgt ctcagctgga acagccagaa cagtaccatc tactacggct tttgcatgta 900
gcagcaaaga aaaaacaact cgaggtagtt cagaagtgta ttcctttcct aattgggcat 960
ttgaaggatt caacccataa tgacatcatc ctaaacatcc tcatagagat agcagtctat 1020
gagccagtgg ctttgaacag ttttcttcca atgctgaaag agattggtga gagattcccc 1080
tacctcactg gacagatggc aaggatttat ggagctgttg ggcatgtgga tgaagagaga 1140
gccaggagct gcctgacata cctggtgagc caactggcca acatggagca ttcgtttcac 1200
catattctcc tgctggagat taaaagcatc accgacacct tctcctcaat cttgggccct 1260
cagagcagag acatcttccg catgagcaac agcttcaccg ccattgctaa actccttacc 1320
cgacaactgg aaaataccaa ggctggaagt ggcaggagaa aaatcagcac tgaaattgaa 1380
ttccctgaga aactggaaga aaccaagctc atagtaactg aaaatgaaga ccatgaaaaa 1440
ctccaagtta aaatccaggc ttttgaagac aagataaatg cagggagcaa tacccctggc 1500
tctatcagaa gatatagtct gggccaagtt tctaaagaag aaagaaaaaa cattagattt 1560
aacaggtcaa aaagtttggc tttccacact atgctcacaa agggtgtggg ttcagatgac 1620
ggcgaagatg aaaacagggg agacatacca gccagcatct ctctttcaga aatagaccca 1680
cttggccaag gaaatgacaa gctgccgttt aagacagaca ctgagagatc acagctgggg 1740
gagtcttcag tttcataccc aaatattata catatagact cagagaattt gtcagaaact 1800
gttaaagaaa actcccagga agaaactcca gagacaactg caagtcctat agaataccaa 1860
gataagctct acttgcactt aaaaaaaaac ctcagcaaag tgaaagcata tgccatggaa 1920
attggaaaga agattccagt ccctgatcag tgtaccattg aagacactgt gagaagttgt 1980
gtagcaaagt tgttcttcac ctgctccctg aagggtcatt actgcctata cagtaagtcc 2040
agttttattc tcatcagcca agaacctcag ccatggatcc agatcatgtt tctatttcag 2100
cagagcctgt ttcctgaacc cctgtccatt cagagtcatt ctgtgcaatt cctcagagct 2160
ctgtgggaga agacccaggc agggggtgct cacagctttg aaactgccat gatggagtcc 2220
acgtttccac agcagaagga tctggaccag gtacagctcc atctggaaga agtgaggttc 2280
tttgacgtgt ttggcttcag tgaaacagca ggagcatggc aatgcttcat gtgcaacaat 2340

cctgagaaag caactgttgt aaatcaagat ggccagcctc tcatagaagg aaaacttaaa 2400
gagaagcaag tcagatggaa gttcatcaaa aggtggaaaa cacgctattt tacactggct 2460
ggaaatcaac ttctgtttca aaaaggaaag tctaaagatg accctgacga ctgcccaata 2520
gaactcagca aagtacagag tgtgaaggct gtggccaaga aacgcaggga ccgctctctc 2580
ccccgggctt tcgaaatctt cacagacaat aaaacctatg tctttaaggc caaggatgag 2640
aagaatgcag aagaatggct ccagtgcatc aacgtggcag ttgcccaagc caaagaaagg 2700
gaaagtagag aagtaaccac atatctgtag ggatttataa gtcagccatg acaattatac 2760
accacaggca ttgtattatc attgccaatg tcaagaaaaa gagctaaatt taccaagcca 2820
tgttgttttt tactaaatac caatggaatt gttgtccttt aagaagaagg gcctaaaatg 2880
gcaggattct tagtaaatgt catactctaa cagctttagt attgacttca gaatatatct 2940
gatgcccaca aaaataaaat aaaataaaag gagctacaga gtatgccctc agatagtgtg 3000
gggccaggaa gggaaaagtc actgataaaa gaacttctgt agatatgtca tttaaaactg 3060
tgagaaaaca acatgaaaag atgcctgaac tctccatcct gaggaaagat tagcacttct 3120
gatgaaaatc acagtctgtc aaaggtatta aaagaaaact ttaaaaaaaa aaacctttag 3180
aaacgttgaa ctaatgtaca ctgagaaaaa ccatataact tacttataga catataatta 3240
catgatcctc tcttactaca actgaggcta acacatggta aaatggttcc caaatcaagc 3300
cagttatgtc attaaatact tgtctttcag aattacttat ttaattattc catttaagtt 3360
catataatat aatctacagt ttagatactt ggacatatat cctgcccaag gcactaagtg 3420
taatatagga aaaggataga aagccatttt ctctatcttc aaaataatgt gccatctccg 3480
ttaaaattaa gactttaaag ttctataatt ttttctttgt attcaacttt ttaatacctc 3540
caaattacaa acatctattt ttttcactac atagatatat aattacacta tgatagacag 3600
aattctagag tgattctcaa taatccatgc cttggtataa tcctctcccc cattgagtgt 3660
gggtgggaca tgtaacctgc ttctaaccaa cagaatatgg caaaagtgat gggatcatct 3720
ctcccatatt atgttatgtt atataacaaa agtgaagaga agttgcagat atacataagg 3780
tttctaatca gctgactttg agttaatcaa agagatcccc tgggtgggcc tgacctaatc 3840
agactagtcc tttaaaagag agtgaaccag tcagagactc tctctcctat tagccttaaa 3900
taagcaagct gccatgagct ctacggctgc aaggaaataa gttttgtgaa caaccatgtg 3960
atcttggaag aagcatctga acctcagata aaagcccagc cccagccaac gccttgcttg 4020
cagctttgtg aaactctgag cagaggaccc agctaaatca cgtttggact cctgacccac 4080
agaaactttc agataataaa tgtatgctgt tttaaatct 4119

<210> 18
<211> 3044
<212> DNA
<213> Homo sapiens

<400> 18
attcttccac ccggcaccgg ctccgcaatg aacaacttca ggttcactga gctgagcgga 60

ttccactcgc tgtgccctcg gagcctgcct gaatctcccg cccagtctca aaccatgcag 120
ctgagtaact gcaagggcgc ctttgaggag tagcacgcag agagtgtcaa tgaagacctc 180
aaagtctgga gaaaaatgac ctttcatgga ataagaagta tacctccttc tacatgtttt 240
tgtcttactg acctctgata actggaacac atgactctgg gtctgtagaa agtcaactga 300
tcaaactcat cctcaccatg catcaactgt tcagactggt tttgggacaa aaagatcttt 360
cacgagctgg ggacctcttc tccttagatg actctgagat tgaagacagc cttacagaag 420
ctttggagca aattaagata attagctcat cttcagatta ccaaaccaat aacaatgacc 480
aggcagtagt tgaaatctgt atcacaagaa tcacaacagc catcagagag accgagtcca 540
ttgaaaagca tgcaaaggcc cttgtggggc tctgggactc ctgcttggaa cataacctga 600
gaccctttgg gaaagacgaa gacactcctc atgcaaaaat cgcatctgat atcatgagtt 660
gcattttaca gaattacaac cgacccccag tgatggcatt agccatcccc attgcagtga 720
aattcctcca cagaggcaac aaggaactgt gcaggaatat gtctaactac ctgtctctgg 780
ctgcaattac caaggcagat ctcctggctg atcacacgga agttatagta aagagcatac 840
tccaaggtaa caccatgttg ttgagagtgt tacctgctgt gtatgaaaag cagcctcagc 900
caattaatag acacctgaca gaactcctgg ccttgatgtc tcagctggaa cagccagaac 960
agtaccatct actacggctt ttgcatgtag cagcaaagaa aaaacaactc gaggtagttc 1020
agaagtgtat tcctttccta attgggcatt tgaaggattc aacccataat gacatcatcc 1080
taaacatcct catggagata gcagtctatg agccagtggc tttgaacagt tttcttccaa 1140
tgctgaaaga gattggtgag agattcccct acctcactgg acagatggca aggatttatg 1200
gagctgttgg gcatgtggat gaagagagag ccaggagctg cctgacatac ctggtgagcc 1260
aactggccaa catggagcat tcgtttcacc atattctcct gctggagatt aaaagcatca 1320
ccgacacctt ctcctcaatc ttgggccctc agagcagaga catcttccgc atgagcaaca 1380
gcttcaccgc cattgctaaa ctccttaccc gacaactgga aaataccaag gctggaagtg 1440
gcaggagaaa aatcagcact gaaattgaat tccctgagaa actggaagaa accaagctca 1500
tagtaactga aatgaagacc atgaaaaacc tccaagttaa aatccaggct tttgaagaca 1560
agataaatgc agggagcaat acccctggct ctatcagaag atatagtctg ggccaagttt 1620
ctaaagaaga aagaaaaaac attagattta acaggtcaaa aagtttggct ttccacacta 1680
tgctcacaaa gggtgtgggt tcagatgacg gcgaagatga aaacagggga gacataccag 1740
ccagcatctc tctttcagaa atagacccac ttggccaagg aaatgacaag ctgccgttta 1800
agacagacac tgagagatca cagctggggg agtcttcagt ttcataccca aatattatac 1860
atatagactc agagaatttg tcagaaactg ttaaagaaaa ctcccaggaa gaaactccag 1920
agacaactgc aagtcctata gaataccaag ataagctcta cttgcactta aaaaaaaacc 1980
tcagcaaagt gaaagcatat gccatggaaa ttggaaagaa gattccagtc cctgatcagt 2040
gtaccattga agacactgtg agaagttgtg tagcaaagtt gttcttcacc tgctccctga 2100

agggtcatta ctgcctatac agtaagtcca gttttattct catcagccaa gaacctcagc 2160
catggatcca gatcatgttt ctatttcagc agagcctgtt tcctgaaccc ctgtccattc 2220
agagtcattc tgtgcaattc ctcagagctc tgtgggagaa gacccaggca gggggtgctc 2280
acagctttga aactgccatg atggagtcca cgtttccaca gcagaaggat ctggaccagg 2340
tacagctcca tctggaagaa gtgaggttct ttgacgtgtt tggcttcagt gaaacagcag 2400
gagcatggca atgcttcatg tgcaacaatc ctgagaaagc aactgttgta aatcaagatg 2460
gccagcctct catagaagga aaacttaaag agaagcaagt cagatggaag ttcatcaaaa 2520
ggtggaaaac acgctatttt acactggctg gaaatcaact tctgtttcaa aaaggaaagt 2580
ctaaagatga ccctgacgac tgcccaatag aactcagcaa agtacagagt gtgaaggctg 2640
tggccaagaa acgcagggac cgctctctcc cccgggcttt cgaaatcttc acagacaata 2700
aaacctatgt ctttaaggcc aaggatgaga agaatgcagc agaatggctc cagtgcatca 2760
acgtggcagt tgcccaagcc aaagaaaggg aaagtagaga agtaaccaca tatctgtagg 2820
gatttataag tcagccatga caattataca ccacaggcat tgtattatca ttgccaatgt 2880
caagaaaaag agctaaattt accaagccat gttgtttttt actaaatacc aatggaattg 2940
ttgtccttta agaagaaggg cctaaaatgg caggattctt agtaaatgtc atactctaac 3000
agctttagta ttgacttcag aatatatctg atgcccacaa aaat 3044

<210> 19
<211> 2866
<212> DNA
<213> Mus musculus

<400> 19
ttactcattc gcctgatacc acacggtgag tccacaggag ggactcaggc gcagcgctgc 60
atgacaagct cacagggacg tgccagcagc caccggcagg acaggcaggc gctgcagggc 120
tgcctctgag cgtggtcctg ggttccagct gaagtgttcc aagggatcaa gcatttgcct 180
tctgttgcct gcacctgcca gtgtcccgtg ctaggagaga cagccaaagg agaccttaga 240
gcctgcagaa ccatggctct tcatggaata tgaagtgtac cagctgctac gtggttttct 300
atctctaacc ctgacaactg accatggccc tggattcaca gaaatccaac tgatccaaca 360
cacaatgcat caacttttca gattggtttt gggacaaaaa gatctttcaa aagctgggga 420
cctcttctct ttagatgatg ctgagattga agacagcctg acagaagctt tggagcagat 480
taaagtaatt agctcatctt tggattacca aactaataac aatgaccagg cggtggttga 540
aatctgtatc acaaggatta ccacagccat cagagagaca gaatccattg aaaagcatgc 600
cagagccctg gtggggctgt gggattcctg cttggaacac aacctgagac ctgcagggaa 660
agacgaagat actccacatg caaaaatcgc ctctgatatc atgagttgca tcttacagaa 720
ttacaaccgg acccctgtga tggtcttagc tgtccccatc gcagtgaaat tcctccacag 780
aggcagcaag gagctgtgta ggaacatgtc caactacctg tccctggctg ccatcaccaa 840

ggcggatctg ctggctgacc acactgaagg gataataaag agcatactcc aaggaaatgc 900
catgctgtta agggtgctac ctgctgtata tgaaaagcag ccccaaccaa tcaacagaca 960
cctggcagaa ctcctggcct tgatgtctca attggagcag acagaacagt accatctact 1020
aaggcttctg cacgtagcag caaagagaaa agatgtggag gtggttcaga aatgtgttcc 1080
tttcctgatc agaaatttga aggattcaac ctataatgac atcatcctaa acattctcat 1140
agaaatagca ggccatgagc cactggcctt gaacagtttc ctcccgatgc tgaaagagat 1200
tgcagagcaa ttcccctacc tcactggaca gatggcaagg atctttggtg ctgttggtca 1260
tgtggatgaa gagagagcca ggagctgcct acgatacctg gtgagccagc tggctaatat 1320
ggagcatcca ttccaccata ttctcctgct ggaaattaag agtatcacag atgccttctc 1380
ctccatcctg ggcccgcaca gcagggacat cttccgaatg agcaacagct tcaccaacat 1440
tgccaagctc ctttccagac aactggaaaa cagcaaggct gacagtagca ggagaaaaac 1500
cagcactgaa gttagcattc ctgagaaact gagagaactc aattccatgg agcctgaaag 1560
tgaagaccat gaaaaactcc aagttaaaat ccaagccttt gaagacaaaa taaatgctga 1620
gagcaatacc ccaggctctg gccggagata cagcttagac cacatctcta aggaagaacg 1680
aaaaagcatt agatttagca ggtccagaag tttggctttg aatactgtgc tcacaaacgg 1740
tgtgagtgtg gaagataacg aagtcgaaga aaaggcagga atgcacgcca gcatctctct 1800
ttcgcaaata gacccactca gccacgggat tggcaagttg ccctttaaga cagacactca 1860
tggatcacca ctgagaaatt cctcagcttc acacccaagt attatacata ctgaaccaga 1920
gactatgccc gaaactttta aagaaaacat ccaagaagaa attctagagg cagccacaag 1980
tcctattgaa taccaagata agctctactt gcatttaagg gaaaacctca gcaaagtgaa 2040
ggcttatgcc ttggaaatcg caaaaaaggt cccaatccca gaccagtgca ccatcgaaga 2100
tactatgaga agttgcgtgg caaagctgtt ctttacctgt tctttaaagg gccattactg 2160
cctatacagc aagtccagct tcatccttgt cagccaagca ccccagccgt ggatccaggt 2220
catgtttctc tcccaacaga gcctgtttcc tgagcccctg tccattcaga gtgggtctgt 2280
gcagttcctc aaagccctat gggagaaaac gcaggacacg ggagcgcaca gctttgaagt 2340
cgccatgaca gagtccacct ttccacagca gaaggatctg gagcagcttc agctccatct 2400
ggaagaagtg cggttcttcg acgtgtttgg cttcagtgaa acagcggggg catggcagtg 2460
cttcatgtgc aacaaccctg aaaaggcaac tgttgtaaat caagatggcc agcctctcat 2520
cgaaggaaaa cttaaggaga aacaagtgag gtggaagttc atcaagaggt ggaaaacgcg 2580
ttattttaca ctggccggaa accaacttct gtttcaaaag gggaaatcta aggatgaccc 2640
tgatgatccc ccgatagaac tcagcaaggt acagagcgtg aaagctgtgg ctaagaaacg 2700
ccgggaccgc tctctgcctc gggcgtttga aatcttcaca gatagtaaga cctatgtttt 2760
caaagccaag gatgagaaga acgcagagga atggctgcag tgcatcaacg tggcgctggc 2820
ccaggcaaag gagagggaaa gccgagaagt aaccacatat ctgtag 2866

<210> 20
<211> 1414
<212> DNA
<213> Mus musculus

<400> 20
tggacttaga gcgtgctctg ggcgagtcca cctgtggttc aggaaaaggg tttcaaagac 60

ttttgttgca gccttctggt gatcactgga ggggcaggca gctgctcaga gaagctgggt 120

cctgacctcg tgcaggcaga ctgggatgcc acatttagct catccacaaa gctttggata 180

ctatgagaag ttgcgtggca aagctgttct ttacctgttc tttaaagggc cattactgcc 240

tatacagcaa gtccagcttc atccttgtca gccaagcacc ccagccgtgg atccaggtca 300

tgtttctctc ccaacagagc ctgtttcctg agcccctgtc cattcagagt gggtctgtgc 360

agttcctcaa agccctatgg gagaaaacgc aggacacggg agcgcacagc tttgaagtcg 420

ccatgacaga gtccaccttt ccacagcaga aggatctgga gcagcttcag ctccatctgg 480

aagaagtgcg gttcttcgac gtgtttggct tcagtgaaac agcgggggca tggcagtgct 540

tcatgtgcaa caaccctgaa aaggcaactg ttgtaaatca agatggccag cctctcatcg 600

aaggaaaact taaggagaaa caagtgaggt ggaagttcat caagaggtgg aaaacgcatt 660

attttacact ggccggaaac caacttctgt ttcaaaaggg gaaatctaag gatgaccctg 720

atgattcccc gatagaactc agcaaggtac agagcgtgaa agctgtggct aagaaacgcc 780

gggaccgctc tctgcctcgg gcgtttgaaa tcttcacaga tagtaagacc tatgttttca 840

aagccaagga tgagaagaac gcagaggaat ggctgcagtg catcaacgtg gcgctggccc 900

aggcaaagga gagggaaagc cgagaagtaa ccacatatct gtagcgattc gcacacccgc 960

tcttatggcc atgcaagata agcattgcca gtgtcaacaa agaggaaccc accatggcgc 1020

tattgggttt cactaaatac cagggaaacc attatcccta aggagagcct aaaatggcag 1080

ggttctcagt aaacctcacg ccctcgcaac tctggtatta actccaagtt ttctgcctga 1140

agttcatgag aaagagaggc cacggagttc acagtggatc gtgtgggcca gaaagggaac 1200

gctgcgaata aaagaacttc tgtagctatg tgatttatga ctgtaagaaa acagtgagtg 1260

gggccagcag gaaggctcag caggtaaagg gcacttgctg ccaagccaga ttgcctgagt 1320

tctagcccca taactgactt ggtggaagga gaactaactc ttggcggttt tcctctgttt 1380

tctgcatgcc atgggacatc tatttcccca gtct 1414

<210> 21
<211> 10141
<212> DNA
<213> artificial

<220>
<223> pUAS-dMeltPH-GFP sequence

<400> 21
gaattcgagc tcgctagcgc taccggtcgc caccatggtg agcaagggcg aggagctgtt 60

caccggggtg gtgcccatcc tggtcgagct ggacggcgac gtaaacggcc acaagttcag 120

cgtgtccggc gagggcgagg gcgatgccac ctacggcaag ctgaccctga agttcatctg 180
caccaccggc aagctgcccg tgccctggcc caccctcgtg accaccctga cctacggcgt 240
gcagtgcttc agccgctacc ccgaccacat gaagcagcac gacttcttca agtccgccat 300
gcccgaaggc tacgtccagg agcgcaccat cttcttcaag gacgacggca actacaagac 360
ccgcgccgag gtgaagttcg agggcgacac cctggtgaac cgcatcgagc tgaagggcat 420
cgacttcaag gaggacggca acatcctggg gcacaagctg gagtacaact acaacagcca 480
caacgtctat atcatggccg acaagcagaa gaacggcatc aaggtgaact tcaagatccg 540
ccacaacatc gaggacggca gcgtgcagct cgccgaccac taccagcaga acacccccat 600
cggcgacggc cccgtgctgc tgcccgacaa ccactacctg agcacccagt ccgccctgag 660
caaagacccc aacgagaagc gcgatcacat ggtcctgctg gagttcgtga ccgccgccgg 720
gatcactctc ggcatggacg agctgtacaa gtccggactc agatctcgag gctttctcaa 780
tggcagcaat cagcccatga tcgagggcca gctgaaggag aagaagggca agtggcgact 840
attcaggcgc tggcgaacgc gctacttcac tttgtccgga gcgcacttgt cctgcaaagg 900
ctctagcgga ggtgagagca tcgatgtgaa tcagatacgt tcggtaaagg tatctcgtgg 960
tgcccgtaac ataccgaagg ccttcgaaat cttcaccgcc gaccagacat taatactcaa 1020
gcccaaggac ggcaagaatg ccgaggagtg ggtgcagtgc ctcagcatcg tggtagccca 1080
ctcgcaggcc cgagataatc ccacgtagga tcctctagag tcgagggtac ctctagagga 1140
tctttgtgaa ggaaccttac ttctgtggtg tgacataatt ggacaaacta cctacagaga 1200
tttaaagctc taaggtaaat ataaaatttt taagtgtata atgtgttaaa ctactgattc 1260
taattgtttg tgtattttag attccaacct atggaactga tgaatgggag cagtggtgga 1320
atgcctttaa tgaggaaaac ctgttttgct cagaagaaat gccatctagt gatgatgagg 1380
ctactgctga ctctcaacat tctactcctc caaaaaagaa gagaaaggta gaagacccca 1440
aggactttcc ttcagaattg ctaagttttt tgagtcatgc tgtgtttagt aatagaactc 1500
ttgcttgctt tgctatttac accacaaagg aaaaagctgc actgctatac aagaaaatta 1560
tggaaaaata ttctgtaacc tttataagta ggcataacag ttataatcat aacatactgt 1620
tttttcttac tccacacagg catagagtgt ctgctattaa taactatgct caaaaattgt 1680
gtacctttag ctttttaatt tgtaaagggg ttaataagga atatttgatg tatagtgcct 1740
tgactagaga tcataatcag ccataccaca tttgtagagg ttttacttgc tttaaaaaac 1800
ctcccacacc tccccctgaa cctgaaacat aaaatgaatg caattgttgt tgttaacttg 1860
tttattgcag cttataatgg ttacaaataa agcaatagca tcacaaattt cacaaataaa 1920
gcattttttt cactgcattc tagttgtggt ttgtccaaac tcatcaatgt atcttatcat 1980
gtctggatcg gatccactag aaggccttag tatgtatgta agttaataaa accctttttt 2040
ggagaatgta gatttaaaaa aacatatttt tttttatttt tttactgcac tggacatcat 2100
tgaacttatc tgatcagttt taaatttact tcgatccaag ggtatttgaa gtaccaggtt 2160

ctttcgatta cctctcactc aaaatgacat tccactcaaa gtcagcgctg tttgcctcct 2220
tctctgtcca cagaaatatc gccgtctctt tcgccgctgc gtccgctatc tctttcgcca 2280
ccgtttgtag cgttacctag cgtcaatgtc cgccttcagt tgcactttgt cagcggtttc 2340
gtgacgaagc tccaagcggt ttacgccatc aattaaacac aaagtgctgt gccaaaactc 2400
ctctcgcttc ttatttttgt ttgttttttg agtgattggg gtggtgattg gttttgggtg 2460
ggtaagcagg ggaaagtgtg aaaaatcccg gcaatgggcc aagaggatca ggagctatta 2520
attcgcggag gcagcaaaca cccatctgcc gagcatctga acaatgtgag tagtacatgt 2580
gcatacatct taagttcact tgatctatag gaactgcgat tgcaacatca aattgtctgc 2640
ggcgtgagaa ctgcgaccca caaaaatccc aaaccgcaat cgcacaaaca aatagtgaca 2700
cgaaacagat tattctggta gctgtgctcg ctatataaga caatttttaa gatcatatca 2760
tgatcaagac atctaaaggc attcattttc gactacattc ttttttacaa aaaatataac 2820
aaccagatat tttaagctga tcctagatgc acaaaaaata aataaaagta taaacctact 2880
tcgtaggata cttcgttttg ttcggggtta gatgagcata acgcttgtag ttgatatttg 2940
agatccccta tcattgcagg gtgacagcgg agcggcttcg cagagctgca ttaaccaggg 3000
cttcgggcag gccaaaaact acggcacgct cctgccaccc agtccgccgg aggactccgg 3060
ttcagggagc ggccaactag ccgagaacct cacctatgcc tggcacaata tggacatctt 3120
tggggcggtc aatcagccgg gctccggatg gcggcagctg gtcaaccgga cacgcggact 3180
attctgcaac gagcgacaca taccggcgcc caggaaacat ttgctcaaga acggtgagtt 3240
tctattcgca gtcggctgat ctgtgtgaaa tcttaataaa gggtccaatt accaatttga 3300
aactcagttt gcggcgtggc ctatccgggc gaacttttgg ccgtgatggg cagttccggt 3360
gccggaaaga cgaccctgct gaatgccctt gcctttcgat cgccgcaggg catccaagta 3420
tcgccatccg ggatgcgact gctcaatggc caacctgtgg acgccaagga gatgcaggcc 3480
aggtgcgcct atgtccagca ggatgacctc tttatcggct ccctaacggc cagggaacac 3540
ctgattttcc aggccatggt gcggatgcca cgacatctga cctatcggca gcgagtggcc 3600
cgcgtggatc aggtgatcca ggagctttcg ctcagcaaat gtcagcacac gatcatcggt 3660
gtgcccggca gggtgaaagg tctgtccggc ggagaaagga agcgtctggc attcgcctcc 3720
gaggcactaa ccgatccgcc gcttctgatc tgcgatgagc ccacctccgg actggactca 3780
tttaccgccc acagcgtcgt ccaggtgctg aagaagctgt cgcagaaggg caagaccgtc 3840
atcctgacca ttcatcagcc gtcttccgag ctgtttgagc tctttgacaa gatccttctg 3900
atggccgagg gcagggtagc tttcttgggc actcccagcg aagccgtcga cttcttttcc 3960
tagtgagttc gatgtgttta ttaagggtat ctagcattac attacatctc aactcctatc 4020
cagcgtgggt gcccagtgtc ctaccaacta caatccggcg gacttttacg tacaggtgtt 4080
ggccgttgtg cccggacggg agatcgagtc ccgtgatcgg atcgccaaga tatgcgacaa 4140
ttttgctatt agcaaagtag cccgggatat ggagcagttg ttggccacca aaaatttgga 4200

gaagccactg gagcagccgg agaatgggta cacctacaag gccacctggt tcatgcagtt 4260
ccgggcggtc ctgtggcgat cctggctgtc ggtgctcaag gaaccactcc tcgtaaaagt 4320
gcgacttatt cagacaacgg tgagtggttc cagtggaaac aaatgatata acgcttacaa 4380
ttcttggaaa caaattcgct agattttagt tagaattgcc tgattccaca cccttcttag 4440
tttttttcaa tgagatgtat agtttatagt tttgcagaaa ataaataaat ttcatttaac 4500
tcgcgaacat gttgaagata tgaatattaa tgagatgcga gtaacatttt aatttgcaga 4560
tggttgccat cttgattggc ctcatctttt tgggccaaca actcacgcaa gtgggcgtga 4620
tgaatatcaa cggagccatc ttcctcttcc tgaccaacat gacctttcaa aacgtctttg 4680
ccacgataaa tgtaagtctt gtttagaata catttgcata ttaataattt actaactttc 4740
taatgaatcg attcgattta ggtgttcacc tcagagctgc cagtttttat gagggaggcc 4800
cgaagtcgac tttatcgctg tgacacatac tttctgggca aaacgattgc cgaattaccg 4860
ctttttctca cagtgccact ggtcttcacg gcgattgcct atccgatgat cggactgcgg 4920
gccggagtgc tgcacttctt caactgcctg gcgctggtca ctctggtggc caatgtgtca 4980
acgtccttcg gatatctaat atcctgcgcc agctcctcga cctcgatggc gctgtctgtg 5040
ggtccgccgg ttatcatacc attcctgctc tttggcggct tcttcttgaa ctcgggctcg 5100
gtgccagtat acctcaaatg gttgtcgtac ctctcatggt tccgttacgc caacgagggt 5160
ctgctgatta accaatgggc ggacgtggag ccgggcgaaa ttagctgcac atcgtcgaac 5220
accacgtgcc ccagttcggg caaggtcatc ctggagacgc ttaacttctc cgccgccgat 5280
ctgccgctgg actacgtggg tctggccatt ctcatcgtga gcttccgggt gctcgcatat 5340
ctggctctaa gacttcgggc ccgacgcaag gagtagccga catatatccg aaataactgc 5400
ttgttttttt ttttaccatt attaccatcg tgtttactgt ttattgcccc ctcaaaaagc 5460
taatgtaatt atatttgtgc caataaaaac aagatatgac ctatagaata caagtatttc 5520
cccttcgaac atccccacaa gtagactttg gatttgtctt ctaaccaaaa gacttacaca 5580
cctgcatacc ttacatcaaa aactcgttta tcgctacata aaacaccggg atatattttt 5640
tatatacata cttttcaaat cgcgcgccct cttcataatt cacctccacc acaccacgtt 5700
tcgtagttgc tctttcgctg tctcccaccc gctctccgca acacattcac cttttgttcg 5760
acgaccttgg agcgactgtc gttagttccg cgcgattcgg ttcgctcaaa tggttccgag 5820
tggttcattt cgtctcaata gaaattagta ataaatattt gtatgtacaa tttatttgct 5880
ccaatatatt tgtatatatt tccctcacag ctatatttat tctaatttaa tattatgact 5940
ttttaaggta attttttgtg acctgttcgg agtgattagc gttacaattt gaactgaaag 6000
tgacatccag tgtttgttcc ttgtgtagat gcatctcaaa aaaatggtgg gcataatagt 6060
gttgtttata tatatcaaaa ataacaacta taataataag aatacattta atttagaaaa 6120
tgcttggatt tcactggaac tagaattaat tcggctgctg ctctaaacga cgcatttcgt 6180
actccaaagt acgaattttt tccctcaagc tcttattttc attaaacaat gaacaggacc 6240

taacgcacag tcacgttatt gtttacataa atgatttttt ttactattca aacttactct 6300
gtttgtgtac tcccactggt atagccttct tttatctttt ctggttcagg ctctatcact 6360
ttactaggta cggcatctgc gttgagtcgc ctccttttaa atgtctgacc ttttgcaggt 6420
gcagccttcc actgcgaatc attaaagtgg gtatcacaaa tttgggagtt ttcaccaagg 6480
ctgcacccaa ggctctgctc ccacaatttt ctcttaatag cacacttcgg cacgtgaatt 6540
aattttactc cagtcacagc tttgcagcaa aatttgcaat atttcatttt tttttattcc 6600
acgtaagggt taatgttttc aaaaaaaaat tcgtccgcac acaacctttc ctctcaacaa 6660
gcaaacgtgc actgaattta agtgtatact tcggtaagct tcggctatcg acgggaccac 6720
cttatgttat ttcatcatgg gccagaccca cgtagtccag cggcagatcg gcggcggaga 6780
agttaagcgt ctccaggatg accttgcccg aactggggca cgtggtgttc gacgatgtgc 6840
agctaatttc gcccggctcc acgtccgccc attggttaat cagcagaccc tcgttggcgt 6900
aacggaacca tgagaggtac gacaaccatt tgaggtatac tggcaccgag cccgagttca 6960
agaagaaggc gtttttccat aggctccgcc cccctgacga gcatcacaaa aatcgacgct 7020
caagtcagag gtggcgaaac ccgacaggac tataaagata ccaggcgttt ccccctggaa 7080
gctccctcgt gcgctctcct gttccgaccc tgccgcttac cggatacctg tccgcctttc 7140
tcccttcggg aagcgtggcg ctttctcaat gctcacgctg taggtatctc agttcggtgt 7200
aggtcgttcg ctccaagctg ggctgtgtgc acgaaccccc cgttcagccc gaccgctgcg 7260
ccttatccgg taactatcgt cttgagtcca acccggtaag acacgactta tcgccactgg 7320
cagcagccac tggtaacagg attagcagag cgaggtatgt aggcggtgct acagagttct 7380
tgaagtggtg gcctaactac ggctacacta gaaggacagt atttggtatc tgcgctctgc 7440
tgaagccagt taccttcgga aaaagagttg gtagctcttg atccggcaaa caaaccaccg 7500
ctggtagcgg tggttttttt gtttgcaagc agcagattac gcgcagaaaa aaaggatctc 7560
aagaagatcc tttgatcttt tctacggggt ctgacgctca gtggaacgaa aactcacgtt 7620
aagggatttt ggtcatgaga ttatcaaaaa ggatcttcac ctagatcctt ttaaattaaa 7680
aatgaagttt taaatcaatc taaagtatat atgagtaaac ttggtctgac agttaccaat 7740
gcttaatcag tgaggcacct atctcagcga tctgtctatt tcgttcatcc atagttgcct 7800
gactccccgt cgtgtagata actacgatac gggagggctt accatctggc cccagtgctg 7860
caatgatacc gcgagaccca cgctcaccgg ctccagattt atcagcaata aaccagccag 7920
ccggaagggc cgagcgcaga agtggtcctg caactttatc cgcctccatc cagtctatta 7980
attgttgccg ggaagctaga gtaagtagtt cgccagttaa tagtttgcgc aacgttgttg 8040
ccattgctac aggcatcgtg gtgtcacgct cgtcgtttgg tatggcttca ttcagctccg 8100
gttcccaacg atcaaggcga gttacatgat cccccatgtt gtgcaaaaaa gcggttagct 8160
ccttcggtcc tccgatcgtt gtcagaagta agttggccgc agtgttatca ctcatggtta 8220
tggcagcact gcataattct cttactgtca tgccatccgt aagatgcttt tctgtgactg 8280

gtgagtactc aaccaagtca ttctgagaat agtgtatgcg gcgaccgagt tgctcttgcc 8340
cggcgtcaac acgggataat accgcgccac atagcagaac tttaaaagtg ctcatcattg 8400
gaaaacgttc ttcggggcga aaactctcaa ggatcttacc gctgttgaga tccagttcga 8460
tgtaacccac tcgtgcaccc aactgatctt cagcatcttt tactttcacc agcgtttctg 8520
ggtgagcaaa aacaggaagg caaaatgccg caaaaaaggg aataagggcg acacggaaat 8580
gttgaatact catactcttc ctttttcaat attattgaag catttatcag ggttattgtc 8640
tcatgagcgg atacatattt gaatgtattt agaaaaataa acaaataggg gttccgcgca 8700
catttccccg aaaagtgcca cctgacgtct aagaaaccat tattatcatg acattaacct 8760
ataaaaatag gcgtatcacg aggccctttc gtctcgcgcg tttcggtgat gacggtgaaa 8820
acctctgaca catgcagctc ccggagacgg tcacagcttg tctgtaagcg gatgccggga 8880
gcagacaagc ccgtcagggc gcgtcagcgg gtgttggcgg gtgtcggggc tggcttaact 8940
atgcggcatc agagcagatt gtactgagag tgcaccatat gcggtgtgaa ataccgcacc 9000
gaatcgcgcg gaactaacga cagtcgctcc aaggtcgtcg aacaaaaggt gaatgtgttg 9060
cggagagcgg gtgggagaca gcgaaagagc aactacgaaa cgtggtgtgg tggaggtgaa 9120
ttatgaagag ggcgcgcgat ttgaaaagta tgtatataaa aaatatatcc cggtgtttta 9180
tgtagcgata aacgagtttt tgatgtaagg tatgcaggtg tgtaagtctt ttggttagaa 9240
gacaaatcca aagtctactt gtggggatgt tcgaagggga aatacttgta ttctataggt 9300
catatcttgt ttttattggc acaaatataa ttacattagc tttttgaggg ggcaataaac 9360
agtaaacacg atggtaataa tggtaaaaaa aaaaacaagc agttatttcg gatatatgtc 9420
ggctactcct tgcgtcgggc ccgaagtctt agagccagat atgcgagcac ccggaagctc 9480
acgatgagaa tggccagacc atgatgaaat aacataaggt ggtcccgtcg gcaagagaca 9540
tccacttaac gtatgcttgc aataagtgcg agtgaaagga atagtattct gagtgtcgta 9600
ttgagtctga gtgagacagc gatatgattg ttgattaacc cttagcatgt ccgtggggtt 9660
tgaattaact cataatatta attagacgaa attattttta aagttttatt tttaataatt 9720
tgcgagtacg caaagcttct gcatgagctc ggatccaagc ttgcatgcct gcaggtcgga 9780
gtactgtcct ccgagcggag tactgtcctc cgagcggagt actgtcctcc gagcggagta 9840
ctgtcctccg agcggagtac tgtcctccga gcggagactc tagcgagcgc cggagtataa 9900
atagaggcgc ttcgtctacg gagcgacaat tcaattcaaa caagcaaagt gaacacgtcg 9960
ctaagcgaaa gctaagcaaa taaacaagcg cagctgaaca agctaaacaa tctgcagtaa 10020
agtgcaagtt aaagtgaatc aattaaaagt aaccagcaac caagtaaatc aactgcaact 10080
actgaaatct gccaagaagt aattattgaa tacaagaaga gaactctgaa tagggaattg 10140
g 10141

<210> 22
<211> 3864

<212>  DNA
<213>  artificial

<220>
<223>  pRmHa-3

<400>  22
gaattcgagc tcgctagcaa gcttggtacc cggggatcct ctagagtcga cctgcaggca      60

tgcaagctat tcgatgcaca ctcacattct tctcctaata cgataataaa actttccatg     120

aaaaatatgg aaaaatatat gaaaattgag aaatccaaaa aactgataaa cgctctactt     180

aattaaaata gataaatggg agcggcagga atggcggagc atggccaagt tcctccgcca     240

atcagtcgta aaacagaagt cgtggaaagc ggatagaaag aatgttcgat ttgacgggca     300

agcatgtctg ctatgtggcg gattgcggag gaattgcact ggagaccagc aaggttctca     360

tgaccaagaa tatagcggtg agtgagcggg aagctcggtt tctgtccaga tcgaactcaa     420

aactagtcca gccagtcgct gtcgaaacta attaagtaaa tgagtttttc atgttagttt     480

cgcgctgagc aacaattaag tttatgtttc agttcggctt agatttcgct gaaggacttg     540

ccactttcaa tcaatacttt agaacaaaat caaaactcat tctaatagct tggtgttcat     600

cttttttttt aatgataagc attttgtcgt ttatactttt tatatttcga tattaaacca     660

cctatgaagt tcattttaat cgccagataa gcaatatatt gtgtaaatat ttgtattctt     720

tatcaggaaa ttcagggaga cggggaagtt actatctact aaaagccaaa caatttctta     780

cagttttact ctctctactc tagagtagct tggcactggc cgtcgtttta caacgtcgtg     840

actgggaaaa ccctggcgtt acccaactta atcgccttgc agcacatccc cctttcgcca     900

gctggcgtaa tagcgaagag gcccgcaccg atcgcccttc ccaacagttg cgcagcctga     960

atggcgaatg gcgcctgatg cggtattttc tccttacgca tctgtgcggt atttcacacc    1020

gcatatggtg cactctcagt acaatctgct ctgatgccgc atagttaagc cagccccgac    1080

acccgccaac acccgctgac gcgccctgac gggcttgtct gctcccggca tccgcttaca    1140

gacaagctgt gaccgtctcc gggagctgca tgtgtcagag gttttcaccg tcatcaccga    1200

aacgcgcgag acgaaagggc ctcgtgatac gcctattttt ataggttaat gtcatgataa    1260

taatggtttc ttagacgtca ggtggcactt ttcggggaaa tgtgcgcgga acccctattt    1320

gtttattttt ctaaatacat tcaaatatgt atccgctcat gagacaataa ccctgataaa    1380

tgcttcaata atattgaaaa aggaagagta tgagtattca acatttccgt gtcgccctta    1440

ttcccttttt tgcggcattt tgccttcctg tttttgctca cccagaaacg ctggtgaaag    1500

taaaagatgc tgaagatcag ttgggtgcac gagtgggtta catcgaactg gatctcaaca    1560

gcggtaagat ccttgagagt tttcgccccg aagaacgttt tccaatgatg agcactttta    1620

aagttctgct atgtggcgcg gtattatccc gtattgacgc cgggcaagag caactcggtc    1680

gccgcataca ctattctcag aatgacttgg ttgagtactc accagtcaca gaaaagcatc    1740

ttacggatgg catgacagta agagaattat gcagtgctgc cataaccatg agtgataaca    1800

ctgcggccaa cttacttctg acaacgatcg gaggaccgaa ggagctaacc gcttttttgc    1860

acaacatggg ggatcatgta actcgccttg atcgttggga accggagctg aatgaagcca 1920
taccaaacga cgagcgtgac accacgatgc ctgtagcaat ggcaacaacg ttgcgcaaac 1980
tattaactgg cgaactactt actctagctt cccggcaaca attaatagac tggatggagg 2040
cggataaagt tgcaggacca cttctgcgct cggcccttcc ggctggctgg tttattgctg 2100
ataaatctgg agccggtgag cgtgggtctc gcggtatcat tgcagcactg gggccagatg 2160
gtaagccctc ccgtatcgta gttatctaca cgacggggag tcaggcaact atggatgaac 2220
gaaatagaca gatcgctgag ataggtgcct cactgattaa gcattggtaa ctgtcagacc 2280
aagtttactc atatatactt tagattgatt taaaacttca tttttaattt aaaaggatct 2340
aggtgaagat cctttttgat aatctcatga ccaaaatccc ttaacgtgag ttttcgttcc 2400
actgagcgtc agaccccgta gaaaagatca aaggatcttc ttgagatcct ttttttctgc 2460
gcgtaatctg ctgcttgcaa acaaaaaaac caccgctacc agcggtggtt tgtttgccgg 2520
atcaagagct accaactctt tttccgaagg taactggctt cagcagagcg cagataccaa 2580
atactgtcct tctagtgtag ccgtagttag gccaccactt caagaactct gtagcaccgc 2640
ctacatacct cgctctgcta atcctgttac cagtggctgc tgccagtggc gataagtcgt 2700
gtcttaccgg gttggactca agacgatagt taccggataa ggcgcagcgg tcgggctgaa 2760
cggggggttc gtgcacacag cccagcttgg agcgaacgac ctacaccgaa ctgagatacc 2820
tacagcgtga gctatgagaa agcgccacgc ttcccgaagg gagaaaggcg gacaggtatc 2880
cggtaagcgg cagggtcgga acaggagagc gcacgaggga gcttccaggg ggaaacgcct 2940
ggtatcttta tagtcctgtc gggtttcgcc acctctgact tgagcgtcga tttttgtgat 3000
gctcgtcagg ggggcggagc ctatggaaaa acgccagcaa cgcggccttt ttacggttcc 3060
tggccttttg ctggcctttt gctcacatgt tctttcctgc gttatcccct gattctgtgg 3120
ataaccgtat taccgccttt gagtgagctg ataccgctcg ccgcagccga acgaccgagc 3180
gcagcgagtc agtgagcgag gaagcggaag agcgcccaat acgcaaaccg cctctccccg 3240
cgcgttggcc gattcattaa tgcagctggc acgacaggtt tcccgactgg aaagcgggca 3300
gtgagcgcaa cgcaattaat gtgagttagc tcactcatta ggcaccccag gctttacact 3360
ttatgcttcc ggctcgtatg ttgtgtggaa ttgtgagcgg ataacaattt cacacaggaa 3420
acagctatga ccatgattac gcgttgcagg acaggatgtg gtgcccgatg tgactagctc 3480
tttgctgcag gccgtcctat cctctggttc cgataagaga cccagaactc cggcccccca 3540
ccgcccaccg ccacccccat acatatgtgg tacgcaagta agagtgcctg cgcatgcccc 3600
atgtgcccca ccaagagttt tgcatcccat acaagtcccc aaagtggaga accgaaccaa 3660
ttcttcgcgg gcagaacaaa agcttctgca cacgtctcca ctcgaatttg gagccggccg 3720
gcgtgtgcaa aagaggtgaa tcgaacgaaa gacccgtgtg taaagccgcg tttccaaaat 3780
gtataaaacc gagagcatct ggccaatgtg catcagttgt ggtcagcagc aaaatcaagt 3840
gaatcatctc agtgcaacta aagg 3864

<210>   23
<211>   9050
<212>   DNA
<213>   artificial

<220>
<223>   pUAST

<400>   23
   gaattcgtta acagatctgc ggccgcggct cgagggtacc tctagaggat ctttgtgaag      60
   gaaccttact tctgtggtgt gacataattg gacaaactac ctacagagat ttaaagctct     120
   aaggtaaata taaaattttt aagtgtataa tgtgttaaac tactgattct aattgtttgt     180
   gtattttaga ttccaaccta tggaactgat gaatgggagc agtggtggaa tgcctttaat     240
   gaggaaaacc tgttttgctc agaagaaatg ccatctagtg atgatgaggc tactgctgac     300
   tctcaacatt ctactcctcc aaaaaagaag agaaaggtag aagaccccaa ggactttcct     360
   tcagaattgc taagtttttt gagtcatgct gtgtttagta atagaactct tgcttgcttt     420
   gctatttaca ccacaaagga aaaagctgca ctgctataca agaaaattat ggaaaaatat     480
   tctgtaacct ttataagtag gcataacagt tataatcata acatactgtt ttttcttact     540
   ccacacaggc atagagtgtc tgctattaat aactatgctc aaaaattgtg tacctttagc     600
   tttttaattt gtaaaggggt taataaggaa tatttgatgt atagtgcctt gactagagat     660
   cataatcagc cataccacat ttgtagaggt tttacttgct ttaaaaaacc tcccacacct     720
   ccccctgaac ctgaaacata aaatgaatgc aattgttgtt gttaacttgt ttattgcagc     780
   ttataatggt tacaaataaa gcaatagcat cacaaatttc acaaataaag catttttttc     840
   actgcattct agttgtggtt tgtccaaact catcaatgta tcttatcatg tctggatcgg     900
   atccactaga aggccttagt atgtatgtaa gttaataaaa cccttttttg gagaatgtag     960
   atttaaaaaa acatattttt tttttatttt ttactgcact ggacatcatt gaacttatct    1020
   gatcagtttt aaatttactt cgatccaagg gtatttgaag taccaggttc tttcgattac    1080
   ctctcactca aaatgacatt ccactcaaag tcagcgctgt ttgcctcctt ctctgtccac    1140
   agaaatatcg ccgtctcttt cgccgctgcg tccgctatct ctttcgccac cgtttgtagc    1200
   gttacctagc gtcaatgtcc gccttcagtt gcactttgtc agcggtttcg tgacgaagct    1260
   ccaagcggtt tacgccatca attaaacaca aagtgctgtg ccaaaactcc tctcgcttct    1320
   tatttttgtt tgttttttga gtgattgggg tggtgattgg ttttgggtgg gtaagcaggg    1380
   gaaagtgtga aaaatcccgg caatgggcca agaggatcag gagctattaa ttcgcggagg    1440
   cagcaaacac ccatctgccg agcatctgaa caatgtgagt agtacatgtg catacatctt    1500
   aagttcactt gatctatagg aactgcgatt gcaacatcaa attgtctgcg gcgtgagaac    1560
   tgcgacccac aaaaatccca aaccgcaatc gcacaaacaa atagtgacac gaaacagatt    1620
   attctggtag ctgtgctcgc tatataagac aatttttaag atcatatcat gatcaagaca    1680
   tctaaaggca ttcattttcg actacattct tttttacaaa aaatataaca accagatatt    1740
   ttaagctgat cctagatgca caaaaaataa ataaaagtat aaacctactt cgtaggatac 1800
   ttcgttttgt tcggggttag atgagcataa cgcttgtagt tgatatttga gatcccctat 1860
   cattgcaggg tgacagcgga gcggcttcgc agagctgcat taaccagggc ttcgggcagg 1920
   ccaaaaacta cggcacgctc ctgccaccca gtccgccgga ggactccggt tcagggagcg 1980
   gccaactagc cgagaacctc acctatgcct ggcacaatat ggacatcttt ggggcggtca 2040
   atcagccggg ctccggatgg cggcagctgg tcaaccggac acgcggacta ttctgcaacg 2100
   agcgacacat accggcgccc aggaaacatt tgctcaagaa cggtgagttt ctattcgcag 2160
   tcggctgatc tgtgtgaaat cttaataaag ggtccaatta ccaatttgaa actcagtttg 2220
   cggcgtggcc tatccgggcg aacttttggc cgtgatgggc agttccggtg ccggaaagac 2280
   gaccctgctg aatgcccttg cctttcgatc gccgcagggc atccaagtat cgccatccgg 2340
   gatgcgactg ctcaatggcc aacctgtgga cgccaaggag atgcaggcca ggtgcgccta 2400
   tgtccagcag gatgacctct ttatcggctc cctaacggcc agggaacacc tgattttcca 2460
   ggccatggtg cggatgccac gacatctgac ctatcggcag cgagtggccc gcgtggatca 2520
   ggtgatccag gagctttcgc tcagcaaatg tcagcacacg atcatcggtg tgcccggcag 2580
   ggtgaaaggt ctgtccggcg gagaaaggaa gcgtctggca ttcgcctccg aggcactaac 2640
   cgatccgccg cttctgatct gcgatgagcc cacctccgga ctggactcat ttaccgccca 2700
   cagcgtcgtc caggtgctga agaagctgtc gcagaagggc aagaccgtca tcctgaccat 2760
   tcatcagccg tcttccgagc tgtttgagct ctttgacaag atccttctga tggccgaggg 2820
   cagggtagct ttcttgggca ctcccagcga agccgtcgac ttcttttcct agtgagttcg 2880
   atgtgtttat taagggtatc tagcattaca ttacatctca actcctatcc agcgtgggtg 2940
   cccagtgtcc taccaactac aatccggcgg acttttacgt acaggtgttg gccgttgtgc 3000
   ccggacggga gatcgagtcc cgtgatcgga tcgccaagat atgcgacaat tttgctatta 3060
   gcaaagtagc ccgggatatg gagcagttgt tggccaccaa aaatttggag aagccactgg 3120
   agcagccgga gaatgggtac acctacaagg ccacctggtt catgcagttc cgggcggtcc 3180
   tgtggcgatc ctggctgtcg gtgctcaagg aaccactcct cgtaaaagtg cgacttattc 3240
   agacaacggt gagtggttcc agtggaaaca aatgatataa cgcttacaat tcttggaaac 3300
   aaattcgcta gattttagtt agaattgcct gattccacac ccttcttagt ttttttcaat 3360
   gagatgtata gtttatagtt ttgcagaaaa taaataaatt tcatttaact cgcgaacatg 3420
   ttgaagatat gaatattaat gagatgcgag taacatttta atttgcagat ggttgccatc 3480
   ttgattggcc tcatcttttt gggccaacaa ctcacgcaag tgggcgtgat gaatatcaac 3540
   ggagccatct tcctcttcct gaccaacatg acctttcaaa acgtctttgc cacgataaat 3600
   gtaagtcttg tttagaatac atttgcatat taataattta ctaactttct aatgaatcga 3660
   ttcgatttag gtgttcacct cagagctgcc agtttttatg agggaggccc gaagtcgact 3720
   ttatcgctgt gacacatact ttctgggcaa aacgattgcc gaattaccgc tttttctcac 3780
   agtgccactg gtcttcacgg cgattgccta tccgatgatc ggactgcggg ccggagtgct 3840
   gcacttcttc aactgcctgg cgctggtcac tctggtggcc aatgtgtcaa cgtccttcgg 3900
   atatctaata tcctgcgcca gctcctcgac ctcgatggcg ctgtctgtgg gtccgccggt 3960
   tatcatacca ttcctgctct ttggcggctt cttcttgaac tcgggctcgg tgccagtata 4020
   cctcaaatgg ttgtcgtacc tctcatggtt ccgttacgcc aacgagggtc tgctgattaa 4080
   ccaatgggcg gacgtggagc cgggcgaaat tagctgcaca tcgtcgaaca ccacgtgccc 4140
   cagttcgggc aaggtcatcc tggagacgct taacttctcc gccgccgatc tgccgctgga 4200
   ctacgtgggt ctggccattc tcatcgtgag cttccgggtg ctcgcatatc tggctctaag 4260
   acttcggccc cgacgcaagg agtagccgac atatatccga aataactgct tgtttttttt 4320
   tttaccatta ttaccatcgt gtttactgtt tattgccccc tcaaaaagct aatgtaatta 4380
   tatttgtgcc aataaaaaca agatatgacc tatagaatac aagtatttcc ccttcgaaca 4440
   tccccacaag tagactttgg atttgtcttc taaccaaaag acttacacac ctgcatacct 4500
   tacatcaaaa actcgtttat cgctacataa aacaccggga tatatttttt atatacatac 4560
   ttttcaaatc gcgcgccctc ttcataattc acctccacca caccacgttt cgtagttgct 4620
   ctttcgctgt ctcccacccg ctctccgcaa cacattcacc ttttgttcga cgaccttgga 4680
   gcgactgtcg ttagttccgc gcgattcggt tcgctcaaat ggttccgagt ggttcatttc 4740
   gtctcaatag aaattagtaa taaatatttg tatgtacaat ttatttgctc caatatattt 4800
   gtatatattt ccctcacagc tatatttatt ctaatttaat attatgactt tttaaggtaa 4860
   ttttttgtga cctgttcgga gtgattagcg ttacaatttg aactgaaagt gacatccagt 4920
   gtttgttcct tgtgtagatg catctcaaaa aaatggtggg cataatagtg ttgtttatat 4980
   atatcaaaaa taacaactat aataataaga atacatttaa tttagaaaat gcttggattt 5040
   cactggaact agaattaatt cggctgctgc tctaaacgac gcatttcgta ctccaaagta 5100
   cgaatttttt ccctcaagct cttattttca ttaaacaatg aacaggacct aacgcacagt 5160
   cacgttattg tttacataaa tgattttttt tactattcaa acttactctg tttgtgtact 5220
   cccactggta tagccttctt ttatcttttc tggttcaggc tctatcactt tactaggtac 5280
   ggcatctgcg ttgagtcgcc tccttttaaa tgtctgacct tttgcaggtg cagccttcca 5340
   ctgcgaatca ttaaagtggg tatcacaaat ttgggagttt tcaccaaggc tgcacccaag 5400
   gctctgctcc cacaattttc tcttaatagc acacttcggc acgtgaatta attttactcc 5460
   agtcacagct ttgcagcaaa atttgcaata tttcattttt ttttattcca cgtaagggtt 5520
   aatgttttca aaaaaaaatt cgtccgcaca caacctttcc tctcaacaag caaacgtgca 5580
   ctgaatttaa gtgtatactt cggtaagctt cggctatcga cgggaccacc ttatgttatt 5640
   tcatcatggg ccagacccac gtagtccagc ggcagatcgg cggcggagaa gttaagcgtc 5700
   tccaggatga ccttgcccga actggggcac gtggtgttcg acgatgtgca gctaatttcg 5760
   cccggctcca cgtccgccca ttggttaatc agcagaccct cgttggcgta acggaaccat 5820
   gagaggtacg acaaccattt gaggtatact ggcaccgagc ccgagttcaa gaagaaggcg 5880
   tttttccata ggctccgccc ccctgacgag catcacaaaa atcgacgctc aagtcagagg 5940
   tggcgaaacc cgacaggact ataaagatac caggcgtttc cccctggaag ctccctcgtg 6000
   cgctctcctg ttccgaccct gccgcttacc ggatacctgt ccgcctttct cccttcggga 6060
   agcgtggcgc tttctcaatg ctcacgctgt aggtatctca gttcggtgta ggtcgttcgc 6120
   tccaagctgg gctgtgtgca cgaacccccc gttcagcccg accgctgcgc cttatccggt 6180
   aactatcgtc ttgagtccaa cccggtaaga cacgacttat cgccactggc agcagccact 6240
   ggtaacagga ttagcagagc gaggtatgta ggcggtgcta cagagttctt gaagtggtgg 6300
   cctaactacg gctacactag aaggacagta tttggtatct gcgctctgct gaagccagtt 6360
   accttcggaa aaagagttgg tagctcttga tccggcaaac aaaccaccgc tggtagcggt 6420
   ggtttttttg tttgcaagca gcagattacg cgcagaaaaa aaggatctca agaagatcct 6480
   ttgatctttt ctacggggtc tgacgctcag tggaacgaaa actcacgtta agggattttg 6540
   gtcatgagat tatcaaaaag gatcttcacc tagatccttt taaattaaaa atgaagtttt 6600
   aaatcaatct aaagtatata tgagtaaact tggtctgaca gttaccaatg cttaatcagt 6660
   gaggcaccta tctcagcgat ctgtctattt cgttcatcca tagttgcctg actccccgtc 6720
   gtgtagataa ctacgatacg ggagggctta ccatctggcc ccagtgctgc aatgataccg 6780
   cgagacccac gctcaccggc tccagattta tcagcaataa accagccagc cggaagggcc 6840
   gagcgcagaa gtggtcctgc aactttatcc gcctccatcc agtctattaa ttgttgccgg 6900
   gaagctagag taagtagttc gccagttaat agtttgcgca acgttgttgc cattgctaca 6960
   ggcatcgtgg tgtcacgctc gtcgtttggt atggcttcat tcagctccgg ttcccaacga 7020
   tcaaggcgag ttacatgatc ccccatgttg tgcaaaaaag cggttagctc cttcggtcct 7080
   ccgatcgttg tcagaagtaa gttggccgca gtgttatcac tcatggttat ggcagcactg 7140
   cataattctc ttactgtcat gccatccgta agatgctttt ctgtgactgg tgagtactca 7200
   accaagtcat tctgagaata gtgtatgcgg cgaccgagtt gctcttgccc ggcgtcaaca 7260
   cgggataata ccgcgccaca tagcagaact ttaaaagtgc tcatcattgg aaaacgttct 7320
   tcggggcgaa aactctcaag gatcttaccg ctgttgagat ccagttcgat gtaacccact 7380
   cgtgcaccca actgatcttc agcatctttt actttcacca gcgtttctgg gtgagcaaaa 7440
   acaggaaggc aaaatgccgc aaaaaaggga ataagggcga cacggaaatg ttgaatactc 7500
   atactcttcc tttttcaata ttattgaagc atttatcagg gttattgtct catgagcgga 7560
   tacatatttg aatgtattta gaaaaataaa caaatagggg ttccgcgcac atttccccga 7620
   aaagtgccac ctgacgtcta agaaaccatt attatcatga cattaaccta taaaaatagg 7680
   cgtatcacga ggccctttcg tctcgcgcgt ttcggtgatg acggtgaaaa cctctgacac 7740
   atgcagctcc cggagacggt cacagcttgt ctgtaagcgg atgccgggag cagacaagcc 7800
   cgtcagggcg cgtcagcggg tgttggcggg tgtcggggct ggcttaacta tgcggcatca 7860
   gagcagattg tactgagagt gcaccatatg cggtgtgaaa taccgcaccg aatcgcgcgg 7920
   aactaacgac agtcgctcca aggtcgtcga acaaaaggtg aatgtgttgc ggagagcggg 7980
   tgggagacag cgaaagagca actacgaaac gtggtgtggt ggaggtgaat tatgaagagg 8040
   gcgcgcgatt tgaaaagtat gtatataaaa aatatatccc ggtgttttat gtagcgataa 8100
   acgagttttt gatgtaaggt atgcaggtgt gtaagtcttt tggttagaag acaaatccaa 8160
   agtctacttg tggggatgtt cgaaggggaa atacttgtat tctataggtc atatcttgtt 8220
   tttattggca caaatataat tacattagct ttttgagggg gcaataaaca gtaaacacga 8280
   tggtaataat ggtaaaaaaa aaaacaagca gttatttcgg atatatgtcg gctactcctt 8340
   gcgtcgggcc cgaagtctta gagccagata tgcgagcacc cggaagctca cgatgagaat 8400
   ggccagacca tgatgaaata acataaggtg gtcccgtcgg caagagacat ccacttaacg 8460
   tatgcttgca ataagtgcga gtgaaaggaa tagtattctg agtgtcgtat tgagtctgag 8520
   tgagacagcg atatgattgt tgattaaccc ttagcatgtc cgtggggttt gaattaactc 8580
   ataatattaa ttagacgaaa ttatttttaa agttttattt ttaataattt gcgagtacgc 8640
   aaagcttctg catgagctcg gatccaagct tgcatgcctg caggtcggag tactgtcctc 8700
   cgagcggagt actgtcctcc gagcggagta ctgtcctccg agcggagtac tgtcctccga 8760
   gcggagtact gtcctccgag cggagactct agcgagcgcc ggagtataaa tagaggcgct 8820
   tcgtctacgg agcgacaatt caattcaaac aagcaaagtg aacacgtcgc taagcgaaag 8880
   ctaagcaaat aaacaagcgc agctgaacaa gctaaacaat ctgcagtaaa gtgcaagtta 8940
   aagtgaatca attaaaagta accagcaacc aagtaaatca actgcaacta ctgaaatctg 9000
   ccaagaagta attattgaat acaagaagag aactctgaat agggaattgg 9050

<210> 24
<211> 4938
<212> DNA
<213> artificial

<220>
<223> pAT195

<400> 24
gaattcgagc tcgctagcgc taccggtcgc caccatggtg agcaagggcg aggagctgtt 60

caccggggtg gtgcccatcc tggtcgagct ggacggcgac gtaaacggcc acaagttcag 120

cgtgtccggc gagggcgagg gcgatgccac ctacggcaag ctgaccctga agttcatctg 180

caccaccggc aagctgcccg tgccctggcc caccctcgtg accaccctga cctacggcgt 240

gcagtgcttc agccgctacc ccgaccacat gaagcagcac gacttcttca agtccgccat 300

gcccgaaggc tacgtccagg agcgcaccat cttcttcaag gacgacggca actacaagac 360

ccgcgccgag gtgaagttcg agggcgacac cctggtgaac cgcatcgagc tgaagggcat 420

cgacttcaag gaggacggca acatcctggg gcacaagctg gagtacaact acaacagcca 480

caacgtctat atcatggccg acaagcagaa gaacggcatc aaggtgaact tcaagatccg 540

ccacaacatc gaggacggca gcgtgcagct cgccgaccac taccagcaga acacccccat 600
cggcgacggc cccgtgctgc tgcccgacaa ccactacctg agcacccagt ccgccctgag 660
caaagacccc aacgagaagc gcgatcacat ggtcctgctg gagttcgtga ccgccgccgg 720
gatcactctc ggcatggacg agctgtacaa gtccggactc agatctcgag gctttctcaa 780
tggcagcaat cagcccatga tcgagggcca gctgaaggag aagaagggca agtggcgact 840
attcaggcgc tggcgaacgc gctacttcac tttgtccgga gcgcacttgt cctgcaaagg 900
ctctagcgga ggtgagagca tcgatgtgaa tcagatacgt tcggtaaagg tatctcgtgg 960
tgcccgtaac ataccgaagg ccttcgaaat cttcaccgcc gaccagacat taatactcaa 1020
gcccaaggac ggcaagaatg ccgaggagtg ggtgcagtgc ctcagcatcg tggtagccca 1080
ctcgcaggcc cgagataatc ccacgtagga tcctctagag tcgacctgca ggcatgcaag 1140
ctattcgatg cacactcaca ttcttctcct aatacgataa taaaactttc catgaaaaat 1200
atggaaaaat atatgaaaat tgagaaatcc aaaaaactga taaacgctct acttaattaa 1260
aatagataaa tgggagcggc aggaatggcg gagcatggcc aagttcctcc gccaatcagt 1320
cgtaaaacag aagtcgtgga aagcggatag aaagaatgtt cgatttgacg ggcaagcatg 1380
tctgctatgt ggcggattgc ggaggaattg cactggagac cagcaaggtt ctcatgacca 1440
agaatatagc ggtgagtgag cgggaagctc ggtttctgtc cagatcgaac tcaaaactag 1500
tccagccagt cgctgtcgaa actaattaag taaatgagtt tttcatgtta gtttcgcgct 1560
gagcaacaat taagtttatg tttcagttcg gcttagattt cgctgaagga cttgccactt 1620
tcaatcaata ctttagaaca aaatcaaaac tcattctaat agcttggtgt tcatcttttt 1680
ttttaatgat aagcattttg tcgtttatac tttttatatt tcgatattaa accacctatg 1740
aagttcattt taatcgccag ataagcaata tattgtgtaa atatttgtat tctttatcag 1800
gaaattcagg gagacgggga agttactatc tactaaaagc caaacaattt cttacagttt 1860
tactctctct actctagagt agcttggcac tggccgtcgt tttacaacgt cgtgactggg 1920
aaaaccctgg cgttacccaa cttaatcgcc ttgcagcaca tccccctttc gccagctggc 1980
gtaatagcga agaggcccgc accgatcgcc cttcccaaca gttgcgcagc ctgaatggcg 2040
aatggcgcct gatgcggtat tttctcctta cgcatctgtg cggtatttca caccgcatat 2100
ggtgcactct cagtacaatc tgctctgatg ccgcatagtt aagccagccc cgacacccgc 2160
caacacccgc tgacgcgccc tgacgggctt gtctgctccc ggcatccgct tacagacaag 2220
ctgtgaccgt ctccgggagc tgcatgtgtc agaggttttc accgtcatca ccgaaacgcg 2280
cgagacgaaa gggcctcgtg atacgcctat ttttataggt taatgtcatg ataataatgg 2340
tttcttagac gtcaggtggc acttttcggg gaaatgtgcg cggaacccct atttgtttat 2400
ttttctaaat acattcaaat atgtatccgc tcatgagaca ataaccctga taaatgcttc 2460
aataatattg aaaaaggaag agtatgagta ttcaacattt ccgtgtcgcc cttattccct 2520
tttttgcggc attttgcctt cctgtttttg ctcacccaga aacgctggtg aaagtaaaag 2580

atgctgaaga tcagttgggt gcacgagtgg gttacatcga actggatctc aacagcggta 2640
agatccttga gagttttcgc cccgaagaac gttttccaat gatgagcact tttaaagttc 2700
tgctatgtgg cgcggtatta tcccgtattg acgccgggca agagcaactc ggtcgccgca 2760
tacactattc tcagaatgac ttggttgagt actcaccagt cacagaaaag catcttacgg 2820
atggcatgac agtaagagaa ttatgcagtg ctgccataac catgagtgat aacactgcgg 2880
ccaacttact tctgacaacg atcggaggac cgaaggagct aaccgctttt ttgcacaaca 2940
tgggggatca tgtaactcgc cttgatcgtt gggaaccgga gctgaatgaa gccataccaa 3000
acgacgagcg tgacaccacg atgcctgtag caatggcaac aacgttgcgc aaactattaa 3060
ctggcgaact acttactcta gcttcccggc aacaattaat agactggatg gaggcggata 3120
aagttgcagg accacttctg cgctcggccc ttccggctgg ctggtttatt gctgataaat 3180
ctggagccgg tgagcgtggg tctcgcggta tcattgcagc actggggcca gatggtaagc 3240
cctcccgtat cgtagttatc tacacgacgg ggagtcaggc aactatggat gaacgaaata 3300
gacagatcgc tgagataggt gcctcactga ttaagcattg gtaactgtca gaccaagttt 3360
actcatatat actttagatt gatttaaaac ttcattttta atttaaaagg atctaggtga 3420
agatcctttt tgataatctc atgaccaaaa tcccttaacg tgagttttcg ttccactgag 3480
cgtcagaccc cgtagaaaag atcaaaggat cttcttgaga tccttttttt ctgcgcgtaa 3540
tctgctgctt gcaaacaaaa aaaccaccgc taccagcggt ggtttgtttg ccggatcaag 3600
agctaccaac tctttttccg aaggtaactg gcttcagcag agcgcagata ccaaatactg 3660
tccttctagt gtagccgtag ttaggccacc acttcaagaa ctctgtagca ccgcctacat 3720
acctcgctct gctaatcctg ttaccagtgg ctgctgccag tggcgataag tcgtgtctta 3780
ccgggttgga ctcaagacga tagttaccgg ataaggcgca gcggtcgggc tgaacggggg 3840
gttcgtgcac acagcccagc ttggagcgaa cgacctacac cgaactgaga tacctacagc 3900
gtgagctatg agaaagcgcc acgcttcccg aagggagaaa ggcggacagg tatccggtaa 3960
gcggcagggt cggaacagga gagcgcacga gggagcttcc agggggaaac gcctggtatc 4020
tttatagtcc tgtcgggttt cgccacctct gacttgagcg tcgatttttg tgatgctcgt 4080
caggggggcg gagcctatgg aaaaacgcca gcaacgcggc ctttttacgg ttcctggcct 4140
tttgctggcc ttttgctcac atgttctttc ctgcgttatc ccctgattct gtggataacc 4200
gtattaccgc ctttgagtga gctgataccg ctcgccgcag ccgaacgacc gagcgcagcg 4260
agtcagtgag cgaggaagcg gaagagcgcc caatacgcaa accgcctctc cccgcgcgtt 4320
ggccgattca ttaatgcagc tggcacgaca ggtttcccga ctggaaagcg ggcagtgagc 4380
gcaacgcaat taatgtgagt tagctcactc attaggcacc ccaggcttta cactttatgc 4440
ttccggctcg tatgttgtgt ggaattgtga gcggataaca atttcacaca ggaaacagct 4500
atgaccatga ttacgcgttg caggacagga tgtggtgccc gatgtgacta gctctttgct 4560
gcaggccgtc ctatcctctg gttccgataa gagacccaga actccggccc cccaccgccc 4620

accgccaccc ccatacatat gtggtacgca agtaagagtg cctgcgcatg ccccatgtgc 4680

cccaccaaga gttttgcatc ccatacaagt ccccaaagtg gagaaccgaa ccaattcttc 4740

gcgggcagaa caaaagcttc tgcacacgtc tccactcgaa tttggagccg gccggcgtgt 4800

gcaaaagagg tgaatcgaac gaaagacccg tgtgtaaagc cgcgtttcca aaatgtataa 4860

aaccgagagc atctggccaa tgtgcatcag ttgtggtcag cagcaaaatc aagtgaatca 4920

tctcagtgca actaaagg 4938

<210> 25
<211> 3666
<212> DNA
<213> artificial

<220>
<223> pET23a

<400> 25
ggatccgaat tcgagctccg tcgacaagct tgcggccgca ctcgagcacc accaccacca 60

ccactgagat ccggctgcta acaaagcccg aaaggaagct gagttggctg ctgccaccgc 120

tgagcaataa ctagcataac cccttggggc ctctaaacgg gtcttgaggg gttttttgct 180

gaaaggagga actatatccg gattggcgaa tgggacgcgc cctgtagcgg cgcattaagc 240

gcggcgggtg tggtggttac gcgcagcgtg accgctacac ttgccagcgc cctagcgccc 300

gctcctttcg ctttcttccc ttcctttctc gccacgttcg ccggctttcc ccgtcaagct 360

ctaaatcggg ggctcccttt agggttccga tttagtgctt tacggcacct cgaccccaaa 420

aaacttgatt agggtgatgg ttcacgtagt gggccatcgc cctgatagac ggtttttcgc 480

cctttgacgt tggagtccac gttctttaat agtggactct tgttccaaac tggaacaaca 540

ctcaacccta tctcggtcta ttcttttgat ttataaggga ttttgccgat ttcggcctat 600

tggttaaaaa atgagctgat ttaacaaaaa tttaacgcga attttaacaa aatattaacg 660

tttacaattt caggtggcac ttttcgggga aatgtgcgcg gaacccctat ttgtttattt 720

ttctaaatac attcaaatat gtatccgctc atgagacaat aaccctgata aatgcttcaa 780

taatattgaa aaaggaagag tatgagtatt caacatttcc gtgtcgccct tattcccttt 840

tttgcggcat tttgccttcc tgtttttgct cacccagaaa cgctggtgaa agtaaaagat 900

gctgaagatc agttgggtgc acgagtgggt tacatcgaac tggatctcaa cagcggtaag 960

atccttgaga gttttcgccc cgaagaacgt tttccaatga tgagcacttt taaagttctg 1020

ctatgtggcg cggtattatc ccgtattgac gccgggcaag agcaactcgg tcgccgcata 1080

cactattctc agaatgactt ggttgagtac tcaccagtca cagaaaagca tcttacggat 1140

ggcatgacag taagagaatt atgcagtgct gccataacca tgagtgataa cactgcggcc 1200

aacttacttc tgacaacgat cggaggaccg aaggagctaa ccgctttttt gcacaacatg 1260

ggggatcatg taactcgcct tgatcgttgg gaaccggagc tgaatgaagc cataccaaac 1320

gacgagcgtg acaccacgat gcctgcagca atggcaacaa cgttgcgcaa actattaact 1380

ggcgaactac ttactctagc ttcccggcaa caattaatag actggatgga ggcggataaa 1440
gttgcaggac cacttctgcg ctcggccctt ccggctggct ggtttattgc tgataaatct 1500
ggagccggtg agcgtgggtc tcgcggtatc attgcagcac tggggccaga tggtaagccc 1560
tcccgtatcg tagttatcta cacgacgggg agtcaggcaa ctatggatga acgaaataga 1620
cagatcgctg agataggtgc ctcactgatt aagcattggt aactgtcaga ccaagtttac 1680
tcatatatac tttagattga tttaaaactt catttttaat ttaaaaggat ctaggtgaag 1740
atcctttttg ataatctcat gaccaaaatc ccttaacgtg agttttcgtt ccactgagcg 1800
tcagaccccg tagaaaagat caaaggatct tcttgagatc ctttttttct gcgcgtaatc 1860
tgctgcttgc aaacaaaaaa accaccgcta ccagcggtgg tttgtttgcc ggatcaagag 1920
ctaccaactc tttttccgaa ggtaactggc ttcagcagag cgcagatacc aaatactgtc 1980
cttctagtgt agccgtagtt aggccaccac ttcaagaact ctgtagcacc gcctacatac 2040
ctcgctctgc taatcctgtt accagtggct gctgccagtg gcgataagtc gtgtcttacc 2100
gggttggact caagacgata gttaccggat aaggcgcagc ggtcgggctg aacggggggt 2160
tcgtgcacac agcccagctt ggagcgaacg acctacaccg aactgagata cctacagcgt 2220
gagctatgag aaagcgccac gcttcccgaa gggagaaagg cggacaggta tccggtaagc 2280
ggcagggtcg gaacaggaga gcgcacgagg gagcttccag ggggaaacgc ctggtatctt 2340
tatagtcctg tcgggtttcg ccacctctga cttgagcgtc gatttttgtg atgctcgtca 2400
ggggggcgga gcctatggaa aaacgccagc aacgcggcct ttttacggtt cctggccttt 2460
tgctggcctt ttgctcacat gttctttcct gcgttatccc ctgattctgt ggataaccgt 2520
attaccgcct ttgagtgagc tgataccgct cgccgcagcc gaacgaccga gcgcagcgag 2580
tcagtgagcg aggaagcgga agagcgcctg atgcggtatt ttctccttac gcatctgtgc 2640
ggtatttcac accgcatata tggtgcactc tcagtacaat ctgctctgat gccgcatagt 2700
taagccagta tacactccgc tatcgctacg tgactgggtc atggctgcgc cccgacaccc 2760
gccaacaccc gctgacgcgc cctgacgggc ttgtctgctc ccggcatccg cttacagaca 2820
agctgtgacc gtctccggga gctgcatgtg tcagaggttt tcaccgtcat caccgaaacg 2880
cgcgaggcag ctgcggtaaa gctcatcagc gtggtcgtga agcgattcac agatgtctgc 2940
ctgttcatcc gcgtccagct cgttgagttt ctccagaagc gttaatgtct ggcttctgat 3000
aaagcgggcc atgttaaggg cggttttttc ctgtttggtc actgatgcct ccgtgtaagg 3060
gggatttctg ttcatggggg taatgatacc gatgaaacga gagaggatgc tcacgatacg 3120
ggttactgat gatgaacatg cccggttact ggaacgttgt gagggtaaac aactggcggt 3180
atggatgcgg cgggaccaga gaaaaatcac tcagggtcaa tgccagcgct tcgttaatac 3240
agatgtaggt gttccacagg gtagccagca gcatcctgcg atgcagatcc ggaacataat 3300
ggtgcagggc gctgacttcc gcgtttccag actttacgaa acacggaaac cgaagaccat 3360
tcatgttgtt gctcaggtcg cagacgtttt gcagcagcag tcgcttcacg ttcgctcgcg 3420

tatcggtgat tcattctgct aaccagtaag gcaaccccgc cagcctagcc gggtcctcaa 3480

cgacaggagc acgatcatgc gcacccgtgg ccaggaccca acgctgcccg agatctcgat 3540

cccgcgaaat taatacgact cactataggg agaccacaac ggtttccctc tagaaataat 3600

tttgtttaac tttaagaagg agatatacat atggctagca tgactggtgg acagcaaatg 3660

ggtcgc 3666

<210> 26
<211> 5997
<212> DNA
<213> artificial

<220>
<223> pGBDU

<400> 26
agtggaatca aggctagaaa gactggaaca gctatttcta ctgatttttc ctcgagaaga 60

ccttgacatg attttgaaaa tggattcttt acaggatata aaagcattgt taacaggatt 120

atttgtacaa gataatgtga ataaagatgc cgtcacagat agattggctt cagtggagac 180

tgatatgcct ctaacattga gacagcatag aataagtgcg acatcatcat cggaagagag 240

tagtaacaaa ggtcaaagac agttgactgt atcgccggaa ttccccgggg gatccatcga 300

tgtcgacctg cagagatcta tgaatcgtag atactgaaaa accccgcaag ttcacttcaa 360

ctgtgcatcg tgcaccatct caatttcttt catttataca tcgttttgcc ttcttttatg 420

taactatact cctctaagtt tcaatcttgg ccatgtaacc tctgatctat agaatttttt 480

aaatgactag aattaatgcc catctttttt ttggacctaa attcttcatg aaaatatatt 540

acgagggctt attcagaagc tttggacttc ttcgccagag gtttggtcaa gtctccaatc 600

aaggttgtcg gcttgtctac cttgccagaa atttacgaaa agatggaaaa gggtcaaatc 660

gttggtagat acgttgttga cacttctaaa taagcgaatt tcttatgatt tatgattttt 720

attattaaat aagttataaa aaaaataagt gtatacaaat tttaaagtga ctcttaggtt 780

ttaaaacgaa aattcttgtt cttgagtaac tctttcctgt aggtcaggtt gctttctcag 840

gtatagcatg aggtcgctct tattgaccac acctctaccg gcatgcagct tttcaattca 900

attcatcatt ttttttttat tctttttttt gatttcggtt tctttgaaat ttttttgatt 960

cggtaatctc cgaacagaag gaagaacgaa ggaaggagca cagacttaga ttggtatata 1020

tacgcatatg tagtgttgaa gaaacatgaa attgcccagt attcttaacc caactgcaca 1080

gaacaaaaac cggaaacgaa gataaatcat gtcgaaagct acatataagg aacgtgctgc 1140

tactcatcct agtcctgttg ctgccaagct atttaatatc atgcacgaaa agcaaacaaa 1200

cttgtgtgct tcattggatg ttcgtaccac caaggaatta ctggagttag ttgaagcatt 1260

aggtcccaaa atttgtttac taaaaacaca tgtggatatc ttgactgatt tttccatgga 1320

gggcacagtt aagccgctaa aggcattatc cgccaagtac aattttttac tcttcgaaga 1380

cagaaaattt gctgacattg gtaatacagt caaattgcag tactctgcgg gtgtatacag 1440

aatagcagaa tgggcagaca ttacgaatgc acacggtgtg gtgggcccag gtattgttag 1500
cggtttgaag caggcggcag aagaagtaac aaaggaacct agaggccttt tgatgttagc 1560
agaattgtca tgcaagggct ccctatctac tggagaatat actaagggta ctgttgacat 1620
tgcgaagagc gacaaagatt ttgttatcgg ctttattgct caaagagaca tgggtggaag 1680
agatgaaggt tacgattggt tgattatgac acccggtgtg ggtttagatg acaagggaga 1740
cgcattgggt caacagtata gaaccgtgga tgatgtggtc tctacaggat ctgacattat 1800
tattgttgga agaggactat ttgcaaaggg aagggatgct aaggtagagg gtgaacgtta 1860
cagaaaagca ggctgggaag catatttgag aagatgcggc cagcaaaact aaaaaactgt 1920
attataagta aatgcatgta tactaaactc acaaattaga gcttcaattt aattatatca 1980
gttattaccc ctggattaat gaatcggcca acgcgcgggg agaggcggtt tgcgtattgg 2040
gcgctcttcc gcttcctcgc tcactgactc gctgcgctcg gtcgttcggc tgcggcgagc 2100
ggtatcagct cactcaaagg cggtaatacg gttatccaca gaatcagggg ataacgcagg 2160
aaagaacatg tgagcaaaag gccagcaaaa ggccaggaac cgtaaaaagg ccgcgttgct 2220
ggcgtttttc cataggctcc gcccccctga cgagcatcac aaaaatcgac gctcaagtca 2280
gaggtggcga aacccgacag gactataaag ataccaggcg tttccccctg gaagctccct 2340
cgtgcgctct cctgttccga ccctgccgct taccggatac ctgtccgcct ttctcccttc 2400
gggaagcgtg gcgctttctc atagctcacg ctgtaggtat ctcagttcgg tgtaggtcgt 2460
tcgctccaag ctgggctgtg tgcacgaacc ccccgttcag cccgaccgct gcgccttatc 2520
cggtaactat cgtcttgagt ccaacccggt aagacacgac ttatcgccac tggcagcagc 2580
cactggtaac aggattagca gagcgaggta tgtaggcggt gctacagagt tcttgaagtg 2640
gtggcctaac tacggctaca ctagaaggac agtatttggt atctgcgctc tgctgaagcc 2700
agttaccttc ggaaaaagag ttggtagctc ttgatccggc aaacaaacca ccgctggtag 2760
cggtggtttt tttgtttgca agcagcagat tacgcgcaga aaaaaaggat ctcaagaaga 2820
tcctttgatc ttttctacgg ggtctgacgc tcagtggaac gaaaactcac gttaagggat 2880
tttggtcatg agattatcaa aaaggatctt cacctagatc cttttaaatt aaaaatgaag 2940
ttttaaatca atctaaagta tatatgagta aacttggtct gacagttacc aatgcttaat 3000
cagtgaggca cctatctcag cgatctgtct atttcgttca tccatagttg cctgactccc 3060
cgtcgtgtag ataactacga tacgggaggg cttaccatct ggccccagtg ctgcaatgat 3120
accgcgagac ccacgctcac cggctccaga tttatcagca ataaaccagc cagccggaag 3180
ggccgagcgc agaagtggtc ctgcaacttt atccgcctcc atccagtcta ttaattgttg 3240
ccgggaagct agagtaagta gttcgccagt taatagtttg cgcaacgttg ttgccattgc 3300
tacaggcatc gtggtgtcac gctcgtcgtt tggtatggct tcattcagct ccggttccca 3360
acgatcaagg cgagttacat gatcccccat gttgtgcaaa aaagcggtta gctccttcgg 3420
tcctccgatc gttgtcagaa gtaagttggc cgcagtgtta tcactcatgg ttatggcagc 3480

actgcataat tctcttactg tcatgccatc cgtaagatgc ttttctgtga ctggtgagta 3540
ctcaaccaag tcattctgag aatagtgtat gcggcgaccg agttgctctt gcccggcgtc 3600
aatacgggat aataccgcgc cacatagcag aactttaaaa gtgctcatca ttggaaaacg 3660
ttcttcgggg cgaaaactct caaggatctt accgctgttg agatccagtt cgatgtaacc 3720
cactcgtgca cccaactgat cttcagcatc ttttactttc accagcgttt ctgggtgagc 3780
aaaaacagga aggcaaaatg ccgcaaaaaa gggaataagg gcgacacgga aatgttgaat 3840
actcatactc ttcctttttc aatattattg aagcatttat cagggttatt gtctcatgag 3900
cggatacata tttgaatgta tttagaaaaa taaacaaata ggggttccgc gcacatttcc 3960
ccgaaaagtg ccacctgacg tctaagaaac cattattatc atgacattaa cctataaaaa 4020
taggcgtatc acgaggccct ttcgtctcgc gcgtttcggt gatgacggtg aaaacctctg 4080
acacatgcag ctcccggaga cggtcacagc ttgtctgtaa gcggatgccg ggagcagaca 4140
agcccgtcag ggcgcgtcag cgggtgttgg cgggtgtcgg ggctggctta actatgcggc 4200
atcagagcag attgtactga gagtgcacca taacgcattt aagcataaac acgcactatg 4260
ccgttcttct catgtatata tatatacagg caacacgcag atataggtgc gacgtgaaca 4320
gtgagctgta tgtgcgcagc tcgcgttgca ttttcggaag cgctcgtttt cggaaacgct 4380
ttgaagttcc tattccgaag ttcctattct ctagctagaa agtataggaa cttcagagcg 4440
cttttgaaaa ccaaaagcgc tctgaagacg cactttcaaa aaaccaaaaa cgcaccggac 4500
tgtaacgagc tactaaaata ttgcgaatac cgcttccaca aacattgctc aaaagtatct 4560
ctttgctata tatctctgtg ctatatccct atataaccta cccatccacc tttcgctcct 4620
tgaacttgca tctaaactcg acctctacat tttttatgtt tatctctagt attactcttt 4680
agacaaaaaa attgtagtaa gaactattca tagagtgaat cgaaaacaat acgaaaatgt 4740
aaacatttcc tatacgtagt atatagagac aaaatagaag aaaccgttca taattttctg 4800
accaatgaag aatcatcaac gctatcactt tctgttcaca aagtatgcgc aatccacatc 4860
ggtatagaat ataatcgggg atgcctttat cttgaaaaaa tgcacccgca gcttcgctag 4920
taatcagtaa acgcgggaag tggagtcagg ctttttttat ggaagagaaa atagacacca 4980
aagtagcctt cttctaacct taacggacct acagtgcaaa aagttatcaa gagactgcat 5040
tatagagcgc acaaaggaga aaaaaagtaa tctaagatgc tttgttagaa aaatagcgct 5100
ctcgggatgc atttttgtag aacaaaaaag aagtatagat tctttgttgg taaaatagcg 5160
ctctcgcgtt gcatttctgt tctgtaaaaa tgcagctcag attctttgtt tgaaaaatta 5220
gcgctctcgc gttgcatttt tgttttacaa aaatgaagca cagattcttc gttggtaaaa 5280
tagcgctttc gcgttgcatt tctgttctgt aaaaatgcag ctcagattct ttgtttgaaa 5340
aattagcgct ctcgcgttgc atttttgttc tacaaaatga agcacagatg cttcgttgct 5400
tgcatgcaac ttcttttctt tttttttctt ttctctctcc cccgttgttg tctcaccata 5460
tccgcaatga caaaaaaaat gatggaagac actaaaggaa aaaattaacg acaaagacag 5520

caccaacaga tgtcgttgtt ccagagctga tgaggggtat cttcgaacac acgaaacttt 5580
ttccttcctt cattcacgca cactactctc taatgagcaa cggtatacgg ccttccttcc 5640
agttacttga atttgaaata aaaaaagttt gccgctttgc tatcaagtat aaatagacct 5700
gcaattatta atcttttgtt tcctcgtcat tgttctcgtt ccctttcttc cttgtttctt 5760
tttctgcaca atatttcaag ctataccaag catacaatca actccaagct tgaagcaagc 5820
ctcctgaaag atgaagctac tgtcttctat cgaacaagca tgcgatattt gccgacttaa 5880
aaagctcaag tgctccaaag aaaaaccgaa gtgcgccaag tgtctgaaga acaactggga 5940
gtgtcgctac tctcccaaaa ccaaaaggtc tccgctgact agggcacatc tgacaga 5997

## Description

The present invention is based on the observation that reduced activity of a protein involved in the regulation of energy homeostasis results in body fat reduction. Thus, the present invention relates to a pharmaceutical composition comprising a compound (a) reducing or inhibiting, or a compound (b) increasing the biological activity of the protein or its expression. The present invention also relates to the use of said compounds for the prevention/treatment of diseases wherein decrease or increase of the activity of the protein has a therapeutic effect, e.g. obesity, adiposity diabetes, dyslipidemia, or an anorectic disease, anorexia or emaciation. Finally, the present invention also relates to methods for the diagnosis of diseases associated with an aberrant activity of the protein.

Obesity and diabetes are highly prevalent diseases caused by the aberrant regulation of carbohydrate and lipid metabolism. Although the biochemical pathways of carbohydrate and lipid metabolism are well known, much remains to be learned about the molecular mechanisms and signals controlling flux through these pathways, and thus homeostasis. Humoral signaling molecules such as insulin and leptin are important in both carbohydrate and lipid metabolic control. How these signals link to the metabolic genes controlling the relevant biochemical pathways is a topic of current interest (Friedman, Nutr.Rev.60 (2000), pp 1-14, discussion pp 68-84, 85-17.)

Components of the insulin signal transduction pathway have been extensively studied in the context of tissue growth control during development. Mutations in the core components of the pathway - the insulin receptor (InR), phosphoinositide-3-kinase (PI3K), PDK1, Akt/PKB, Tuberous sclerosis complex (Tsc1/Tsc2), Rheb, the target of Rapamycin (TOR), and ribosomal protein S6 Kinase (S6K) - all cause tissue growth abnormalities or lethality. Stimulation of the insulin receptor activates PI3K, which increases the level of phosphoinositol (3, 4, 5) triphosphate (PIP3) at the plasma membrane. PIP3 binding recruits the protein kinase Akt to the membrane, where it is activated through phosphorylation by PDK1. The connection between Akt and TOR is less well understood, but TOR is genetically downstream of Akt and is activated. TOR is a protein kinase that regulates the translational machinery of the cell via two well-characterized effectors: S6K and 4E-binding protein (4E-BP). S6K controls ribosome biogenesis and thus the biosynthetic capacity of the cell. 4E-BP binds to the translation factor eIF4E and prevents assembly of a protein complex that facilitates recruitment of the ribosome. TOR phosphorylates 4E-BP and prevents its binding to eIF4E, promoting cap dependent translation.

In addition to insulin-response, this signaling cassette also integrates information on cellular nutritional status. TOR receives information on the cellular energy levels from the heterodimeric Tsc1/2 complex. Tsc2 serves as a GTPase activating protein that inactivates Rheb, and thereby reduces TOR activity. Tsc1/2 mutations result in TOR hyperactivation and tissue overgrowth. Mutants in mouse Tsc1 or Tsc2 have benign overgrowths called harmatomas and exhibit an increased susceptibility to tumor formation. Cellular. AMP levels are sensed by AMP-activated protein kinase (AMPK) which phosphorylates and activates Tsc2 to inhibit TOR. TOR activity is also regulated by oxygen, through the hypoxia-induced transcription factor, HIF. HIF controls expression of REDD1/Scylla/Charybdis, which reduce Tsc1/2 activity.

Considerable evidence has accumulated to indicate that the Insulin/TOR pathway controls fat metabolism as well as tissue growth. Flies and mice with reduced InR/IGF receptor and PI3K activity are small and have elevated fat levels. Mice mutant for Akt2 become insulin resistant and develop lipoatrophy as they age; mice mutants for Akt1 have normal glucose metabolism but are growth defective. Human mutants for Akt2 also have abnormal metabolism and are 35% slimmer than average. One output-branch of the core pathway is via transcription factors of the winged helix-Forkhead family. In response to insulin, Akt phosphorylates FOXO proteins, which promotes their interaction with 14-3-3 proteins and leads to cytoplasmic retention and inactivation. Foxo transcription factors have been implicated in the control of fat metabolism and lifespan in *C. elegans,* flies and mice. In addition, elements of the TOR branch of the pathway are beginning to be implicated in fat metabolism. TOR phosphorylates and regulates S6K and 4E-BP. S6K mutant mice are resistant to diet induced obesity. 4E-BP1 mutant mice, have a defect in fat metabolism. Recent studies have also identified the eIF4E kinase LK6 as a modulator of growth and fat metabolism. However, although there is now much known about the biochemical pathways of carbohydrate and lipid metabolism, effective therapies for diseases associated with an aberrant regulation of these pathways, e.g., obesity and diabetes, are hardly available.

Thus, the technical problem underlying the present invention is to provide means suitable for treating or preventing diseases being based on aberrant regulation of carbohydrate and/or lipid metabolism.

The solution of this technical problem is achieved by providing the embodiments characterized in the claims.

During the experiments resulting in the present invention, a novel modulator of the Insulin/TOR pathway, *"melted",* was identified. Flies mutant for *melted* show pronounced defects in fat metabolism. The *melted* gene encodes a PH domain protein that interacts with both Tsc1 and FOXO. Melted protein can recruit the Tsc1/2 complex to the cell membrane and thereby modulate its output via the TOR pathway. Melted protein can also recruit FOXO to the membrane in an insulin-regulated manner and thereby influence expression of Foxo target genes. Thus, *melted* mutants cause reduced TOR activity and increased FOXO activity. In so doing, reduced Melted activity results in physiological changes that mimic what happens when a normal animal is placed under conditions of nutrient deprivation. Thus, a method to reduce Melted activity would be useful to treat obesity and obesity-related diseases in humans and animals.

It has been found, that Melted contains two functional domains, identified as regions of high conservation between the fly and human proteins - an N-terminal protein interaction domain and a C-terminal PH domain. The PH domain targets Melted to the cell membrane. Overexpressed PH-GFP fusions show sharp membrane localization, and subcellular fractionation assays showed that in steady state the majority of endogenous Melted protein is membrane associated. Rescue assays show that, unlike the full-length protein, Melted protein missing the PH domain cannot rescue the *melt^{Δ1}* mutant phenotypes. Thus, Melted protein requires its PH domain, and presumably its membrane localization, to have biological activity in vivo.

Melted functions as an adaptor, facilitating association of the TSC complex and FOXO with their upstream signaling inputs at the membrane. Both FOXO and Tsc2 are phosphorylated *in vivo* by PKB, which becomes activated at the cell membrane when it binds PIP3 and is phosphorylated by PDK1. Although the phosphorylation of Tsc2 by PKB is not strictly necessary for viability it is possible that this phosphorylation is modulatory in function. Tsc2 is also regulated via phosphorylation by AMPK. AMPK is membrane associated through its myristoylated beta-subunit. Therefore, recruiting the Tsc complex and FOXO to the cell membrane may alter their state of activation.

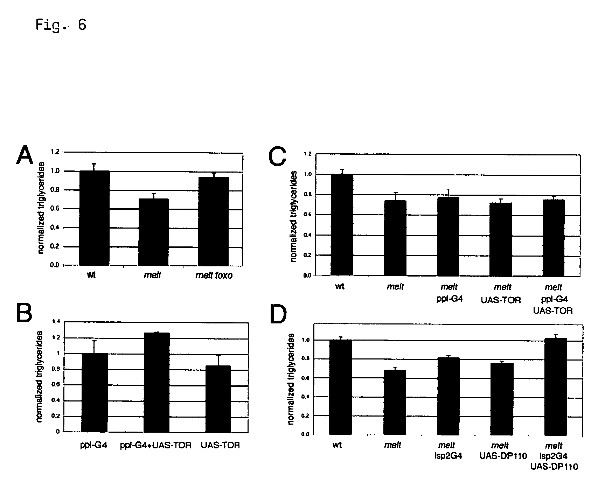

The activity of signaling pathways downstream of Tsc1/2 and FOXO are altered in the *melted* mutant. *Melted* mutants cause reduced TOR activity, observed by measuring the level of 4E-BP phosphorylation in adipose tissue. TOR activity is not normally limiting for 4E-BP phosphorylation. Increased 4E-BP level leads to increased phosphorylated 4E-BP in normal flies. In *melted* mutants, total 4E-BP protein increased, but the level of 4E-BP phosphorylation did not, indicating reduced relative TOR activity in the *melted* mutant. TOR activity positively regulates fat accumulation in adipose tissue (c.f. Figure 6). Therefore, the reduced TOR activity in the *melted* mutant adipose tissue contributes to the mutant leanness.

FOXO activity is increased in *melted* mutants as indicated by the increased level of 4E-BP transcription (4E-BPtranscription is a well characterized FOXO target (Junger et al., J.Biol. 2 (2003), 20; Puig et al., Genes Dev. 17 (2003), 2006-20). 4EBP transcription is upregulated in the *melted* mutant, and this upregulation is FOXO dependent, suggesting that FOXO activity is increased in the *melted* mutant. This was confirmed by showing that decreasing FOXO activity by removing the foxo gene in a *melted* mutant decreases the severity of the *melted* mutant fat defect. Thus, FOXO is an important effector of *melted* in regulation of fat metabolism.

*Melt*^{*Δ*1} mutant animals are lean due to reduced lipid accumulation in adipose tissue, and this phenotype is autonomous to the adipose tissue since it can be rescued by adipose-tissue specific expression of *melted.* Since the leanness phenotype is adipose-tissue autonomous, the transcriptional changes that occur in the adipose tissue upon loss of Melted function were examined. Adipose tissue undergoes a dramatic transcriptional change upon loss of Melted function. At the 99% confidence level, 720 genes had altered expression levels in the fat body, and 249 were misregulated by more than 1.5-fold. 63% of the misregulated genes for which a function is annotated are metabolism related. Although many of these are involved in lipid or carbohydrate metabolism, it was surprising to find a significant number of genes involved in proteolysis and amino acid metabolism. Recently, FOXO has been shown to promote protein turnover during fasting suggesting a possible link between those genes and FOXO.

**Table 1**

| **Misregulated lipid metabolism genes in *melt*^{Δ1} fat body** | | | |
|---|---|---|---|
| Up-regulated | | | |
| **Gene** | **Function** | **fold Δ** | **RT-PCR** |
| **CG4757** | carboxylesterase/lipase | 8.5 | >5000 |
| **Cyp6d5** | cytochrome P450 | 5.7 | 28.7 |
| **Cyp12d1-d** | cytochrome P450 | 5.3 | 7.4 |
| CG4842 | alcohol dehydrogenase | 2.5 | |
| **CG5171** | trehalose-phosphatase | 2.4 | 11.8 |
| CG10512 | Malate/L-lactate dehydrogenase | 2.4 | |
| α-Est8 | lipase/esterase | 2.0 | |
| Adh | alcohol dehydrogenase | 2.0 | |
| CG3940 | carbonic anhydrase | 1.9 | |
| Csat | galactose transport | 1.8 | |
| CG6022 | holocytochrome-c synthase | 1.8 | |
| Glaz | lipid transport | 1.7 | |
| CG10962 | short-chain dehyrogenase | 1.6 | |
| Cyp28d1 | cytochrome P450 | 1.5 | |
| CG8690 | alpha-glucosidase | 1.5 | |
| CG4670 | glucosidase activity | 1.5 | |

| Down-regulated | | | |
|---|---|---|---|
| **Gene** | **Function** | **fold** Δ | **RT-PCR** |
| Cyp4p1 | cytochrome P450 | 15.0 | |
| Cyp4p3 | cytochrome P450 | 10.6 | |
| CG12224 | aldo/keto reductase | 6.1 | |
| Cyp4d8 | cytochrome P450 | 2.8 | |
| Cyp9b2 | cytochrome p450 | 2.5 | |
| Sug | transcription factor, sugar dependent | 2.4 | |
| CG30148 | glucosidase | 2.2 | |
| CG3488 | Lipase | 2.2 | |
| CG8323 | mitochondrial transport | 2.1 | |
| CG15828 | lipid transport | 2.0 | |
| CG5288 | galactokinase | 2.0 | |
| CG5295 | adipose triglyceride lipase homolog | 2.0 | |
| yip2 | acetyl-CoA C-acyltransferase | 2.0 | |
| Cyp6a17 | cytochrome P450 | 1.9 | |
| CG11963 | succinate-CoA ligase | 1.8 | |
| **CG13890** | dodecenoyl-CoA delta-isomerase | 1.8 | 1.9 |
| **AcCoAS** | acetyl-CoA synthase | 1.8 | 1.9 |
| **Acox57D-d** | acyl-CoA oxidase/dehyrogenase | 1.8 | 2.9 |
| Nes | O-acyl transferase | 1.8 | |
| CG2065 | short-chain dehydrogenase | 1.7 | |
| CG1041 | carnitine O-acetyltransferase | 1.7 | |
| CG6178 | fatty acid ligase | 1.7 | |
| Gyk | glycerol kinase | 1.6 | |
| Pgm | phosphoglucomutase | 1.6 | |
| CG17292 | triacylglyerollipase | 1.6 | |
| CG2767 | alcohol dehydrogenase | 1.6 | |
| CG30069 | short-chain dehydrogenase | 1.6 | |
| Cyp9f2 | cytochrome P450 | 1.6 | |
| Fbp1 | fat body storage protein uptake | 1.5 | |
| Cyp6d2 | cytochrome P450 | 1.5 | |
| PEPCK | gluconeogenesis | 1.5 | 1.2 |

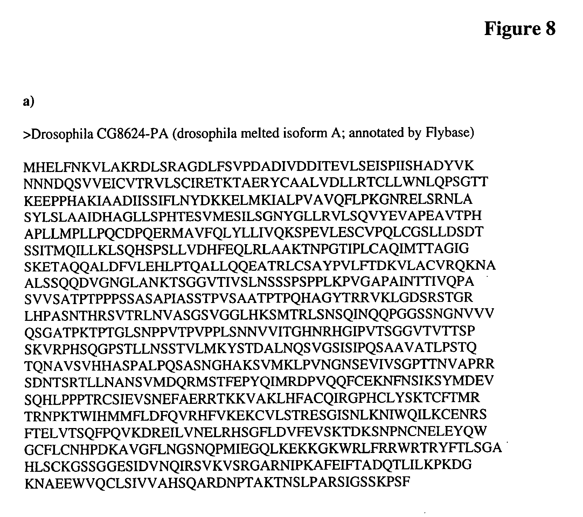

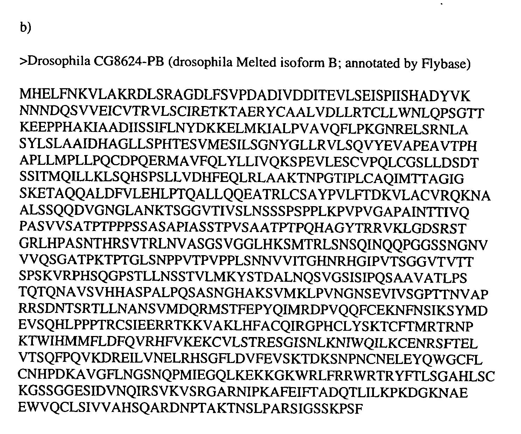

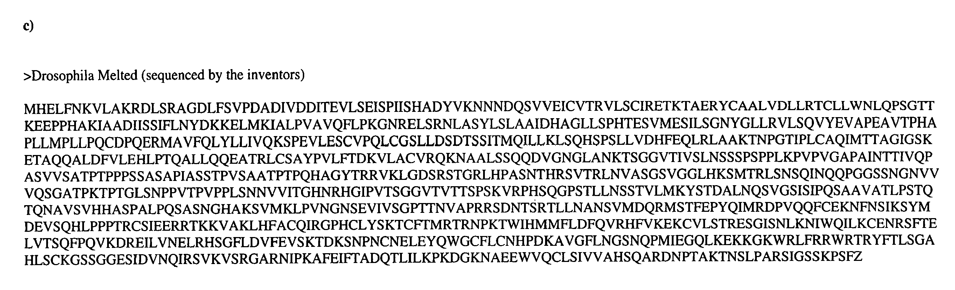

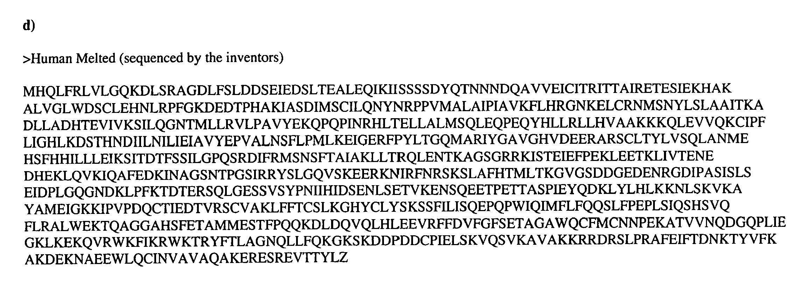

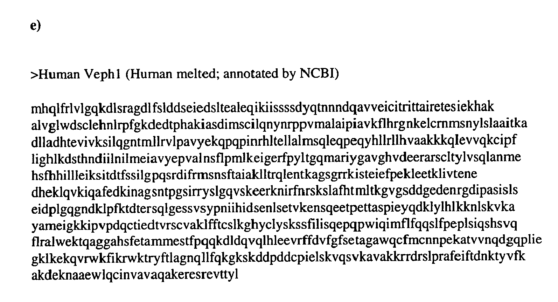

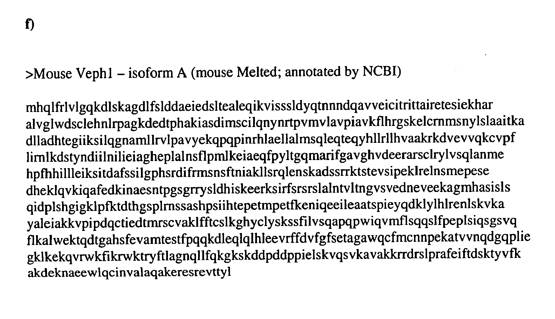

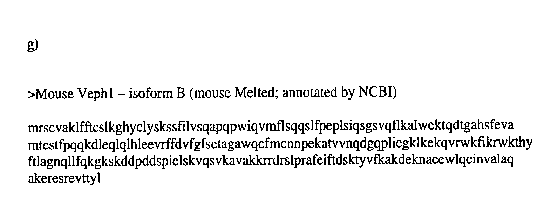

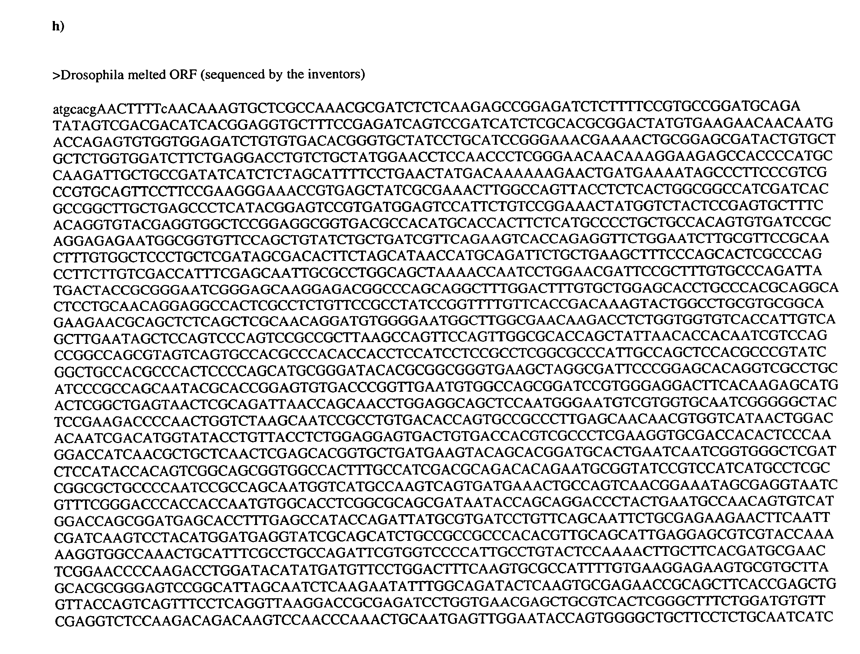

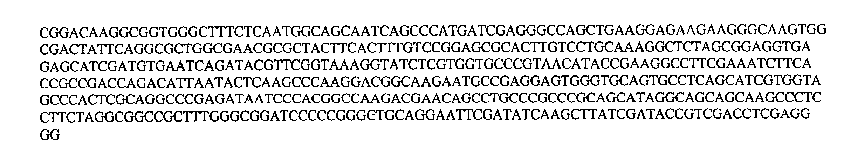

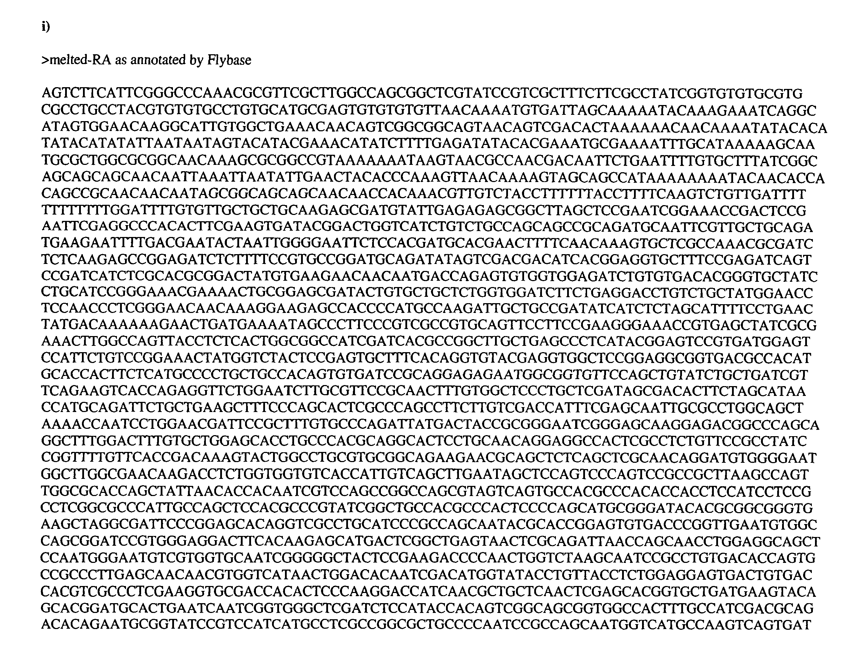

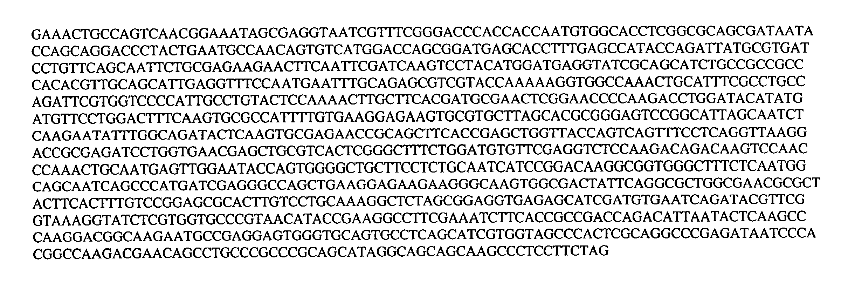

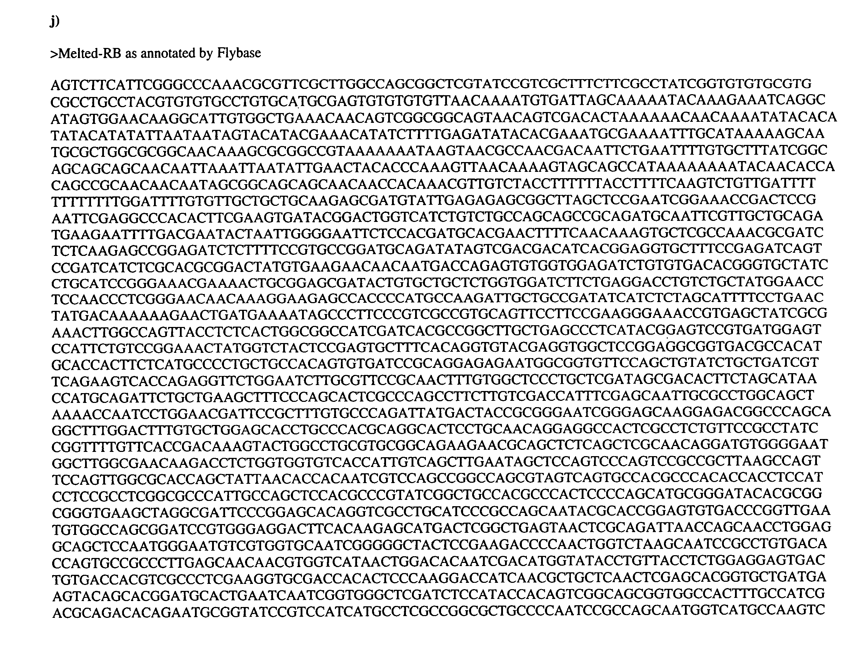

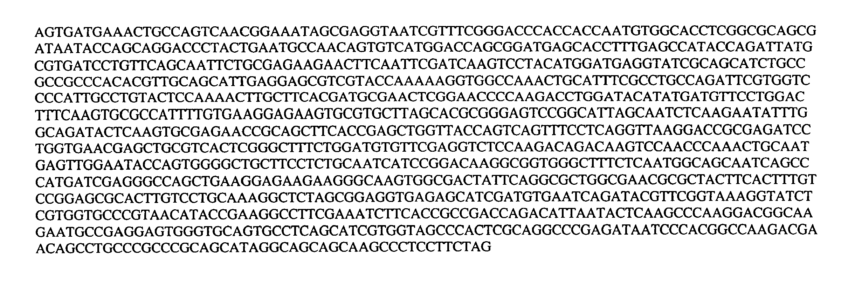

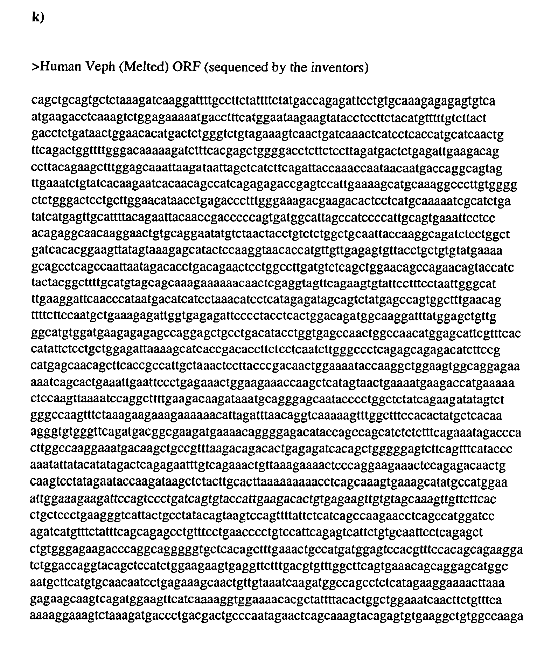

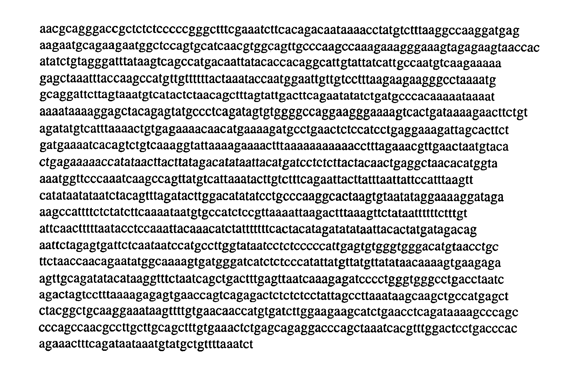

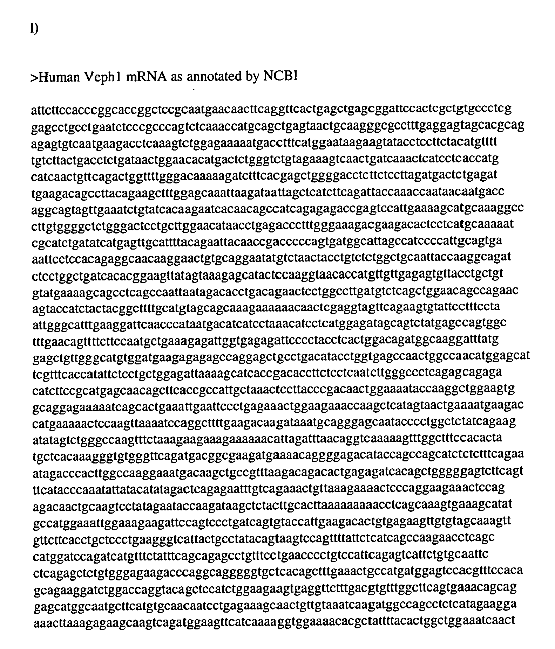

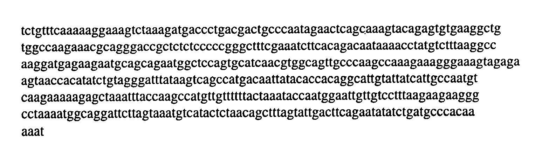

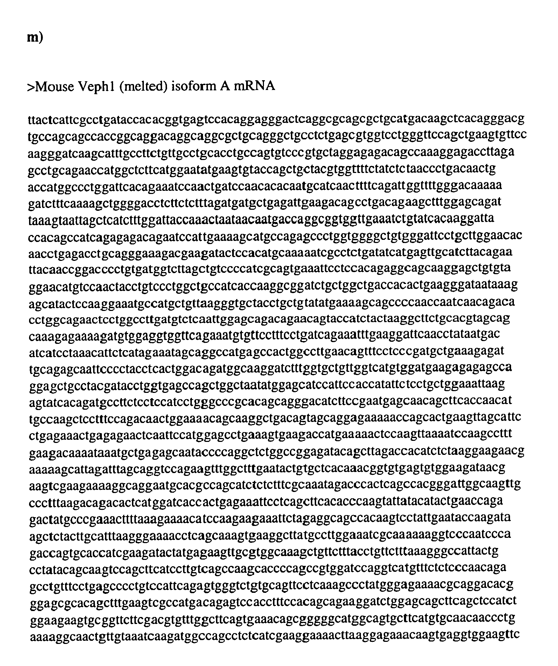

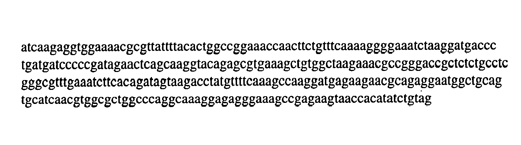

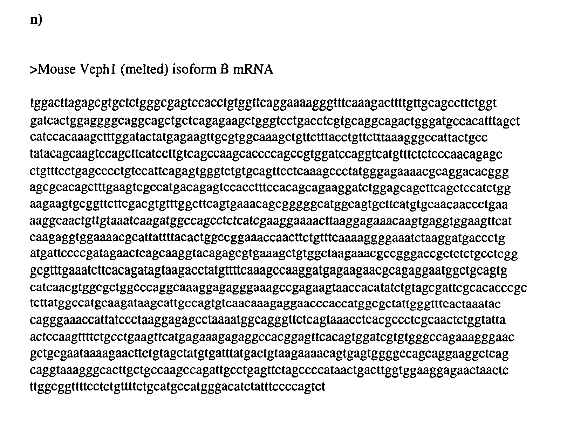

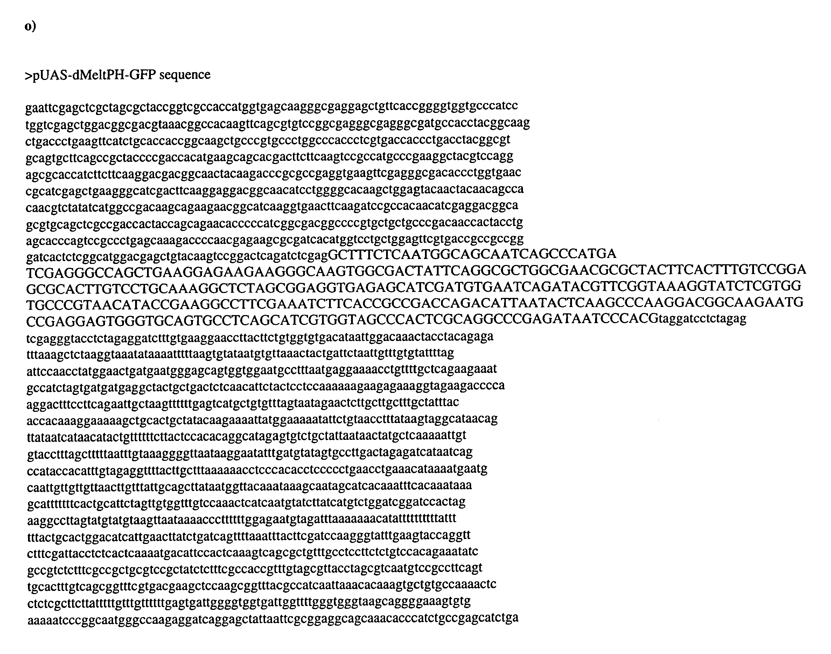

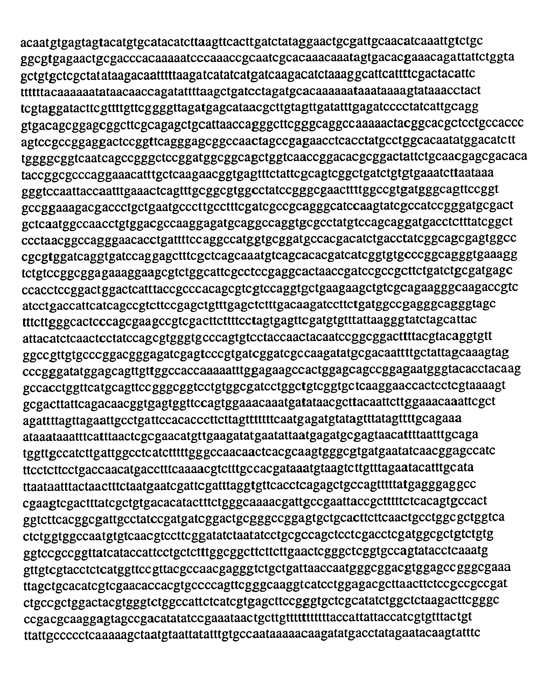

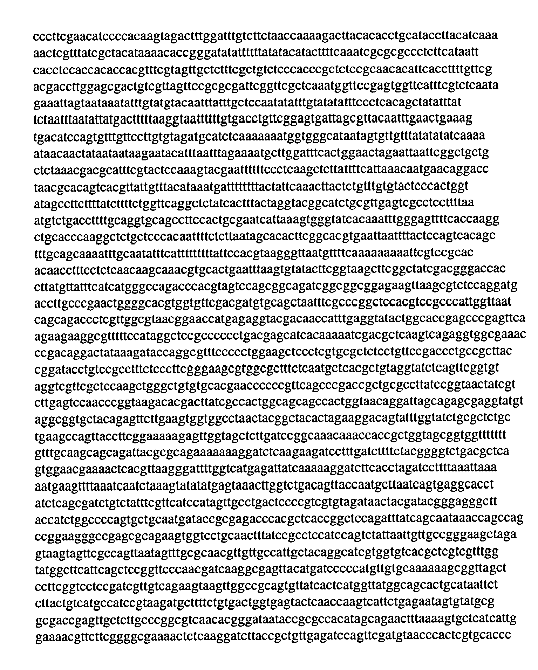

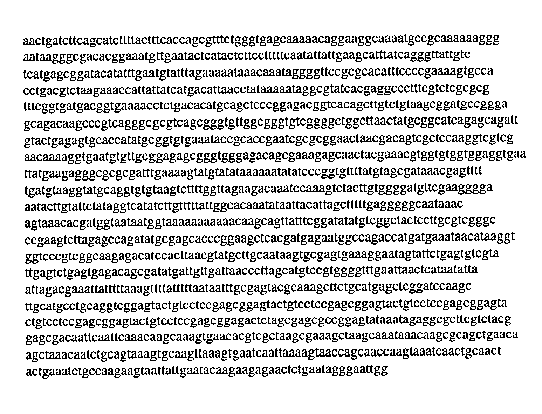

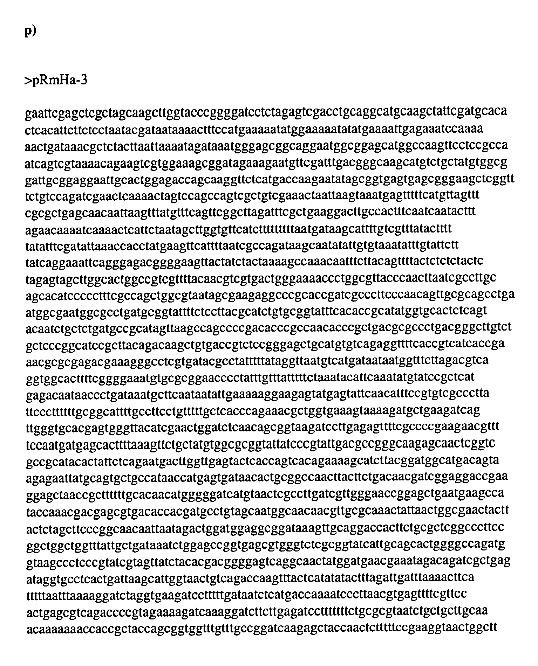

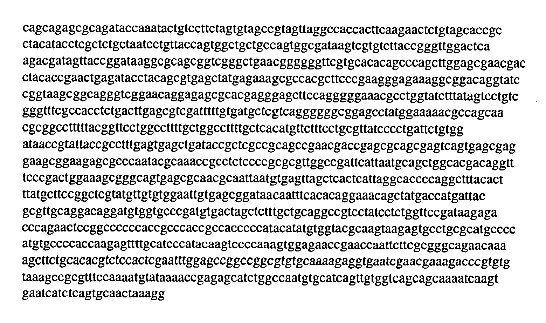

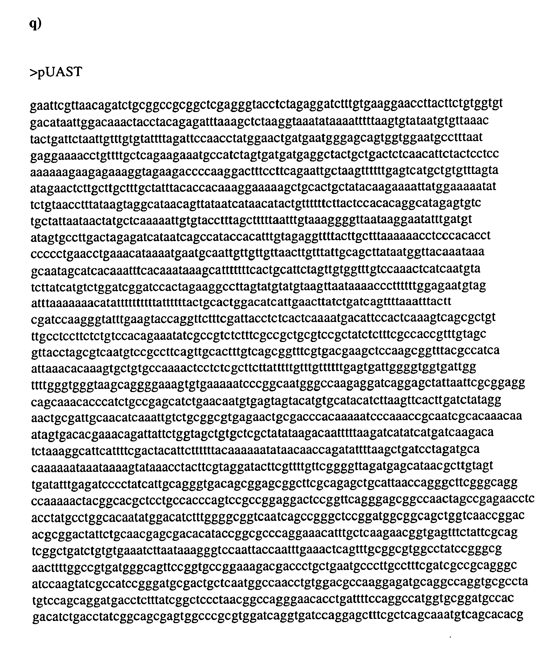

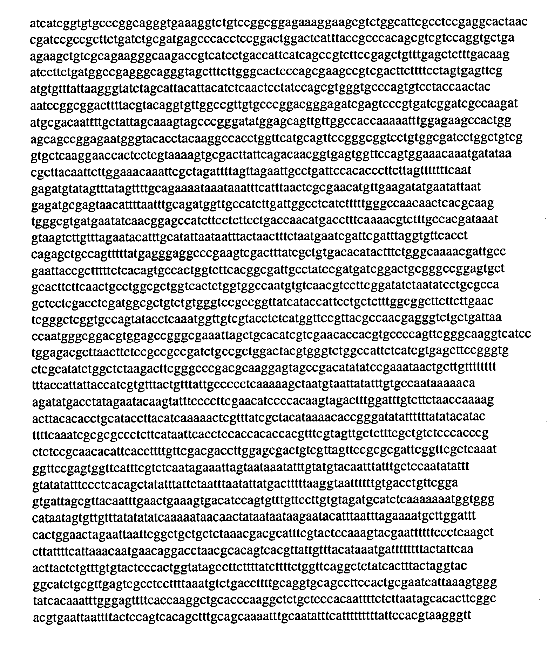

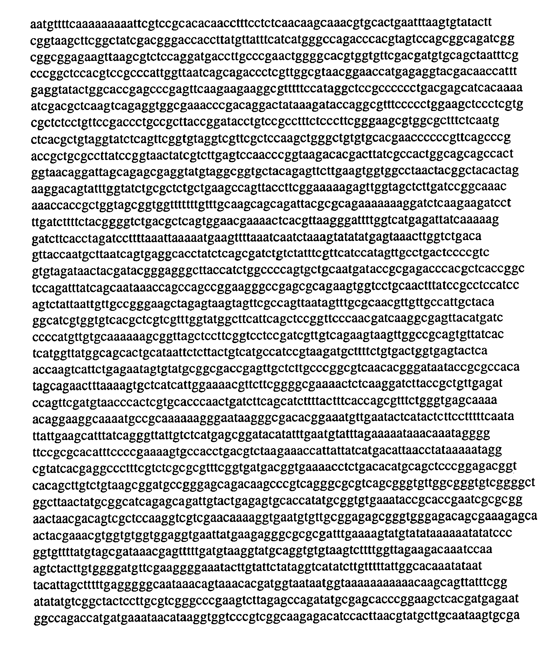

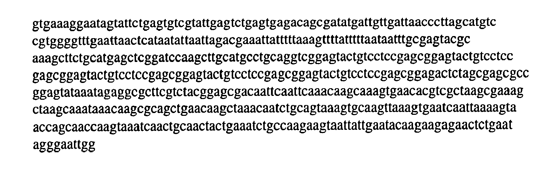

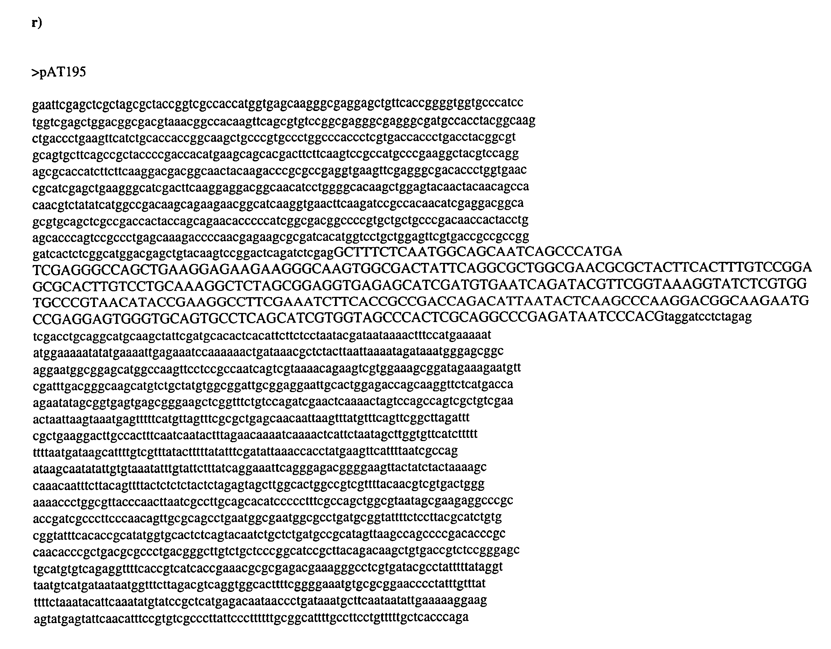

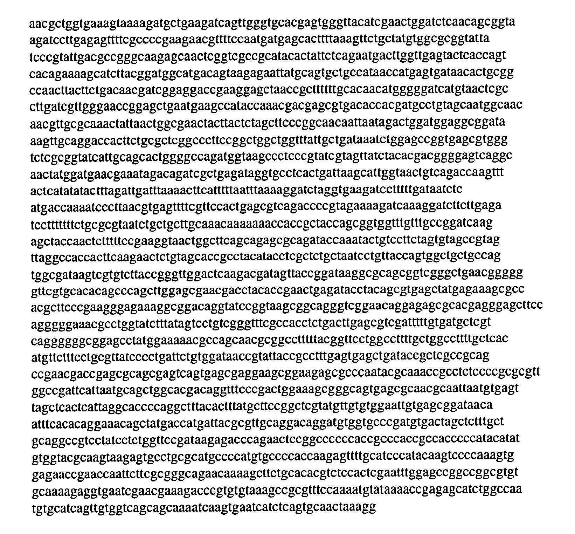

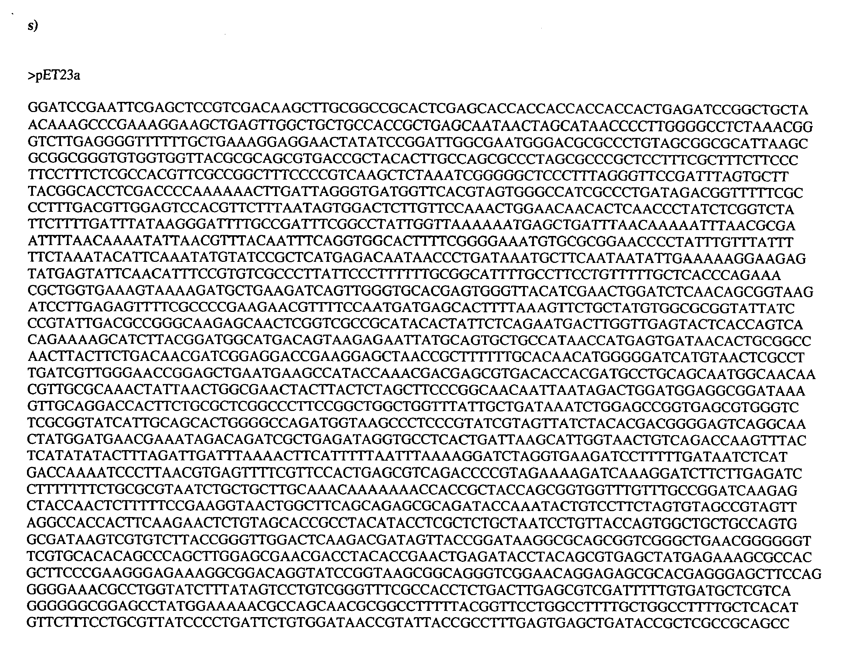

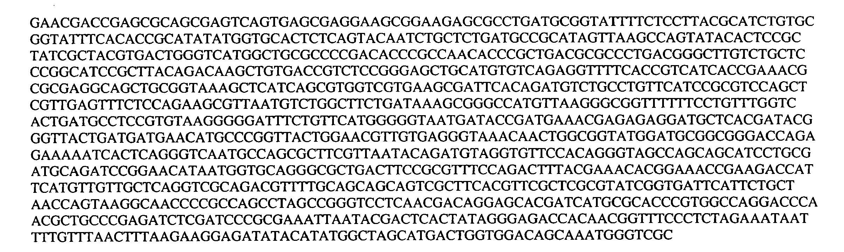

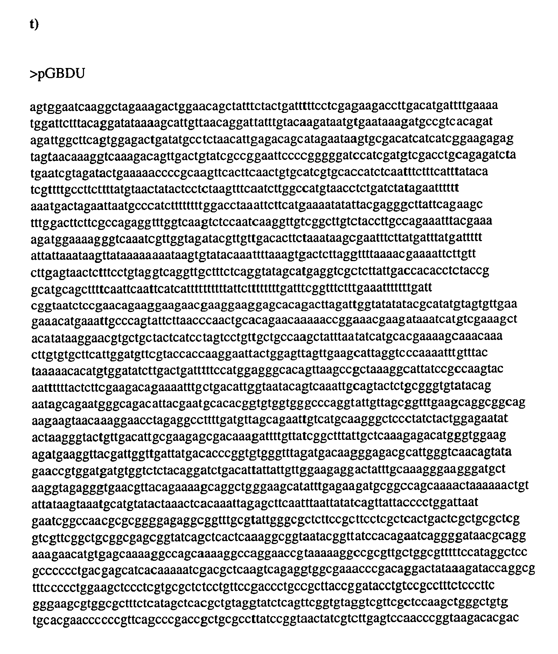

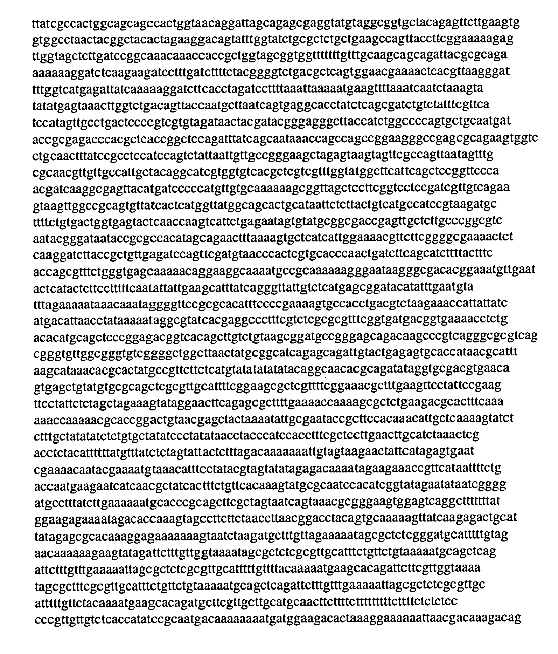

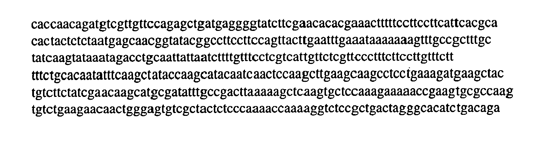

Human Melted protein is highly homologous to the fly protein, and can rescue the *melted* mutant fly. This can be seen from the various sequences contained in Fig. 8. It can be expected that the organismal function of fat metabolism regulation is also conserved in mammals and that blocking *melted* activity in mammals has similar effects as in the fly - reduced triglyceride accumulation by adipose tissue. The changes in the physiology of *melted* mutant flies resemble the effects of nutrient deprivation in normal animals. Since *melted* is highly conserved between flies and humans, and because of its pronounced effects on fat metabolism, modification of its activity provides a basis for therapy. Thus, methods to reduce Melted activity are expected to be useful in treating obesity and obesity-related diseases including diabetes.

### Brief description of the drawings

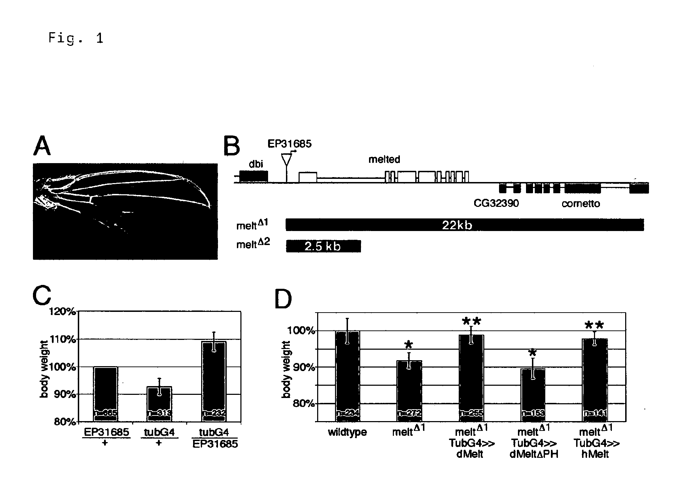

Figure 1: The *melted* gene product regulates tissue growth
   (A) Overexpression of *melted* in the posterior compartment (red wing; *engrailed-gal*4*,* EP31685). A normal wing is shown in green for comparison.
      Note that the anterior compartments are the same size (overlap) but the posterior (lower) is larger in the red wing.
   (B) Schematic representation of the *melted* locus. *melt^{Δ1}* is a 22 kb deletion, including the adjacent genes CG32390 and *cornetto. melt^{Δ2}* deleted 2.5 kb of DNA, including exon 1. The first exon of the predicted gene is non-coding, with the ATG for the open reading frame located in exon 2. RT-PCR of exons 5-7 showed that an alternate form of *melt* transcript was produced in the homozygous *melt^{Δ2}* deletion flies, suggesting that it is not a null allele.
   (C) Average body weight of 3-day old wild-type male flies, tubulin-GAL4 flies and flies overexpressing EP31685*.* n=number of flies measured. Error bars: standard deviation.
   (D) Average body weight of 3-day old wild-type male flies, *melt^{Δ1}* homozygous deletion mutant flies and *melt^{Δ1}* homozygous mutants expressing full-length Drosophila Melted (dmelt), dMelt lacking the PH domain (dMeltΔPH), or the full length Human Melted (hMelt). In addition, eggs laid by *melt^{Δ1}* homozygous females were 13% smaller than control eggs (t-test = 10-7; rescued by expression of *UAS-melt*).
   * indicates statistically significant difference from wildtype.
   ** indicates statistically significant difference from *melt^{Δ1}*

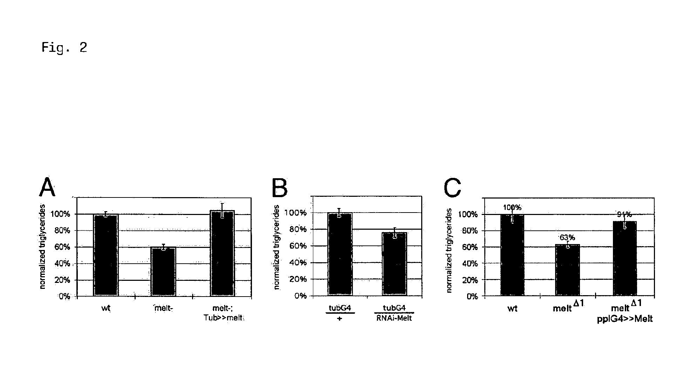

Figure 2: Reduced fat reserves in melted mutants
   (A) Total body fat (normalized to total protein) in wild-type, *melt^{Δ1}* and *melt^{Δ1}* male flies rescued by expression of full length Melted. Error bars: standard deviation (3 replicates).
   (B) Total body fat in male flies expressing UAS-melted-RNAi.
   (C) Normalized total body fat of adult males of indicated genotypes.

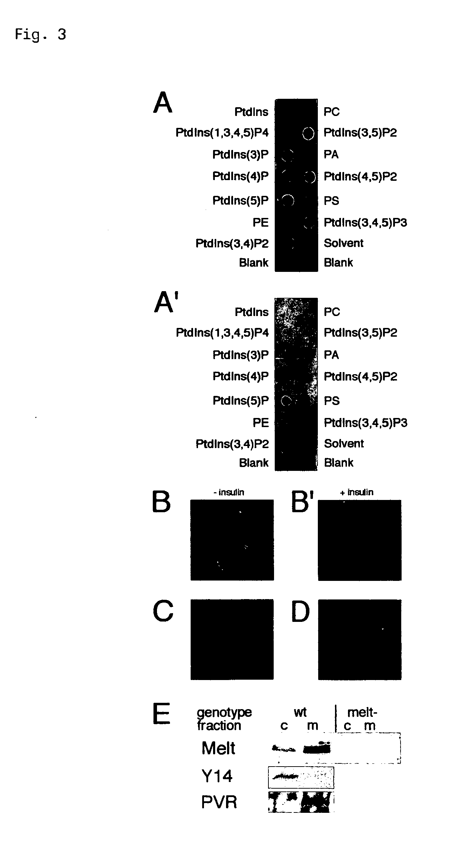

Figure 3: Membrane localization of Melted, a PIP binding protein
   (A, A') Immunoblots showing binding of bacterially expressed Melted protein to immobilized phosphoinositides (PIP Strip, Echelon Biosciences) at Melted protein concentrations of roughly 100 ng/mL (A) or 10ng/mL (A').
      PtdIns=phosphatidylinositol; PE=phosphatidylethanolamine;
      PC=phosphatidylcholine; PS=phosphatidylserine; PA=phosphatidic acid. The binding pattern for a control using the PIP3 binding domain of GRP1 was different.
   (B) Immunofluorescence showing membrane localization of Melted-GFP in serum starved (-insulin) and insulin-treated S2 cells.
   (C) Immunofluorescence showing membrane localization of MeltedPH-GFP in wing imaginal disc cells.
   (D) Immunofluorescence showing cytoplasmic localization of Melted-GFP lacking the PH domain (MeltΔPH-GFP) in wing imaginal disc cells.
   (E) Immunoblot showing the distribution of Melted protein in cytosolic (c) and membrane (m) fractions of wild-type and *melt^{Δ2}* homozygous mutant tissue. Controls are anti-Y14, a cytoplasmic protein (Hachet and Ephrussi, Curr Biol. 11 (2001), 1666-74) and anti-PVR (PDGF/VEGF receptor a transmembrane protein (Duchek et al., Cell 107 (2001), 17-26).

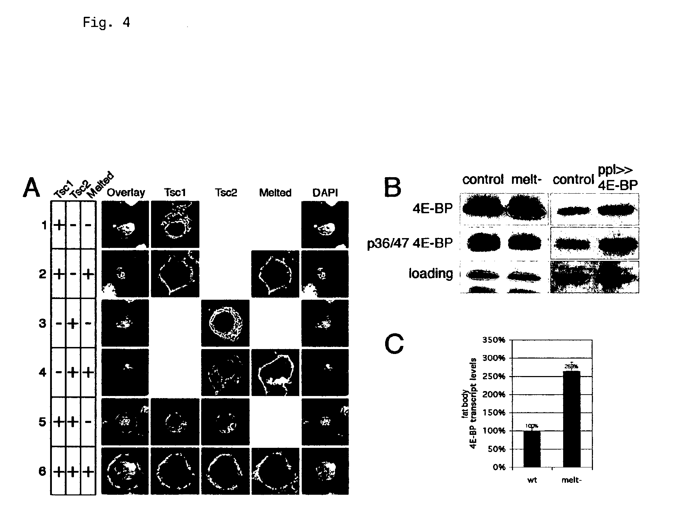

Figure 4: Melted binds Tsc1/2 and regulates TOR activity
   (A) Immunofluorescence showing S2 cells transfected to express Tsc1, Tsc2 and Melted, as indicated. Tsc1 was epitope tagged with myc (green), Tsc2 was tagged with V5 (red). Melted was detected with anti-Melted antibody (Blue). Nuclei were visualized with DAPI (white).
   (B) Left: Immunoblot of fat-body tissue from wild-type and *melt* mutant larvae. Right: Immunoblot of fat-body tissue from wild-type and larvae expressing 4E-BP under ppl-Gal4 control.
      Upper panels: probed with anti-4E-BP to visualize total 4E-BP protein.
      Middle panels: probed with phosphospecific antibody to visualize 4E-BP protein phosphorylated on Thr36/47. Lower panels: loading controls.
   (C) Quantitative RT-PCR comparing 4E-BP transcript levels in wild-type and melted mutant fat body tissue. Error bars: standard deviation.

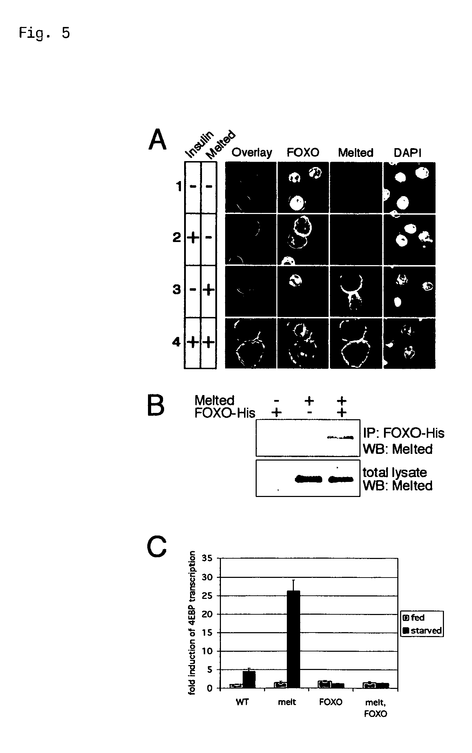

Figure 5: Melted binds FOXO and regulates expression of the FOXO target 4E-BP
   (A) Immunofluorescence showing S2 cells transfected to express FOXO-GFP and Melted, as indicated. FOXO-GFP was visualized with anti-GFP (green), Melted was detected with anti-Melted antibody (red). Nuclei were visualized with DAPI (blue). Cells were treated with insulin (+) or serum starved (-) as indicated.
   (B) Co-immunoprecipitation of Melted with HIS-tagged FOXO. S2 cells were transfected to express FOXO-HIS and/or Melted as indicated. Lysates were immunoprecipitated with anti-HIS and blots probed with anti-Melted. Melted expression in the total lysates is shown below.
   (C) Quantitative PCR comparing 4E-BP transcript levels in wild-type, *melt, foxo* and *melt*/*foxo* double mutant flies. Fed flies have normal levels of circulating insulin and therefore low FOXO activity. 4E-BP gene expression increased after starvation for 24 hours to reduce insulin levels, and showed a further FOXO-dependent increase in the melt mutant. Error bars: standard deviation.
Figure 6: Melted acts via FOXO and TOR
   (A) Total body triglyceride (normalized to total protein) in wild-type, *melt^{Δ1}* and *melt^{Δ1}* foxo double mutant 3-day old male flies. *melt^{Δ1}* and *melt^{Δ1} foxo* double mutant larvae were hand selected and grown without competition from heterozygous siblings. Error bars: standard deviation (5-9 replicates each).
   (B) Normalized total body triglyceride in 3-day old male flies containing the UAS-TOR or ppl-Gal4 transgenes or both combined to express UAS-TOR under ppl-G4 control. Error bars: standard deviation. One representative example is shown. The experiment was repeated 5 times, yielding a t-test=0.03.
   (C) Normalized total body triglyceride in 3-day old male wild-type, *melt^{Δ1}* and *melt^{Δ1}* mutant flies carrying the ppl-gal4 driver, UAS-TOR transgene or expressing UAS-TOR under ppl-Gal4 control.
   (D) Normalized total body triglyceride in newly eclosed wild-type, *melt^{Δ1}* and *melt^{Δ1}* mutant flies carrying the fat body specific gal4 driver lsp2, the UAS-Dp110 transgene (the catalytic subunit of PI3K), or expressing UAS-Dp110 under lsp2-Gal4 control.

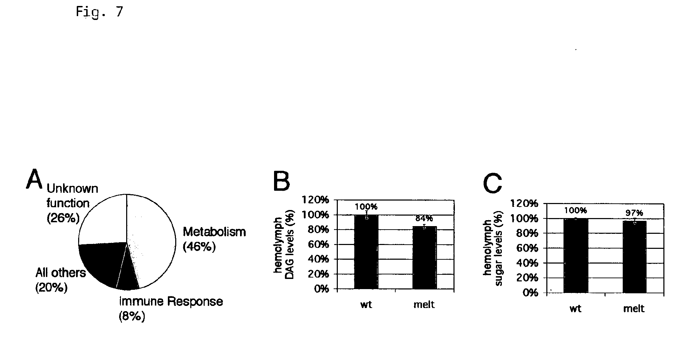

Figure 7: Transcriptional changes in *melt^{Δ1}* mutant fat body and resulting metabolic effects
   (A) Pie-chart showing the distribution of genes misregulated >1.5 fold in the *melt* mutant fat body.
   (B) Total circulating diacylglycide levels in hemolymph from wild-type and *melt^{Δ1}* mutant larvae. The reduction in circulating DAG is statistically significant (t-test = 0.02).
   (C) Total circulating sugar levels in hemolymph from wild-type and *melt^{Δ1}* mutant larvae.
Figure 8: Sequence information

Thus, the present invention relates to a pharmaceutical composition comprising a compound (a) reducing or inhibiting, or a compound (b) increasing the biological activity of the Melted protein or its expression, wherein said compound (a) is an antisense compound, antibody, an inactive version of the Melted protein or a nucleic acid sequence encoding an inactive version of the Melted protein and wherein said compound (b) is a biologically active version of the Melted protein or a nucleic acid sequence encoding a biologically active version of the Melted protein.

The term "Melted protein" as used herein relates to a Melted protein from any organism, with the mammalian protein being preferred. The term "Melted protein" covers in particular the following:
1. the human Melted protein
2. the mouse Melted protein
3. the drosophila Melted protein
4. an isoform, fragment, or variant of said proteins or polypeptides
5. a polypeptide which is at least 85%, preferably at least 90%, more preferably at least 95%, more preferably at least 98% and up to 99,6% identical to the amino acid sequence of said proteins
6. a homolog from any organism of said proteins
7. a polypeptide composed of a fusion between a polypeptide described in 1-6 with another polypeptide that may be, but is not limited to, a fluorescent protein, an epitope tag or an enzyme.

The term "biological activity of a Melted protein" as used herein includes, but is not limited to:
1. localization at the cell membrane
2. phospholipid binding
3. phosphatidylinositol(5)P binding
4. binding to a drosophila FOXO protein, a human FOXO protein, a FOXO homolog from another organism, or functional equivalents thereof
5. binding to drosophila Tsc1 protein, to the human homolog Hamartin, to a homolog from another organism, or to a functional equivalent thereof

The term "reducing, inhibiting or increasing the biological activity of the Melted protein" as used herein means that at least one of the biological activities of Melted is reduced, inhibited or increased, preferably the biological activity associated with the N-terminal protein interaction domain or the C-terminal PH domain.

The generation of suitable antisense oligonucleotides includes determination of a site or sites within the Melted gene for the antisense interaction to occur such that the desired effect, e.g., inhibition of expression of the protein, will result. A preferred intragenic site is (a) the region encompassing the translation initiation or termination codon of the open reading frame (ORF) of the gene or (b) a region of the mRNA which is a "loop" or "bulge", i.e., not part of a secondary structure. Once one or more target sites have been identified, oligonucleotides are chosen which are sufficiently complementary to the target, i.e., hybridize sufficiently well and with sufficient specificity, to give the desired effect. In the context of this invention, "hybridization" means hydrogen bonding, which may be Watson-Crick, Hoogsteen or reversed Hoogsteen hydrogen bonding, between complementary nucleoside or nucleotide bases. "Complementary" as used herein, refers to the capacity for precise pairing between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can hydrogen bond with each other. Thus, "specifically hybridizable" and "complementary" are terms which are used to indicate a sufficient degree of complementarity or precise pairing such that stable and specific binding occurs between the oligonucleotide and the DNA or RNA target. It is understood in the art that the sequence of an antisense compound does not need to be 100% complementary to that of its target nucleic acid to be specifically hybridizable. An antisense compound is specifically hybridizable when binding of the compound to the target DNA or RNA molecule interferes with the normal function of the target DNA or RNA to cause a loss of utility, and there is a sufficient degree of complementarity to avoid non-specific binding of the antisense compound to non-target sequences under conditions in which specific binding is desired, i.e., in the case of therapeutic treatment.

The skilled person can generate antisense compounds according to the present invention on the basis of the known DNA sequences for Melted. In this regard reference is made to Fig. 8.

"Oligonucleotide" refers to an oligomer or polymer of ribonucleic acid (RNA) or deoxyribonucleic acid (DNA) or mimetics thereof. This term includes oligonucleotides composed of naturally-occurring nucleobases, sugars and covalent internucleoside (backbone) linkages as well as oligonucleotides having non-naturally-occurring portions which function similarly. Such modified or substituted oligonucleotides are often preferred over native forms because of desirable properties such as, for example, enhanced cellular uptake, enhanced affinity for nucleic acid target and increased stability in the presence of nucleases. While antisense oligonucleotides are a preferred form of the antisense compound, the present invention comprehends other oligomeric antisense compounds, including but not limited to oligonucleotide mimetics such as are described below. The antisense compounds in accordance with this invention comprise from about 8 to about 50 nucleobases (i.e. from about 8 to about 50 linked nucleosides). Particularly preferred antisense compounds are antisense oligonucleotides, even more preferably those comprising from about 15 to about 25 nucleobases. Antisense compounds include RNA*ᵢ*, ₛᵢRNA, microₘᵢRNA, ribozymes, external guide sequences (EGS), oligonucleotides (oligozymes), and other short catalytic RNAs or catalytic oligonucleotides which hybridize to the target nucleic acid and inhibit its expression.

Alternatively, the pharmaceutical composition of the invention contains a vector allowing to transcribe an antisense oligonucleotide of the invention, e.g., in a mammalian host. Preferably, such a vector is a vector useful for gene therapy. Preferred vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors.. Further suitable vectors and methods for in vitro- or in vivo-gene therapy are described in the literature and are known to the persons skilled in the art; see, e.g., WO 94/29469 or WO 97/00957.

In order to achieve expression only in the target organ, the DNA sequences for transcription of the antisense oligonucleotides can be linked to a tissue specific promoter and used for gene therapy. Such promoters are well known to those skilled in the art (see e.g. Zimmermann et al., (1994) Neuron 12, 11-24; Vidal et al.; (1990) EMBO J. 9, 833-840; Mayford et al., (1995), Cell 81, 891-904; Pinkert et al., (1987) Genes & Dev. 1, 268-76).

Within an oligonucleotide structure, the phosphate groups are commonly referred to as forming the internucleoside backbone of the oligonucleotide. The normal linkage or backbone of RNA and DNA is a 3' to 5' phosphodiester linkage. Specific examples of preferred antisense compounds useful in the present invention include oligonucleotides containing modified backbones or non-natural internucleoside linkages. Oligonucleotides having modified backbones include those that retain a phosphorus atom in the backbone and those that do not have a phosphorus atom in the backbone. Modified oligonucleotide backbones which can result in increased stability are known to the person skilled in the art, preferably such modification is a phosphorothioate linkage.

A preferred oligonucleotide mimetic is an oligonucleotide mimetic that has been shown to have excellent hybridization properties, and is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone (see, e.g., Nielsen et al., Science 254 (1991), 1497-1500.)

Modified oligonucleotides may also contain one or more substituted or modified sugar moieties. Preferred oligonucleotides comprise one of the following at the 2' position: OH; F; 0-, S-, or N-alkyl; 0-, S-, or N-alkenyl; 0-, S- or N-alkynyl; or 0-alkyl-O-alkyl, wherein the alkyl, alkenyl and alkynyl may be substituted or unsubstituted C₁ to C₁₀ alkyl or C₂ to C₁₀ alkenyl and alkynyl. A particularly preferred modified sugar moiety is a 2'-O-methoxyethyl sugar moiety.

Oligonucleotides useful in the present invention may also include nucleobase modifications or substitutions. Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine etc., with 5-methylcytosine substitutions being preferred since these modifications have been shown to increase nucleic acid duplex stability.

Another modification of the oligonucleotides of the invention involves chemically linking to the oligonucleotide one or more moieties or conjugates which enhance the activity, cellular distribution or cellular uptake of the oligonucleotide. Such moieties include lipid moieties such as a cholesterol moiety, cholic acid, a thioether, a thiocholesterol, an aliphatic chain, e.g., dodecandiol or undecyl residues, a phospholipid, a polyamine or a polyethylene glycol chain, or adamantane acetic acid, a palmityl moiety, or an octadecylamine or hexylamino-carbonyl-oxycholesterol moiety.

The present invention also includes antisense compounds which are chimeric compounds. "Chimeric" antisense compounds or "chimeras," in the context of this invention, are antisense compounds, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Chimeric antisense compounds of the invention may be formed as composite structures of two or more oligonucleotides, modified oligonucleotides, oligonucleosides and/or oligonucleotide mimetics as described above. Such compounds have also been referred to in the art as hybrids or gapmers.

Antibodies useful for the present invention are polyclonal or monoclonal antibodies and their derivatives, especially chimeras, Fab-fragments, Fc-fragments, diabodies etc. The term "antibody", preferably, relates to antibodies which consist essentially of pooled monoclonal antibodies with different epitopic specifities, as well as distinct monoclonal antibody preparations. Monoclonal antibodies are made from an antigen containing, e.g., a fragment of Melted by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. (Wahl et al., J. Nucl. Med. 24, 316-325 (1983)). Thus, these fragments are preferred, as well as the products of a FAB or other immunoglobulin expression library. Moreover, antibodies useful for the purposes of the present invention include chimerical, single chain, and humanized antibodies.

Alternatively, preferred compounds for the purpose of the invention are inactive versions of Melted or genes encoding inactive versions of Melted. Such inactive versions can be generated according to well known methods. The term "inactive" as used herein means that at least one of the biological activities of Melted is abolished. Such compounds can have a therapeutic effect in the human body by displacing their functionally active counterpart, in particular when applied in excess. A preferred example of an inactive version of Melted is a mutated form of Melted, wherein the PH domain has been mutated.

Further compounds suitable for the purposes of the present invention and methods how to identify/select such compounds are in more detail described below.

Preferably, in a pharmaceutical composition, such compounds as described above are combined with a pharmaceutically acceptable carrier. "Pharmaceutically acceptable" is meant to encompass any carrier, which does not interfere with the effectiveness of the biological activity of the active ingredient and that is not toxic to the host to which it is administered. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and the active compound can be administered to the subject at an effective dose.

An "effective dose" refers to an amount of the active ingredient that is sufficient to affect the course and the severity of the disease, leading to the reduction or remission of such pathology. An "effective dose" useful for treating and/or preventing these diseases or disorders may be determined using methods known to one skilled in the art (see for example, Fingl et al., The Pharmocological Basis of Therapeutics, Goodman and Gilman, eds. Macmillan Publishing Co., New York, pp. 1-46 ((1975)).

Administration of the suitable compositions may be effected by different ways, e.g. by intravenous, intraperetoneal, subcutaneous, intramuscular, topical or intradermal administration. The route of administration, of course, depends on the kind of therapy and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of therapy, general health and other drugs being administered concurrently.

The present invention also relates to various therapeutic uses of the compounds of the invention, e.g.,
(a) for the prevention or treatment of a disease wherein an increase or decrease of the activity of Melted has a beneficial effect, including but not limited to obesity, adiposity and diabetes;
(b) for the prevention or treatment of a disease characterized by an increased activity of Melted, including, but not limited to obesity, adiposity or diabetes; and
(c) for the prevention or treatment of a disease characterized by a decreased activity of Melted, including, but not limited to an anorectic disease, anorexia, emaciation or any other disease of low body fat accumulation.

The present invention also relates to a method for identifying a compound capable of modifying a biological activity of the Melted protein or its expression, comprising the steps of:
(a) incubating a candidate compound with a test system comprising a Melted protein or its gene; and
(b) assaying a biological activity of the Melted protein;
wherein an altered biological activity of the Melted protein compared to a test system in the absence of said test compound is indicative of the presence of a candidate compound having the desired properties.

As regards a particular biological activity, such assay may be based on the PH domain function as described in the Examples; see also the results shown in Figure 3C+D: The PH domain is required for biological activity of melted protein in vivo. Mutant flies can be rescued by expression of a full-length Melted protein, but not by a protein lacking the PH domain. Expression of Melted protein, an epitope tagged Melted protein that can be recognised by an antibody or a GFP fusion (or any other fluorescent protein suitable for FRET, FLIM or other imaging technique based on fluorescence energy transfer) to either the full-length Melted protein or to its PH domain will show localization at the cell membrane. A tagged or GFP fusion construct to a version of Melted that is missing the PH domain is cytoplasmic. Thus, the function of the PH domain (i.e., localization of the protein at the cell membrane) can be used in an assay screening for activity of the PH domain, e.g, by transfecting cells with a Melted protein, epitope tagged Melted protein or GFP-Melted (preferably human) fusion protein, adding the candidate compound and determining the localization of the fusion protein to the membrane on the basis of GFP fluorescence, antibody labelling, fluorescence energy transfer or other suitable method. Alternately the degree of membrane localisation can be measured by subcellular fractionation, or by binding in an in vitro assay to immobilized inositol phospholipids. This can be based on use of phospholipids bound to a solid support, or any other means to measure protein-phopspholipid binding, such as Biacore, calorimetry etc.

There are also some other suitable alternative methods to screen for "Melted" function. These methods mentioned below are as well as the methods mentioned before to be understood as exemplary only:
1. Binding to phosphoinositides and/or recruitment to the cell membrane
   ➢ Assay localization of a Melted protein to the cell membrane
      - Said Melted protein may be a fusion to a fluorescent protein, and localization is detected by fluorescent imaging of cells
      - Indirect antibody detection of a Melted protein in a cell, where said Melted protein may have an epitope tag
      - FRET based assay in which a Melted protein has a donor or acceptor fluorophore, and the other fluorophore is tethered to the cell membrane. This tethering may be accomplished by fusing a fluorescent protein to a membrane localization signal such as a farnysylation signal or a CAAX domain. Alternatively, it may be fused to a protein that is constitutively membrane-localized.
   ➢ Assay recruitment of Tsc1 or Tsc2 to the cell membrane
      - said Tsc protein may be epitope tagged or fused to a fluorescent protein or detected by antibody to the native protein
   ➢ Assay recruitment of FOXO to the cell membrane
      - said FOXO protein may be epitope tagged or fused to a fluorescent protein or detected by antibody to the native protein
   ➢ Assay binding of a Melted protein to a phosphoinositide immobilized on a membrane, washing unbound Melted protein, and detection of bound Melted protein directly or indirectly.
   ➢ Assay melted binding to a phosphoinositide immobilized on a substrate suitable for detection by surface plasmon resonance.
   ➢ Assay for binding phosphoinostides in soluble form with a carrier, in micelles or inserted into lipid bilayer membranes to surface immobilized Metled protein or fragments thereof.
2. Binding to and/or affecting Tsc activity
   ➢ Assay binding of a Melted protein to a Tsc1 protein. This may be accomplished by:
      - immobilizing one of the two proteins on a surface, allowing the other to bind, washing unbound protein, and detecting the bound protein either directly or indirectly via epitope tags or fusion to reporters such as enzymes, or fluorophores
      - tagging the two proteins with fluorophores or fluorescent proteins and assaying binding by FRET or other energy transfer method such as FLIM
      - any homogeneous assay known to one skilled in the art, such as the AlphaScreen, BRET2, DELFIA, Fluorescence Polarization, LANCE, or TruPoint assays by Perkin Elmer
   ➢ Assaying modulation of Tsc activity. This may be accomplished by treating (1) cells that do express a Melted protein and (2) cells that do not express a Melted protein with a compound, detecting Tsc activity in each case, and comparing the two. Tsc activity may be detected by measuring activity of a component downstream of the Tsc signaling cascade such as Rheb GTPase or the TOR protein kinase, but not limited to these.
3. Binding to and/or affecting activity of a winged-felix Forkhead-family transcription factor, in particular a FOXO, but not limited to this subfamily.
   ➢ Assay binding of a Melted protein to a FOXO protein. This may be accomplished by:
      - immobilizing one of the two proteins on a surface, allowing the other to bind, washing unbound protein, and detecting the bound protein either directly or indirectly via epitope tags or fusion to reporters such as enzymes, or fluorophores
      - tagging the two proteins with fluorophores or fluorescent proteins and assaying binding by FRET or other energy transfer method such as FLIM
      - any homogeneous assay known to one skilled in the art, such as the AlphaScreen, BRET2, DELFIA, Fluorescence Polarization, LANCE, or TruPoint assays by Perkin Elmer
   ➢ Assaying modulation of activity of a FOXO protein. This may be accomplished by treating (1) cells that do express a Melted protein and (2) cells that do not express a Melted protein with a compound, detecting FOXO activity in each case, and comparing the two. Activity of the FOXO transcription factor may be detected by monitoring expression of a FOXO target gene. Examples of such target genes are 4EBP1-3. Expression levels of such target genes may be detected by Northern blotting or by quantitative RT-PCR or by hybridization of probes derived from RNA samples from the treated cells to DNA microarrays, such as Affymetrix or related products, including spotted cDNA arrays or genomic DNA arrays.

For carrying out assays based on GFP-Melted localisation or subcellular fractionation in cells, cells may be seeded 12 to 24 hours prior to transfection, then the cells are transfected with DNA encoding the GFP-Melted-fusion protein (with the Melted protein being any Melted protein, e.g., human Melted, fly Melted or any fragment thereof.) Then, the cells are allowed to express the transfected fusion protein for 12 to 36 hours and subsequently the cells are treated with a candidate compound. GFP localization in the cells is imaged, e.g., by fluorescence microscopy.

Screening protocol using drosophila S2 cells:
(a) Drosophila cells are seeded into a 6-well dish for 4 hours;
(b) each well is transfected with pMT-GAL4/VP16 (Cu-inducible GAL4 expression, constructed by inserting the GAL4/VP16 open reading frame between the SmoI and BamHI sites of pRmHa-3, sequence provided in attached file) and pUAS-dMeltPH-GFP (c.f. Fig. 8) using 4µL of transfectin reagent (Invitrogen, Carlsbad, CA, USA);
(c) the reaction is stopped by adding 2 ml of complete medium and the mixture is allowed to recover for 12 hours;
(d) induction is carried out with 700 µM cupric sulphate for 12 hours;
(e) the cells are treated with the candidate compound for a sufficient amount of time; and
(f) GFP localization is imaged by fluorescence microscopy.

Binding of Melted to the cell surface can also be assayed by use of surface plasmon resonance imaging, e.g., by the biacore method with PtdIns(5)p **(see www.biacore.com)**

As regards another particular biological activity of a Melted protein, such screening assay may be based on the binding between a Melted protein and (1) a FOXO protein or (2) a Tsc1 protein or homolog such as human Hamartin. Such a binding assay is illustrated in examples 4, 5 and 6. Such binding can be detected in a large number of ways which a person skilled in the art can devise and for which some examples have been mentioned above.

WO 98/25146 describes further methods for screening libraries of complexes for compounds having a desired property, especially, the capacity to agonize, bind to, or antagonize a polypeptide or its cellular receptor. The complexes in such libraries comprise a compound under test, a tag recording at least one step in synthesis of the compound, and a tether susceptible to modification by a reporter molecule. Modification of the tether is used to signify that a complex contains a compound having a desired property. The tag can be decoded to reveal at least one step in the synthesis of such a compound. Other methods for identifying compounds which interact with the melted protein or nucleic acid molecules encoding such molecules are, for example, the in vitro screening with the phage display system as well as filter binding assays or "real time" measuring of interaction using, for example, the BIAcore apparatus (Pharmacia).

Examples of candidate molecules include antibodies, oligonucleotides, proteins, peptides, or small molecules. Such molecules can be rationally designed using known techniques.

Preferably, said test system used for screening comprises substances of similar chemical and/or physical properties, most preferably said substances are almost identical. The compounds which can be tested and identified according to the method of the invention may be expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). Furthermore, genes encoding a putative regulator of Melted and/or which exert their effects up- or downstream of Melted may be identified using, for example, insertion mutagenesis using, for example, gene targeting vectors known in the art. Said compounds can also be functional derivatives or analogues of known inhibitors. Such useful compounds can be for example transacting factors which bind to Melted or regulatory sequences of the gene encoding it. Identification of transacting factors can be carried out using standard methods in the art. To determine whether a protein binds to the protein itself or regulatory sequences, standard native gel-shift analyses can be carried out. In order to identify a transacting factor which binds to the protein or regulatory sequence, the protein or regulatory sequence can be used as an affinity reagent in standard protein purification methods, or as a probe for screening an expression library. The identification of nucleic acid molecules which encode polypeptides which interact with Melted can also be achieved, for example, as described in Scofield (Science 274 (1996), 2063-2065) by use of the so-called yeast "two-hybrid system". In this system Melted is linked to the DNA-binding domain of the GAL4 transcription factor. A yeast strain expressing this fusion polypeptide and comprising a lacZ reporter gene driven by an appropriate promoter, which is recognized by the GAL4 transcription factor, is transformed with a library of cDNAs which will express plant proteins or peptides thereof fused to an activation domain. Thus, if a peptide encoded by one of the cDNAs is able to interact with the fusion peptide comprising a peptide of Melted, the complex is able to direct expression of the reporter gene. In this way, e.g., Melted and the gene encoding Melted can be used to identify peptides and proteins interacting with Melted. It is apparent to the person skilled in the art that this and similar systems may then further be exploited for the identification of inhibitors.

It is also well known to the person skilled in the art, that it is possible to design, synthesize and evaluate mimetics of small organic compounds that, for example, can act as a substrate or ligand to the Melted protein. For example, it has been described that D-glucose mimetics of hapalosin exhibited similar efficiency as hapalosin in antagonizing multidrug resistance assistance-associated protein in cytotoxicity; see Dinh, J. Med. Chem. 41 (1998), 981-987.

All these methods can be used in accordance with the present invention to identify a compound capable of modifying the biological activity of the Melted protein.

The gene encoding the Melted protein can also serve as a target for screening inhibitors. Inhibitors may comprise, for example, proteins that bind to the mRNA of the gene encoding Melted, thereby destabilizing the native conformation of the mRNA and hampering transcription and/or translation. Furthermore, methods are described in the literature for identifying nucleic acid molecules such as an RNA fragment that mimics the structure of a defined or undefined target RNA molecule to which a compound binds inside of a cell resulting in retardation of cell growth or cell death; see, e.g., WO 98/18947 and references cited therein. These nucleic acid molecules can be used for identifying unknown compounds of pharmaceutical interest, and for identifying unknown RNA targets for use in treating a disease. These methods and compositions can be used for identifying compounds useful to reduce expression levels of Melted.

Moreover, the present invention also relates to a diagnostic composition comprising a compound which is capable of specifically (a) binding to the Melted protein or (b) hybridizing to an mRNA encoding the Melted protein. Preferably, said compounds are oligonucleotides or antibodies as already defined above.

The oligonucleotides also comprise primers for PCR. The person skilled in the art is in a position to design suitable nucleic acids probes based on the information as regards the nucleotide sequence of Melted and the description of oligonucleotides provided above (e.g., as regards complementarity, lengths etc.). For evaluating whether the concentrations of Melted encoding mRNAs (or the translated proteins) are decreased or increased, the measured concentrations are compared with the concentration in a normal tissue. In order to restrict hybridisation of the probe to mRNA(s), i.e. to avoid hybridisation with the corresponding DNA which lead to incorrect results, the sample can, e.g., treated with DNAse I.

The probes (nucleic acid molecules or antibodies) can be detectably labelled, for example, with a radioisotope, a bioluminescent compound, a chemiluminescent compound, a fluorescent compound, a metal chelate, or an enzyme.

The expression of Melted in tissues can be studied with classical immunohistological methods. Other antibody based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (¹²⁵I, ¹²¹I), carbon (¹⁴C), sulfur (³⁵S), tritium (³H), indium (¹¹²In), and technetium (⁹⁹mTc), and fluorescent labels, such as fluorescein and rhodamine, and biotin. In addition to assaying Melted levels in a biological sample, Melted can also be detected in vivo by imaging. Antibody labels or markers for in vivo imaging of protein include those detectable by X-radiography, NMR or ESR. For X-radiography, suitable labels include radioisotopes such as barium or caesium, which emit detectable radiation but are not overtly harmful to the subject. Suitable markers for NMR and ESR include those with a detectable characteristic spin, such as deuterium, which may be incorporated into the antibody by labelling of nutrients for the relevant hybridoma. A protein-specific antibody or antibody fragment which has been labelled with an appropriate detectable imaging moiety, such as a radioisotope (for example, ¹³¹I, ¹¹²In, ⁹⁹mTc), a radio-opaque substance, or a material detectable by nuclear magnetic resonance, is introduced (for example, parenterally, subcutaneously, or intraperitoneally) into the patient. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of ⁹⁹mTc. The labelled antibody or antibody fragment will then preferentially accumulate at the location of cells which contain the specific DSM-protein.

Finally, the present invention relates to the use of the diagnostic compounds defined above for the preparation of a diagnostic composition for the diagnosis of a disease associated with an aberrant activity and/or level of the Melted protein or predisposition for such disease.

The below example explains the invention in more detail.

### Example 1: General experimental procedures

### (A) Plasmids

In the examples the *melted* sequences as sequenced by the inventors have been used (c.f. Fig. 8). The other *melted* sequences shown in Fig.8 are to be understood as possible alternatives.

A full-length *melted* cDNA was cloned into pUAST (c.f. Fig. 8) as an EcoRI-NotI fragment (pAT159). This clone differs from the annotated CG8624.**[www.flybase.net]** due to splicing differences. Human *melted* was cloned by RT-PCR from HeLa cell cDNA and cloned into pUAST (pAT206). The dMeltPH-GFP fusion was constructed by amplifying the PH domain (Primersequenzen: ccccctcgagGCTTTCTCAATGGCAGCAAT; cgcggatcctaCGTGGGATTATCTCGGGCCTG) for cloning into a metallothionine promoter vector containing GFP (pAT195; c.f. Fig. 8). The GFP fusion was then cloned into pUAST (pAT202) for transformation of Drosophila. His-tag Melted was made by inserting the dMelt ORF into pET23a (c.f. Fig. 8) (plasmid pAT160). For the yeast two-hybrid screen, a bait plasmid containing dMelt without the PH domain was made by inserting a 1695 bp EcoRI-XhoI fragment from pAT159 into the EcoRI-SalI sites of pGBDU (c.f. Fig. 8) (pAT165). Myc-tagged Tsc1 and of V5/His-tagged Tsc2 plasmids were a kind gift of Duojia Pan. FOXO-GFP was constructed as a C-terminal GFP fusion in pUAST. Two UAS-melted-RNAi transgenes were prepared by PCR (tctagaCTCGCACGCGGACTATGTGAA and tctagaCTTGGCATGGGGTGGCTCTTC; tctagaCGCCCACACGTTGCAGCATTG and tctagaGGACTCCCGCGTGCTAAG) and cloned into pWIZ (Lee and Carthew, Methods 30 (2003), 322-9).

### (B) Strains and Transgenic Lines

*engrailed-Gal4* and *tubulin-Gal4* are described in (http://fly.bio.indiana.edu/gal4.htm) *pumpless-(ppl)-GAL4* is described in Zinke et al., Development 126 (1999), 7275-84. *chico1* flies are described in Böhni et al., Cell 97 (1999), 865-75. Flies with UAS-Tsc1-myc and UAS-Tsc2-V5 were from DJ Pan.

### (C) PIP-binding assay

Bacteria expressing HIS-tagged Melted were lysed by a freeze/thaw cycle, subsequent addition of 0.2 mg/ml lysozyme and 0.2 mg/ml DNAse, incubation on ice for 30 min and vigorous vortexing. No detergent was used because it inhibits binding to phosphoinositides. The lysate was cleared by centrifugation, diluted 1:1000 or 1:10,000 in TBST (10 mM Tris/HCL pH 8.0, 150 mM NaCl, 0.1% Tween-20)+3% BSA(fatty-acid-free)+protease inhibitors and incubated for 1 h with PIP membranes (Echelon Biosciences, Salt Lake City, UT, USA),pre-blocked with 3% fatty-acid-free BSA in TBST. Strips were washed twice with TBST for 30', incubated for 1 h RT with anti-HIS in TBST + 3% BSA followed by HRP-conjugated 2nd antibody.

### (D) Y2H

A Drosophila larval cDNA library in pGAD-C was kindly provided by Grace Gill. The yeast two-hybrid screen was carried out in the strain PJ69-4α, containing reporters for lacZ expression and for growth on adenine and histidine. PJ69-4α. (*ura3*-, *leu2-*) was transformed with pAT165. A dMelt expressing clone was chosen by immunoblotting. This was transformed with library plasmid DNA, plated on ura⁻leu⁻ade⁻ dropout synthetic medium to select for presence of the bait plasmid (URA3), the library plasmid (LEU2) and for activation of the adenine reporter. Colonies were retested for growth on ura⁻leu⁻ade⁻his⁻-medium for activation of the histidine reporter and on X-gal plates. Plasmids were isolated from clones that satisfied all these reporter-construct requirements. Sequences also identified in a second independent screen were considered non-specific and discarded.

### (E) Antibodies

Anti-dMelt was raised in rats against the full-length protein expressed in *E. coli.* Rats were injected with 50 µg dMelt protein in RIBI adjuvant. For immunoprecipitation, cells were lysed for 5-10 minutes on ice in 50 mM Tris pH 7.5, 150 mM NaCl, 1% Triton X-100 (with protease and phosphatase inhibitors). Lysates were cleared by centrifugation at 4°C at maximum speed for 15 minutes, and incubated with antibody for 2 hours at 4°C. 50 µl of 50% agarose-protein A bead slurry were washed twice in lysis buffer and added to the antibody/cell-lysate mixture for 30'. Following 3 washes with cold lysis buffer, proteins were recovered using 2x protein-gel loading buffer and heating for 5' at 95°C.

### (F) Fly measurement and metabolic characterization

Metabolic measurements were done on test and control flies grown under identical conditions. 50 newly-hatched 1st instar larvae were seeded per vial and adults hatching on the same day were aged for 2 or 3 days before testing. Male flies were weighed in batches of 50. Total body triglycerides were measured by homogenizing flies in 0.05% Tween-20 with protease inhibitors. After heating at 70°C for 5' lysates were cleared by centrifugation and triglycerides measured using Infinity Triglyerides Reagent (Thermoelectron; Waltham, MA, USA. Total protein was measured on the same samples using Biorad protein assay. To measure sugars, 1 µl of hemolymph was put into 9 µl of buffer (5 mM Tris, pH 6.6, 137 mM NaCl, 2.7 mM KCl), heated for 5' at 70°C, incubated with 1 µl of porcine trehalase (Sigma T-8778, St. Louis, MO, USA) at 37°C for 12 hr and measured using Sigma glucose (G) assay kit (GAGO-20).

### Example 2: Melted modulates tissue growth

Melted was identified in a gain-of-function screen for genes affecting tissue growth during Drosophila development. EP31685 caused overgrowth when expressed in the posterior half of the wing using *en-GAL4* (Fig. 1A, the red wing expressed EP31685). When expressed ubiquitously with *tubulin-GAL4,* EP31685 caused a small but statistically significant increase in total body weight (student t-test < 0.005; Fig. 1C). EP31685 is located -50bp upstream of the annotated gene *melted (melt,* CG8624; Fig 1B). Using a *UAS-melt* transgene, we verified that the *melted* transcription unit is responsible for the tissue overgrowth phenotype (not shown).

To generate *melted* mutants, deletions were prepared using P-element mediated male recombination (Preston and Engels, Genetics 144, 1611-1622,(1996)) starting from EP31685 (Fig. 1B). *melt^{Δ1}* is a 22 kb deletion that removes the entire *melted* gene and two adjacent genes. The second allele, *melt^{Δ2}* is a 2.5 kb deletion that removes the first exon of CG8624. Flies homozygous mutant for *melt^{Δ1}* were semi-viable (70% viable to adult) and fertile, but were -10% smaller than control flies (Fig. 1D). Though small in magnitude, the reduction in body size was statistically significant (t-test < 0.005). Flies heterozygous mutant for *melt^{Δ1}* /*melt^{Δ2}* were also significantly smaller than control flies (not shown). Two additional lines of evidence indicate that the growth defect was due to loss of *melted* and not the other genes deleted in *melt^{Δ1}.* First, the growth defect of the homozygous *melt^{Δ1}* deletion flies was fully rescued by ubiquitous expression of the *UAS-melt* transgene (Fig. 1D). Second, flies expressing UAS-RNAi constructs directed against two different regions of the *melt* transcript caused tissue undergrowth, resembling the *melt^{Δ1}* phenotype (not shown).

The predicted Melted protein is conserved in C. elegans (CE25943), mice (Muto et al., Biochimie 86, 523-531 (2004)) and humans (HGNC, KIAA1692). Alignment of these sequences showed a conserved region at the N-terminus (76% similar in fly and human) and a conserved Pleckstrin Homology (PH) domain at the C-terminus. Ubiquitous expression of Melted lacking the PH domain did not rescue the *melt^{Δ1}* size defect (Fig. 1D), indicating the PH domain is required for Melted function. The truncated protein is likely to fold correctly because it was used successfully as bait in a yeast two-hybrid screen to detect proteins that also interact with full-length Melted (see below). The human homologue of Melted also rescued the size reduction of the *melt^{Δ1}* mutant (Fig. 1D), indicating that the molecular function of Melted is conserved.

### Example 3: Melted controls fat metabolism

The next question to be answered was whether Melted might also affect fat metabolism. Drosophila stores fat mainly as triglycerides. Total body triglyceride was measured for *melt^{Δ1}* mutant and control flies reared under identical, controlled conditions. *melt* mutant flies had only 60% as much triglyceride as control flies, when normalized to total protein to take into account the 10% reduced body size of *melted* mutants. This reduction in fat content was statistically significant (t-test < 0.001) and was rescued by ubiquitous expression of a *UAS-melt* transgene (Fig. 2A). Further confirmation that the leanness of the mutant was due to reduced *melted* expression was obtained by ubiquitous expression of a *melted* UAS-RNAi construct in flies (25% leaner, Fig. 2B). Total body triglycerides of wandering 3^{rd} instar *melt^{Δ1}* mutant larvae were also 20% lower than controls, indicating that Melted regulates fat levels throughout development, as well as in the adult (not shown).

Fat levels can be controlled by humoral factors, including adipokinetic hormone (AKH), which induces mobilization of fat reserves (Lee and Park, Genetics 167, 311-323 (2004)). Insulin-like peptides (ILPs) also control fat metabolism in the fly (Ikeya et al., Curr. Biol. 12, 1293 (2002); Hwangbo et al., Nature 429, 562-566 (2004)). To ask whether elevated AKH or ILP-2, -3 and -5 expression could explain the leanness of *melted* mutants, the inventors tested transcript levels by quantitative RT-PCR. Transcript levels were not significantly elevated. Since altered expression of the known humoral regulators did not provide an explanation for the mutant phenotype, the inventors asked if there was a defect in adipose tissue. The 'fat body' is the main fat-storage organ of the fly, containing over 80% of total body triglycerides. The inventors isolated *melt^{Δ1}* mutant and control fat body tissue from larvae and found 25% lower triglyceride level in the mutant tissue. The total body leanness of *melt^{Δ1}* mutants was rescued by expressing *melted* specifically in the fat body, using *ppl-GAL4* (Fig. 2C). In contrast, expression of *melted* in the nervous system using *elav-GAL4* or in brain neurosecretory cells using *dILP3-GAL4* did not rescue the mutant (not shown). This indicates that Melted activity is required in adipose tissue.

### Example 4: Membrane localization via the Melted PH domain

Melted contains a C-terminal PH domain. The inventors tested if bacterially expressed Melted protein binds to immobilized phosphoinositides. At higher concentration, Melted bound many phosphoinositides, but at lower concentrations showed preferential binding to PI(5)P (Fig. 3A, A'). The PH domain of Melted expressed as a GST fusion exhibited a similar binding pattern, although with reduced selectivity. Although Melted did not show strong binding to PIP3 in vitro, the inventors generated a GFP fusion to the PH domain (MeltPH-GFP) and tested it in S2 cells for insulin-induced relocalization. MeltPH-GFP was detectable predominantly at the cell membrane when cells were serum-starved to deprive them of insulin and following insulin-stimulation, without apparent difference (Fig. 3B, B'). The MeltPH-GFP fusion was also predominantly cortical in wing imaginal disc cells, whereas a Melted-GFP fusion protein lacking the PH domain was cytoplasmic (Fig. 3C, D), suggesting that the majority of Melted protein is localized to the membrane under normal conditions. This was confirmed by subcellular fractionation of wing disc cells. The majority of the endogenous Melted protein was recovered in the membrane fraction, with a lesser amount in the cytoplasm (Fig 3E). No signal was detected in corresponding fractions prepared from *melted* mutant tissue, confirming the specificity of the antibody. Control proteins fractionated predominantly as cytoplasmic (Y14) or membrane-associated (PVR). These results suggest that the endogenous Melted protein is membrane localized via its PH domain and that its localization is not regulated by insulin-dependent changes in PIP3 levels. The PH domain is required for Melted function in vivo (Fig 1D).

### Example 5: Melted protein binds to Tsc1 and modulates TOR activity

To study the function of the N-terminal domain, the inventors performed a yeast two-hybrid screen using MeltΔPH as bait to screen 2.7 million clones from a larval cDNA library. Six interacting genes were isolated: Tsc1 (4 independent clones), 14-3-3e (3 independent clones), CG16719, CG5171, CG6767, and CG8242 (once each). When expressed in S2 cells by cotransfection, Melted co-immunoprecipitated with Myc-tagged Tsc1. Interaction of Melted and Tsc1 in S2 cells was also visualized by immunofluorescence microscopy. Tsclmyc was uniformly distributed in the cytoplasm when expressed alone (Fig. 4A row 1; S2 cells lack endogenous Melted). Co-expression with Melted caused Tsc1-myc to shift predominantly to the cell membrane (Fig. 4A row 2). When expressed in S2 cells, Tsc2 was uniformly distributed in the cytoplasm (Fig. 4 row 3). Co-expression of Tsc2 with Melted did not cause a relocalization of Tsc2 to the membrane, suggesting that Melted and Tsc2 do not interact directly (Fig. 4A row 4). However, when co-expressed with Melted and Tsc1, Tsc2 also relocated (Fig. 4A row 6), indicating that Melted can recruit the Tsc1/2 complex to the cell membrane.

Does this interaction affect TOR pathway activity? Previous reports have shown that increased PI3K or TOR signaling in the adipose tissue increased fat accumulation or reduced fat consumption (autophagy; Britton et al., Dev Cell 2, 239-249 (2002); Rusten et al., Dev Cell 7, 179-192 (2004)). The observation that *melted* mutants are lean, could be explained if TOR activity is reduced in the mutant. To test this more directly the inventors assayed the level of TOR-dependent phosphorylation of 4E-BP in control and *melt^{D1}* mutant fat bodies (Fig. 4B). Phosphorylation of Drosophila 4E-BP at positions 36/47 depends on TOR activity and can be visualized using an antibody specific to the corresponding phosphorylated peptide from human 4E-BP (Miron et al., Nat Cell Biol 3, 596-601 (2001)). Although the total level of 4E-BP protein was higher in *melt^{Δ1}* mutant tissue, the level of phosphorylation was not correspondingly elevated (perhaps mildly reduced, Fig. 4B, left panel). The increase in total 4E-BP level reflects a 2-3 fold increase in transcript levels in the mutant fat body (Fig. 4C). As a control, the inventors increased 4E-BP levels by a comparable amount in wild-type fat body using *ppl-Gal4 UAS-4E-BP* and observed that the level of 4E-BP phosphorylation was correspondingly elevated (Fig 4B, right panel). This indicates that TOR activity is not limiting in wild-type fat body. Thus the observation that 4E-BP phosphorylation did not increase, despite increased total 4E-BP, provides strong evidence that TOR activity is reduced in the *melted* mutant adipose tissue.

### Example 6: Melted binds to FOXO and modulates FOXO activity

As a second means to identify possible functions of Melted, the inventors used the Eukaryotic Linear Motif server (http://elm.eu.org/) to look for functional motifs conserved between fly and human Melted. The only conserved motifs found in the N-terminal region of these proteins were two Forkhead-associated domain ligand domains (LIG_FHA_1). Forkhead transcription factors, FoxA2, FoxA3, FoxC2 and FoxO1 are involved in glucose and fat metabolism. Insulin signaling activates Akt, which phosphorylates FOXO and leads to its retention in the cytoplasm. The inventors therefore asked if Melted affects the subcellular localization of a FOXO-GFP fusion protein. FOXO-GFP was predominantly nuclear in the absence of insulin stimulation in serum-starved S2 cells and increased in the cytoplasm following insulin stimulation (Fig. 5A, row 1, 2). In serum-starved cells co-transfected to express Melted, FOXO-GFP was still primarily nuclear (Fig. 5A row 3), but much of the nonnuclear protein appeared at the membrane, colocalized with Melted. Upon insulin stimulation, the inventors observed a robust increase in the level of FOXO-GFP at the cell membrane (Fig. 5A row 4). The interaction was confirmed by co-immunoprecipitation of Melted with FOXO in insulin-stimulated S2 cells (Fig. 5B).

The observation that insulin-stimulation induced a shift toward membrane localization of FOXO in the presence of Melted in S2 cells raised the possibility that *melted* regulates FOXO activity in vivo. To address this we examined expression of the FOXO target 4E-BP (Junger et al., 2003) in wild-type and *melted* mutant animals. Under fed conditions, insulin signaling is active and 4E-BP transcript levels are relatively low (Fig 5C). In wild-type flies that were starved for 24h to reduce insulin levels and thereby activate FOXO, 4E-BP transcript increased -4 fold (Fig 5C). In starved flies lacking Melted, 4E-BP transcript increased over 25 fold. This increase in 4EBP transcription was absent in the starved melted/FOXO double mutant, confirming that it is FOXO dependent. Thus in the absence of Melted, FOXO activity is higher than normal, suggesting that Melted limits FOXO activity in vivo.

### Example 7: Melted acts via TOR and FOXO

To ask whether the elevated FOXO activity observed in *melted* mutants contributes to the lean phenotype of these animals, the inventors compared the normalized triglyceride levels of *melted* mutant and *melted* foxo double mutant flies. Reducing FOXO activity suppressed the leanness of the *melted* mutant to a considerable degree, reaching near normal fat levels (Fig 6A). The rescue was highly statistically significant (t-test = 10⁻⁵ ). foxo mutants did not show higher than normal fat levels compared to wild-type (not shown). These observations suggest that Melted acts by regulating FOXO activity to control expression of genes important in fat metabolism.

*Melted* mutants also exhibit reduced TOR activity. To ask whether TOR activity affects fat accumulation, the inventors tested the effects of increasing TOR activity in wild-type and *melted* mutant adipose tissue. The inventors made use of a UAS-TOR transgene that can provide TOR activity in vivo when expressed at appropriate levels (Hennig and Neufeld, Genesis 34, 107-110 (2002)). The inventors first confirmed that expression of UAS-TOR under ppl-Gal4 control in adipose tissue led to increased total body fat (Fig 6B, t-test = 0.03), as did increasing PI3K activity (not shown, see also Britton et al 2002). In contrast, a comparable elevation of TOR expression in *melted* mutant flies had no effect on fat levels (Fig 6C). Both this result and the significant rescue caused by removal of FOXO indicate that in the *melted* mutant, the FOXO branch of the pathway becomes limiting for fat accumulation. In view of this finding, the inventors next asked whether elevated TOR pathway activity could increase fat levels in the *melted* mutant if FOXO activity was simultaneously reduced. To do so the inventors made use of the catalytic subunit of PI3K (Dp110) to inactivate FOXO and simultaneously activate TOR. The fat body driver lsp2-Gal4 or the UAS-Dp110 transgenes had little effect on their own in the *melted* mutant background, but when combined, the elevated PI3K activity in the fat body increased fat levels of the *melted* mutant (Fig 6D). The effect was stronger than that of removing FOXO only, increasing fat levels to above normal (compare Fig. 6A,D). Taken together these observations suggest that the TOR branch of the pathway contributes to the control of fat levels under conditions where FOXO activity levels are low. This is normally the case in feeding animals where insulin levels are relatively high (FOXO activity is elevated under starvation conditions: compare 4E-BP levels in fed vs starved wild-type and foxo mutant flies, Fig 5C). Under conditions where insulin levels are low, or in the melted mutant, where FOXO activity is elevated, the effects of FOXO dominate.

### Example 8: Transcriptional profiling of melted mutant adipose tissue

To better understand the metabolic impact of the loss of Melted function in the adipose tissue, the inventors performed microarray expression profiling of *melted* mutant versus control fat body. The inventors used microarrays containing 11,445 cDNA clones (DGC1 and DGC2 collection) and analyzed the data using Statistical Analysis of Microarrays (SAM) in the MeV module of the TIGR TM4 microarray software suite (http://www.tigr.org/software/tm4/mev.html; Tusher et al., PNAS 98, 5116-5121 (2001)). Allowing for a 1% false-positive rate (q value < 0.01), the inventors identified 315 genes that were upregulated and 405 genes that were downregulated in the *melted* mutant. This represents 6% of all genes sampled, and reflects substantial reprogramming of the transcriptional profile of the adipose tissue. Within this set, the inventors considered genes altered by at least 1.5-fold (249 genes) and grouped them according to their functional annotation (www.flybase.org). 46% of the regulated genes are involved in metabolism (Fig. 7A). Interestingly, many of these are involved in lipid metabolism (Table 1). 10 are cytochrome P450 enzymes, involved in the oxidation of lipophilic molecules. We confirmed the transcriptional regulation of some of these genes by semi-quantitative RT-PCR (2^{nd} column, Table 1). In addition to the genes involved in fat metabolism, a significant proportion of the misregulated metabolic genes are involved in protein degradation.

Several of the down-regulated genes are involved in the accumulation of triglycerides. One of the most highly down-regulated genes in the *melt^{Δ1}* mutant adipose tissue is the transcription factor *sugarbabe* (2.4-fold). *sugarbabe* was previously identified as a gene controlling the conversion of sugars to fats (Zinke et al., EMBO J. 21, 6162-6173 (2002)). It is the 2^{nd} most highly upregulated gene when flies fed sugar, but deprived of lipids, and it becomes expressed in the adipose tissue, gut and malphighian tubules. Acetyl-CoA sythase (AcCoAS) was also identified in the same screen as a gene upregulated on a sugar diet (7.3-fold), whereas in the *melt^{Δ1}* mutant adipose tissue it was down-regulated (1.8-fold). The down-regulation of both *sugarbabe* and AcCoAS suggests that *melt^{Δ1}* mutant adipose tissue might not be able to accumulate enough lipid. This is corroborated by the finding that glycerol kinase (Gyk) and PEPCK are among the genes most down-regulated in *melted* mutant adipose tissue (Table 1). In order to generate triglycerides, both free fatty acids and 3-phosphoglycerol are required by the cell. In vertebrate brown adipose tissue, 3-phosphoglycerol is made by Gyk, whereas in white adipose tissue it is made by PEPCK (Hanson and Reshef, Biochimie 85, 1199-1205(2003)). PEPCK is rate limiting in that loss of function in the mouse leads to lipodystrophy whereas overexpression in mouse adipose tissue leads to obesity. Therefore, the finding that both Gyk and PEPCK are down-regulated in the *melt^{Δ1}* mutant fat-body suggests that these animals are lean because they do not accumulate enough lipid in the adipose tissue. Consistent with what is known in vertebrates, the inventors detected increased PEPCK levels by quantitative RT-PCR in other non-adipose tissues under starvation conditions and in the *melted* mutant, thus the transcriptional changes in adipose and non-adipose tissues do not always correlate. Recently, adipose triglyceride lipase has been reported to catalyze the initial step in triglyceride hydrolysis in mice and inhibition of this enzyme markedly decreases total adipose acyl-hydrolase activity (Zimmermann et al., Science 306, 1383-1386 (2004)). BLAST searches identified two Drosophila homologs of adipose triglyceride lipase: CG5295 and CG5560. Interestingly, CG5295 expression was markedly reduced in the *melt^{Δ1}* mutant fat body. This suggests that lipid hydrolysis might be downregulated in the mutant adipose tissue.

To test experimentally these predictions from expression data, the inventors measured circulating lipids, which in the fly are mobilized from the fat body and delivered to peripheral tissues as diacylglycerides (DAG) in the hemolymph (Canavoso et al., Annu Rev Nutr 21, 23-46 (2001)). Hemolymph DAG was low in *melt^{Δ1}* mutant larvae compared to controls (Fig 7B; t-test = 0.02). Thus, it is not likely that the reduced triglycerides in the adipose tissue can be explained by increased mobilization of fat in the form of circulating DAG. The inventors also measured the level of circulating blood sugar (trehalose + glucose) and found it was not elevated in the *melt^{Δ1}* mutant (Fig. 7C). These experiments, together with the expression data, mean that the leanness observed in the mutant is likely due to reduced triglyceride accumulation in adipose tissue.

### Example 9: Tissue distribution of Melted expression

By use of quantitative RT-PCR on RNA from dissected mouse tissues it was found that in addition to the already published expression domains (ventricles of the brain and the kidneys), Melted is expressed in the hypothalamus and in the liver. This is an interesting observation since both of these organs are important for energy metabolism in mammals. Melted does not appear to be expressed in white adipose tissue (i.e. fat- WAT) and brown adipose tissue (BAT)).

## Claims

1. A pharmaceutical composition comprising a compound (a) reducing or inhibiting, or a compound (b) increasing the biological activity of the Melted protein or its expression, wherein said compound (a) is an antisense compound, an inactive version of the Melted protein or a nucleic acid sequence encoding an inactive version of the Melted protein and wherein said compound (b) is a biologically active version of the Melted protein or a nucleic acid sequence encoding a biologically active version of the Melted protein

2. The pharmaceutical composition of claim 1, wherein said compound (a) is the Melted protein having a mutation within its PH domain or lacking at least parts of its PH domain or lacking its PH domain entirely.

3. A method for identifying a compound capable of modifying a biological activity of the Melted protein or its expression, comprising the steps of:
(a) incubating a candidate compound with a test system comprising the Melted protein or its gene; and
(b) assaying a biological activity of the Melted protein;
wherein an altered biological activity of the Melted protein compared to a test system in the absence of said test compound is indicative of the presence of a candidate compound having the desired properties.

4. Use of a compound as defined in claim 1 or 2 for the preparation of a pharmaceutical composition for the prevention or treatment of a disease wherein decrease or increase of the activity of the Melted protein has a beneficial effect.

5. Use of a compound as defined in claim 1 or 2 for the preparation of a pharmaceutical composition for the prevention or treatment of a disease **characterized by** an increased activity of the Melted protein.

6. Use according to claim 5, wherein said disease is obesity, adiposity, dyslipidemia or diabetes.

7. Use of a compound as defined in claim 1 or 2 for the preparation of a pharmaceutical composition for the prevention or treatment of a disease **characterized by** a decreased activity of the Melted protein.

8. Use according to claim 7, wherein said disease is an anorectic disease, emaciation or any other disease of low body fat accumulation.

9. A diagnostic composition comprising a compound which is capable of specifically (a) binding to the Melted protein or (b) hybridizing to the mRNA encoding the Melted protein.

10. The diagnostic composition of claim 9, wherein said compound is an oligonucleotide probe or an antibody.

11. The diagnostic composition of claim 10, wherein said oliognucleotide probe or antibody are detectably labelled.

12. The diagnostic composition of claim 11, wherein said label is selected from the group consisting of a radioisotope, a bioluminescent compound, a chemiluminescent compound, a fluorescent compound, a metal chelate, or an enzyme.

13. Use of a compound as defined in any one of claims 9 to 12 for the preparation of a diagnostic composition for the diagnosis of a disease associated with an aberrant activity and/or level of the Melted protein or predisposition for such disease.
